# EUROPEAN PATENT SPECIFICATION

(11) **EP 4 651 951 B1**
(45) Date of publication and mention of the grant of the patent: **28.10.2026**
(21) Application number: 24707402.4
(22) Date of filing: 18.01.2024
(51) Int. Cl.: A61P 25/00, C07D 487/04, A61K 31/5025

(54) **COMPOSITIONS USEFUL FOR MODULATING SPLICING**
ZUSAMMENSETZUNGEN ZUR MODULATION DES SPLEISSENS
COMPOSITIONS UTILES POUR MODULER L'ÉPISSAGE

(30) Priority: 19.01.2023 US 202363480558 P
(43) Date of publication of application: 26.11.2025
(73) Proprietor: Skyhawk Therapeutics, Inc., Waltham, MA 02451 (US)
(72) Inventor: RATNI, Hasane, Waltham, Massachusetts 02451 (US); KONETZKI, Ingo, Waltham, Massachusetts 02451 (US); LUCAS, Brian, Waltham, Massachusetts 02451 (US); LUZZIO, Michael, Waltham, Massachusetts 02451 (US)
(74) Representative: Mewburn Ellis LLP
(86) International application number: PCT/US2024/012063
(87) International publication number: WO 2024/155847

(56) References cited:
- WO-A1-2014/143607
- WO-A1-2022/169864
- WO-A1-2022/169868

## Description

### RELATED APPLICATIONS

This application claims the benefit of U.S. provisional patent application no. 63/480,558 filed on January 19, 2023.

### SEQUENCE LISTING

This instant application contains a Sequence Listing which has been submitted electronically in XML format and is hereby incorporated by reference in its entirety. Said XML copy, created on January 17, 2024, is named 51503-769_602_SL.xml and is 12,471 bytes in size.

### BACKGROUND

Spinocerebellar Ataxia 3 (SCA3 or Machado-Joseph Disease) is a rare, inherited, neurodegenerative, autosomal dominant disease. It is characterized by progressive degeneration of the brainstem, cerebellum and spinal cord, however, neurons in other areas of the brain are also affected. Presenting features include gait problems, speech difficulties, clumsiness, and often visual blurring and diplopia; saccadic eye movements become slow and ophthalmoparesis develops, resulting initially in up-gaze restriction. Ambulation becomes increasingly difficult, leading to the need for assistive devices 10 to 15 years following onset. Late in the disease course, individuals are wheelchair bound and have severe dysarthria, dysphagia, facial and temporal atrophy. The disease progresses relentlessly until death occurs at any time from 6 to approximately 30 years after onset through pulmonary complications.

SCA3 is caused by CAG tri-nucleotide repeats in exon 10 of the Ataxin 3 (ATXN3) gene. ATXN3 encodes for a deubiquinase with wide-ranging functions, but it does not appear to be an essential gene. Disease causing variants of the ATXN3 gene have approximately 40 to over 200 CAG tri-nucleotide repeats in exon 10. Expanded CAG repeats in the ATXN3 gene are translated into expanded polyglutamine repeats (polyQ) in the ataxin-3 protein and this toxic Ataxin 3 protein is associated with aggregates. The polyglutamine expanded ataxin-3 protein in these aggregates is ubiquitinated and the aggregates contain other proteins, including heat shock proteins and transcription factors. Aggregates are frequently observed in the brain tissue of SCA3 patients. Compounds with activity against SCA3 have been disclosed in WO 2022/169864 and WO 2022/169868. There are currently no treatments for SCA3.

### SUMMARY

In one aspect, described herein is a compound of Formula (I), or a pharmaceutically acceptable salt thereof. 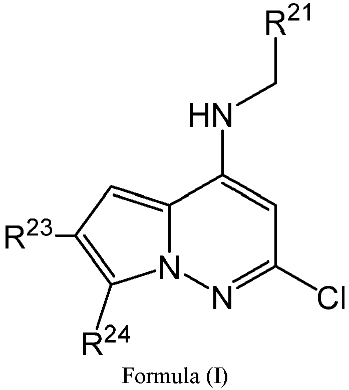 wherein R²¹, R²³ and R²⁴ are as defined herein.

Also provided herein are pharmaceutical compositions comprising a compound disclosed herein, or a pharmaceutically acceptable salt thereof, and a pharmaceutically acceptable excipient or carrier.

In some aspects, described herein, is a method of modulating splicing of a Ataxin3 (ATXN3) pre-mRNA, comprising contacting a small molecule splicing modulator compound disclosed herein (SMSM) to the ATXN3 pre-mRNA with a splice site sequence or cells comprising the ATXN3 pre-mRNA, wherein the SMSM binds to the ATXN3 pre-mRNA and modulates splicing of the ATXN3 pre-mRNA in a cell of a subject to produce a spliced product of the ATXN3 pre-mRNA.

In some aspects, described herein, is a method of treating, preventing, delaying of progress, or ameliorating symptoms of a disease or a condition associated with Ataxin 3 (ATXN3) expression level or activity level in a subject in need thereof, comprising administering a therapeutically effective amount of a small molecule splicing modulator compound disclosed herein (SMSM), wherein the SMSM binds to a pre-mRNA encoded by ATXN3 and modulates splicing of the ATXN3 pre-mRNA in a cell of the subject to produce a spliced product of the ATXN3 pre-mRNA, wherein the amount of full length ATXN3 is reduced.

### DETAILED DESCRIPTION

The invention is set out in the appended set of claims. In addition, any reference to methods of treatment in the subsequent paragraphs of this description is to be interpreted as references to the compounds, pharmaceutical compositions and medicaments of the present invention for use in a method for treatment of the human or animal body by therapy. Unless otherwise defined, all technical and scientific terms used herein have the same meaning as commonly understood by one of ordinary skill in the art to which this disclosure belongs. Although methods and materials similar or equivalent to those described herein can be used in the practice or testing of the present disclosure, suitable methods, and materials are described below.

### Definitions

The term "small molecule splicing modulator" or "SMSM" denotes a small molecule compound that binds to a cell component *(e.g.,* DNA, RNA, pre-mRNA, protein, RNP, snRNA, carbohydrates, lipids, co-factors, nutrients, and/or metabolites) and modulates splicing. For example, a SMSM can bind to a polynucleotide, *e.g.,* an RNA *(e.g.,* a pre-mRNA) with an aberrant splice site, resulting in steric modulation of the polynucleotide. For example, a SMSM can bind to a protein, e.g., a spliceosome protein or a ribonuclear protein, resulting in steric modulation of the protein. For example, a SMSM can bind to a spliceosome component, *e.g.,* a spliceosome protein or snRNA resulting in steric modulation of the spliceosome protein or snRNA. For example, a SMSM is a compound of Formula (I). The term "small molecule splicing modulator" or "SMSM" specifically excludes compounds consisting of oligonucleotides.

"Steric alteration," "steric modification," or "steric modulation" herein refers to changes in the spatial orientation of chemical moieties with respect to each other. A person of ordinary skill in the art would recognize steric mechanisms include, but are not limited to, steric hindrance, steric shielding, steric attraction, chain crossing, steric repulsions, steric inhibition of resonance, and steric inhibition of protonation.

Any open valency appearing on a carbon, oxygen, sulfur or nitrogen atom in the structures herein indicates the presence of a hydrogen, unless indicated otherwise.

The definitions described herein apply irrespective of whether the terms in question appear alone or in combination. It is contemplated that the definitions described herein can be appended to form chemically relevant combinations, such as *e.g.,* "heterocycloalkylaryl," "haloalkylheteroaryl," "arylalkylheterocycloalkyl," or "alkoxyalkyl." The last member of the combination is the radical which is binding to the rest of the molecule. The other members of the combination are attached to the binding radical in reversed order in respect of the literal sequence, *e.g.,* the combination arylalkylheterocycloalkyl refers to a heterocycloalkyl-radical which is substituted by an alkyl which is substituted by an aryl.

When indicating the number of substituents, the term "one or more" refers to the range from one substituent to the highest possible number of substitutions, *i.e.,* replacement of one hydrogen up to replacement of all hydrogens by substituents.

The term "optional" or "optionally" denotes that a subsequently described event or circumstance can but need not occur, and that the description includes instances where the event or circumstance occurs and instances in which it does not.

The term "substituent" denotes an atom or a group of atoms replacing a hydrogen atom on the parent molecule.

The term "substituted" denotes that a specified group bears one or more substituents. Where any group can carry multiple substituents and a variety of possible substituents is provided, the substituents are independently selected and need not to be the same. The term "unsubstituted" means that the specified group bears no substituents. The term "optionally substituted" means that the specified group is unsubstituted or substituted by one or more substituents, independently chosen from the group of possible substituents. When indicating the number of substituents, the term "one or more" means from one substituent to the highest possible number of substitutions, *i.e.,* replacement of one hydrogen up to replacement of all hydrogens by substituents.

The terms "compound(s) of this disclosure," "compound(s) of the present disclosure," "small molecule steric modulator," "small molecule splicing modulator," "steric modulator," "splicing modulator," "compounds that modify splicing," and "compounds modifying splicing" are interchangeably used herein and refer to compounds as disclosed herein and stereoisomers, tautomers, solvates, and salts *(e.g.,* pharmaceutically acceptable salts) thereof.

The following abbreviations are used throughout the specification: acetic acid (AcOH); ethyl acetate (EtOAc); butyl alcohol (n-BuOH); 1,2-dichloroethane (DCE); dichloromethane (CH₂Cl₂, DCM); diisopropylethylamine (Diipea); dimethylformamide (DMF); hydrogen chloride (HCl); methanol (MeOH); methoxymethyl bromide (MOMBr); N-methyl-2-pyrrolidone (NMP); methyl Iodide (MeI); n-propanol (n-PrOH); p-methoxybenzyl (PMB); triethylamine (Et₃N); [1,1'-Bis(diphenylphosphino)ferrocene] dichloropalladium(II); (Pd(dppf)Cl₂); sodium ethane thiolate (EtSNa); sodium acetate (NaOAc); sodium hydride (NaH); sodium hydroxide (NaOH); tetrahydropyran (THP); tetrahydrofuran (THF).

As used herein, C₁-Cₓ includes C₁-C₂, C₁-C₃... C₁-Cₓ. By way of example only, a group designated as "C₁-C₄" indicates that there are one to four carbon atoms in the moiety, *i.e.* groups containing 1 carbon atom, 2 carbon atoms, 3 carbon atoms or 4 carbon atoms. Thus, by way of example only, "C₁-C₄ alkyl" indicates that there are one to four carbon atoms in the alkyl group, *i.e.*, the alkyl group is selected from among methyl, ethyl, propyl, *iso*-propyl, *n*-butyl, *iso*-butyl, *sec-*butyl, and *t*-butyl.

The term "oxo" refers to the =O substituent.

"Carboxyl" refers to -COOH.

"Cyano" refers to -CN.

The term "thioxo" refers to the =S substituent.

"Amidinyl" refers to a radical of the formula -C(=NR^{a})-N(R^{a})₂ wherein each R^{a} is independently a hydrogen, a C₁-C₆ alkyl, C₁-C₆ haloalkyl, C₃-C₆cycloalkyl, or 3-6 membered heterocycloalkyl. In some embodiments, an amidinyl is C(=NH)NH₂. In some embodiments, an amidinyl is C(=NH)NH(C₁-C₆ alkyl).

The term "halo," "halogen," and "halide" are used interchangeably herein and denote fluoro, chloro, bromo, or iodo.

The term "alkyl" refers to a straight or branched hydrocarbon chain radical, having from one to twenty carbon atoms, and which is attached to the rest of the molecule by a single bond. An alkyl comprising up to 10 carbon atoms is referred to as a C₁-C₁₀ alkyl, likewise, for example, an alkyl comprising up to 6 carbon atoms is a C₁-C₆ alkyl. Alkyls (and other moieties defined herein) comprising other numbers of carbon atoms are represented similarly. Alkyl groups include, but are not limited to, C₁-C₁₀ alkyl, C₁-C₉ alkyl, C₁-C₈ alkyl, C₁-C₇ alkyl, C₁-C₆ alkyl, C₁-C₅ alkyl, C₁-C₄ alkyl, C₁-C₃ alkyl, C₁-C₂ alkyl, C₂-C₈ alkyl, C₃-C₈ alkyl and C₄-C₈ alkyl. Representative alkyl groups include, but are not limited to, methyl, ethyl, n-propyl, 1-methylethyl (i-propyl), n-butyl, *i-*butyl, *s*-butyl, *n*-pentyl, 1,1-dimethylethyl (*t*-butyl), 3-methylhexyl, 2-methylhexyl, 1-ethyl-propyl, and the like. In some embodiments, the alkyl is methyl or ethyl. In some embodiments, the alkyl is - CH(CH₃)₂ or -C(CH₃)₃. Unless stated otherwise specifically in the specification, an alkyl group may be optionally substituted as described below. "Alkylene" or "alkylene chain" refers to a straight or branched divalent hydrocarbon chain linking the rest of the molecule to a radical group. In some embodiments, the alkylene is -CH₂-, -CH₂CH₂-, or -CH₂CH₂CH₂-. In some embodiments, the alkylene is -CH₂-. In some embodiments, the alkylene is -CH₂CH₂-. In some embodiments, the alkylene is -CH₂CH₂CH₂-.

The term "alkoxy" refers to a radical of the formula -OR where R is an alkyl radical as defined. Unless stated otherwise specifically in the specification, an alkoxy group may be optionally substituted as described below. Representative alkoxy groups include, but are not limited to, methoxy, ethoxy, propoxy, butoxy, pentoxy. In some embodiments, the alkoxy is methoxy. In some embodiments, the alkoxy is ethoxy.

The term "alkylamino" refers to a radical of the formula -NHR or -NRR where each R is, independently, an alkyl radical as defined above. Unless stated otherwise specifically in the specification, an alkylamino group may be optionally substituted as described below.

The term "alkenyl" refers to a type of alkyl group in which at least one carbon-carbon double bond is present. In one embodiment, an alkenyl group has the formula -C(R)=CR₂, wherein R refers to the remaining portions of the alkenyl group, which may be the same or different. In some embodiments, R is H or an alkyl. In some embodiments, an alkenyl is selected from ethenyl *(i.e.,* vinyl), propenyl *(i.e.,* allyl), butenyl, pentenyl, pentadienyl, and the like. Non-limiting examples of an alkenyl group include -CH=CH₂, -C(CH₃)=CH₂, -CH=CHCH₃, -C(CH₃)=CHCH₃, and - CH₂CH=CH₂.

The term "alkynyl" refers to a type of alkyl group in which at least one carbon-carbon triple bond is present. In one embodiment, an alkenyl group has the formula -C≡C-R, wherein R refers to the remaining portions of the alkynyl group. In some embodiments, R is H or an alkyl. In some embodiments, an alkynyl is selected from ethynyl, propynyl, butynyl, pentynyl, hexynyl, and the like. Non-limiting examples of an alkynyl group include -C≡CH, -C≡CCH₃ -C≡CCH₂CH₃, -CH₂C≡CH.

The term "aromatic" refers to a planar ring having a delocalized π-electron system containing 4n+2 π electrons, where n is an integer. Aromatics can be optionally substituted. The term "aromatic" includes both aryl groups *(e.g.,* phenyl, naphthalenyl) and heteroaryl groups *(e.g.,* pyridinyl, furanyl, quinolinyl).

The term "aryl" refers to a radical derived from a hydrocarbon ring system comprising at least one aromatic ring wherein each of the atoms forming the ring is a carbon atom. Aryl groups can be optionally substituted. Examples of aryl groups include, but are not limited to phenyl, and naphthyl. In some embodiments, the aryl is phenyl. Depending on the structure, an aryl group can be a monoradical or a diradical *(i.e.,* an arylene group). Unless stated otherwise specifically in the specification, the term "aryl" or the prefix "ar-"(such as in "aralkyl") is meant to include aryl radicals that are optionally substituted. In some embodiments, an aryl group is partially reduced to form a cycloalkyl group defined herein. In some embodiments, an aryl group is fully reduced to form a cycloalkyl group defined herein.

The term "haloalkyl" denotes an alkyl group wherein at least one of the hydrogen atoms of the alkyl group has been replaced by same or different halogen atoms, particularly fluoro atoms. Examples of haloalkyl include monofluoro-, difluoro-or trifluoro-methyl, -ethyl or -propyl, for example, 3,3,3-trifluoropropyl, 2-fluoroethyl, 2,2,2-trifluoroethyl, fluoromethyl, or trifluoromethyl. The term "perhaloalkyl" denotes an alkyl group where all hydrogen atoms of the alkyl group have been replaced by the same or different halogen atoms. Exemplary haloalkyl groups further include trifluoromethyl, difluoromethyl, fluoromethyl, trichloromethyl, 2,2,2-trifluoroethyl, 1,2-difluoroethyl, 3-bromo-2-fluoropropyl, 1,2-dibromoethyl, and the like. Unless stated otherwise specifically in the specification, a haloalkyl group may be optionally substituted.

"Hydroxyalkyl" refers to an alkyl radical, as defined above, that is substituted by one or more hydroxyls. In some embodiments, the alkyl is substituted with one hydroxyl. In some embodiments, the alkyl is substituted with one, two, or three hydroxyls. Hydroxyalkyl include, for example, hydroxymethyl, hydroxyethyl, hydroxypropyl, hydroxybutyl, or hydroxypentyl. In some embodiments, the hydroxyalkyl is hydroxymethyl.

"Aminoalkyl" refers to an alkyl radical, as defined above, that is substituted by one or more amines. In some embodiments, the alkyl is substituted with one amine. In some embodiments, the alkyl is substituted with one, two, or three amines. Aminoalkyl include, for example, aminomethyl, aminoethyl, aminopropyl, aminobutyl, or aminopentyl. In some embodiments, the aminoalkyl is aminomethyl.

"Cyanoalkyl" refers to an alkyl radical, as defined above, that is substituted by one or more cyano groups. In some embodiments, the alkyl is substituted with one cyano group. In some embodiments, the alkyl is substituted with one, two, or three cyano groups. Aminoalkyl include, for example, cyanomethyl, cyanoethyl, cyanopropyl, cyanobutyl, or cyanopentyl.

The term "haloalkoxy" denotes an alkoxy group wherein at least one of the hydrogen atoms of the alkoxy group has been replaced by same or different halogen atoms, particularly fluoro atoms. Examples of haloalkoxyl include monofluoro-, difluoro-or trifluoro-methoxy, -ethoxy or -propoxy, for example, 3,3,3-trifluoropropoxy, 2-fluoroethoxy, 2,2,2-trifluoroethoxy, fluoromethoxy, or trifluoromethoxy. The term "perhaloalkoxy" denotes an alkoxy group where all hydrogen atoms of the alkoxy group have been replaced by the same or different halogen atoms. Examples of haloalkoxyl further include trifluoromethoxy, difluoromethoxy, fluoromethoxy, trichloromethoxy, 2,2,2-trifluoroethoxy, 1,2-difluoroethoxy, 3-bromo-2-fluoropropoxy, 1,2-dibromoethoxy, and the like. Unless stated otherwise specifically in the specification, a haloalkoxy group may be optionally substituted.

The term "bicyclic ring system" denotes two rings which are fused to each other via a common single or double bond (annelated bicyclic ring system), via a sequence of three or more common atoms (bridged bicyclic ring system) or via a common single atom (spiro bicyclic ring system). Bicyclic ring systems can be saturated, partially unsaturated, unsaturated, or aromatic. Bicyclic ring systems can comprise heteroatoms selected from N, O, and S.

The terms "carbocyclic" or "carbocycle" refer to a ring or ring system where the atoms forming the backbone of the ring are all carbon atoms. The term thus distinguishes carbocyclic from "heterocyclic" rings or "heterocycles" in which the ring backbone contains at least one atom which is different from carbon. In some embodiments, at least one of the two rings of a bicyclic carbocycle is aromatic. In some embodiments, both rings of a bicyclic carbocycle are aromatic. Carbocycle includes cycloalkyl and aryl.

The term "cycloalkyl" refers to a monocyclic or polycyclic non-aromatic radical, wherein each of the atoms forming the ring *(i.e.,* skeletal atoms) is a carbon atom. In some embodiments, cycloalkyls are saturated or partially unsaturated. In some embodiments, cycloalkyls are spirocyclic or bridged compounds. In some embodiments, cycloalkyls are fused with an aromatic ring (in which case the cycloalkyl is bonded through a non-aromatic ring carbon atom). Cycloalkyl groups include groups having from 3 to 10 ring atoms. Representative cycloalkyls include, but are not limited to, cycloalkyls having from three to ten carbon atoms, from three to eight carbon atoms, from three to six carbon atoms, or from three to five carbon atoms. Monocyclic cycloalkyl radicals include, for example, cyclopropyl, cyclobutyl, cyclopentyl, cyclohexyl, cycloheptyl, and cyclooctyl. In some embodiments, the monocyclic cycloalkyl is cyclopropyl, cyclobutyl, cyclopentyl or cyclohexyl. In some embodiments, the monocyclic cycloalkyl is cyclopentenyl or cyclohexenyl. In some embodiments, the monocyclic cycloalkyl is cyclopentenyl. Polycyclic radicals include, for example, adamantyl, 1,2-dihydronaphthalenyl, 1,4-dihydronaphthalenyl, tetrainyl, decalinyl, 3,4-dihydronaphthalenyl-1(2H)-one, spiro[2.2]pentyl, norbornyl and bicycle[1.1.1]pentyl. Unless otherwise stated specifically in the specification, a cycloalkyl group may be optionally substituted.

The term "bridged" refers to any ring structure with two or more rings that contains a bridge connecting two bridgehead atoms. The bridgehead atoms are defined as atoms that are the part of the skeletal framework of the molecule and which are bonded to three or more other skeletal atoms. In some embodiments, the bridgehead atoms are C, N, or P. In some embodiments, the bridge is a single atom or a chain of atoms that connects two bridgehead atoms. In some embodiments, the bridge is a valence bond that connects two bridgehead atoms. In some embodiments, the bridged ring system is cycloalkyl. In some embodiments, the bridged ring system is heterocycloalkyl.

The term "fused" refers to any ring structure described herein which is fused to an existing ring structure. When the fused ring is a heterocyclyl ring or a heteroaryl ring, any carbon atom on the existing ring structure which becomes part of the fused heterocyclyl ring or the fused heteroaryl ring may be replaced with one or more N, S, and O atoms. The non-limiting examples of fused heterocyclyl or heteroaryl ring structures include 6-5 fused heterocycle, 6-6 fused heterocycle, 5-6 fused heterocycle, 5-5 fused heterocycle, 7-5 fused heterocycle, and 5-7 fused heterocycle.

The term "fluoroalkyl" refers to an alkyl in which one or more hydrogen atoms are replaced by a fluorine atom. In one aspect, a fluoroalkyl is a C₁-C₆ fluoroalkyl. In some embodiments, a fluoroalkyl is selected from trifluoromethyl, difluoromethyl, fluoromethyl, 2,2,2-trifluoroethyl, 1-fluoromethyl-2-fluoroethyl, and the like.

The term "heteroalkyl" refers to an alkyl group in which one or more skeletal atoms of the alkyl are selected from an atom other than carbon, *e.g.,* oxygen, nitrogen *(e.g.,* -NH-, -N(alkyl)-, or - N(aryl)-), sulfur (*e.g.*, -S-, -S(=O)-, or -S(=O)₂-), or combinations thereof. In some embodiments, a heteroalkyl is attached to the rest of the molecule at a carbon atom of the heteroalkyl. In some embodiments, a heteroalkyl is attached to the rest of the molecule at a heteroatom of the heteroalkyl. In some embodiments, a heteroalkyl is a C₁-C₆ heteroalkyl. Representative heteroalkyl groups include, but are not limited to -OCH₂OMe, -OCH₂CH₂OH, -OCH₂CH₂OMe, or - OCH₂CH₂OCH₂CH₂NH₂. In some embodiments, a heteroalkyl contains one skeletal heteroatom. In some embodiments, a heteroalkyl contains 1-3 skeletal heteroatoms.

The term "heteroalkylene" refers to an alkyl radical as described above where one or more carbon atoms of the alkyl is replaced with a O, N or S atom. "Heteroalkylene" or "heteroalkylene chain" refers to a straight or branched divalent heteroalkyl chain linking the rest of the molecule to a radical group. Unless stated otherwise specifically in the specification, the heteroalkyl or heteroalkylene group may be optionally substituted as described below. Representative heteroalkylene groups include, but are not limited to -OCH₂CH₂O-, -OCH₂CH₂OCH₂CH₂O-, or - OCH₂CH₂OCH₂CH₂OCH₂CH₂O-.

The term "heterocycloalkyl" refers to a cycloalkyl group that includes at least one heteroatom selected from nitrogen, oxygen, and sulfur. Unless stated otherwise specifically in the specification, the heterocycloalkyl radical may be a monocyclic, or bicyclic ring system, which may include fused (when fused with an aryl or a heteroaryl ring, the heterocycloalkyl is bonded through a non-aromatic ring atom) or bridged ring systems. In some embodiments, a heterocycloalkyl is monocyclic. In some embodiments, a heterocycloalkyl is bicyclic. In some embodiments, a heterocycloalkyl is partially saturated. In some embodiments, a heterocycloalkyl is fully saturated. The nitrogen, carbon, or sulfur atoms in the heterocyclyl radical may be optionally oxidized. The nitrogen atom may be optionally quaternized. The heterocycloalkyl radical is partially or fully saturated. Examples of heterocycloalkyl radicals include, but are not limited to, dioxolanyl, thienyl[1,3]dithianyl, tetrahydroquinolyl, tetrahydroisoquinolyl, decahydroquinolyl, decahydroisoquinolyl, imidazolinyl, imidazolidinyl, isothiazolidinyl, isoxazolidinyl, morpholinyl, octahydroindolyl, octahydroisoindolyl, 2-oxopiperazinyl, 2-oxopiperidinyl, 2-oxopyrrolidinyl, oxazolidinyl, piperidinyl, piperazinyl, 4-piperidonyl, pyrrolidinyl, pyrazolidinyl, quinuclidinyl, thiazolidinyl, tetrahydrofuryl, trithianyl, tetrahydropyranyl, thiomorpholinyl, thiamorpholinyl, 1-oxo-thiomorpholinyl, 1,1-dioxo-thiomorpholinyl. The term heterocycloalkyl also includes all ring forms of carbohydrates, including but not limited to monosaccharides, disaccharides, and oligosaccharides. Unless otherwise noted, heterocycloalkyls have from 2 to 12 carbons in the ring. In some embodiments, heterocycloalkyls have from 2 to 10 carbons in the ring. In some embodiments, heterocycloalkyls have from 2 to 10 carbons in the ring and 1 or 2 N atoms. In some embodiments, heterocycloalkyls have from 2 to 10 carbons in the ring and 3 or 4 N atoms. In some embodiments, heterocycloalkyls have from 2 to 12 carbons, 0-2 N atoms, 0-2 O atoms, 0-2 P atoms, and 0-1 S atoms in the ring. In some embodiments, heterocycloalkyls have from 2 to 12 carbons, 1-3 N atoms, 0-1 O atoms, and 0-1 S atoms in the ring. It is understood that when referring to the number of carbon atoms in a heterocycloalkyl, the number of carbon atoms in the heterocycloalkyl is not the same as the total number of atoms (including the heteroatoms) that make up the heterocycloalkyl (i.e. skeletal atoms of the heterocycloalkyl ring). Unless stated otherwise specifically in the specification, a heterocycloalkyl group may be optionally substituted.

The term "heterocycle" or "heterocyclic" refers to heteroaromatic rings (also known as heteroaryls) and heterocycloalkyl rings (also known as heteroalicyclic groups) that includes at least one heteroatom selected from nitrogen, oxygen and sulfur, wherein each heterocyclic group has from 3 to 12 atoms in its ring system, and with the proviso that any ring does not contain two adjacent O or S atoms. In some embodiments, heterocycles are monocyclic, bicyclic, polycyclic, spirocyclic or bridged compounds. Non-aromatic heterocyclic groups (also known as heterocycloalkyls) include rings having 3 to 12 atoms in its ring system and aromatic heterocyclic groups include rings having 5 to 12 atoms in its ring system. The heterocyclic groups include benzo-fused ring systems. Examples of non-aromatic heterocyclic groups are pyrrolidinyl, tetrahydrofuranyl, dihydrofuranyl, tetrahydrothienyl, oxazolidinonyl, tetrahydropyranyl, dihydropyranyl, tetrahydrothiopyranyl, piperidinyl, morpholinyl, thiomorpholinyl, thioxanyl, piperazinyl, aziridinyl, azetidinyl, oxetanyl, thietanyl, homopiperidinyl, oxepanyl, thiepanyl, oxazepinyl, diazepinyl, thiazepinyl, 1,2,3,6-tetrahydropyridinyl, pyrrolin-2-yl, pyrrolin-3-yl, indolinyl, 2H-pyranyl, 4H-pyranyl, dioxanyl, 1,3-dioxolanyl, pyrazolinyl, dithianyl, dithiolanyl, dihydropyranyl, dihydrothienyl, dihydrofuranyl, pyrazolidinyl, imidazolinyl, imidazolidinyl, 3-azabicyclo[3.1.0]hexanyl, 3-azabicyclo[4.1.0]heptanyl, ₃ h-indolyl, indolin-2-onyl, isoindolin-1-onyl, isoindoline-1,3-dionyl, 3,4-dihydroisoquinolin-1(2H)-onyl, 3,4-dihydroquinolin-2(1H)-onyl, isoindoline-1,3-dithionyl, benzo[d]oxazol-2(3H)-onyl, 1H-benzo[d]imidazol-2(3H)-onyl, benzo[d]thiazol-2(3H)-onyl, and quinolizinyl. Examples of aromatic heterocyclic groups are pyridinyl, imidazolyl, pyrimidinyl, pyrazolyl, triazolyl, pyrazinyl, tetrazolyl, furyl, thienyl, isoxazolyl, thiazolyl, oxazolyl, isothiazolyl, pyrrolyl, quinolinyl, isoquinolinyl, indolyl, benzimidazolyl, benzofuranyl, cinnolinyl, indazolyl, indolizinyl, phthalazinyl, pyridazinyl, triazinyl, isoindolyl, pteridinyl, purinyl, oxadiazolyl, thiadiazolyl, furazanyl, benzofurazanyl, benzothiophenyl, benzothiazolyl, benzoxazolyl, quinazolinyl, quinoxalinyl, naphthyridinyl, and furopyridinyl. The foregoing groups are either C-attached (or C-linked) or *N-*attached where such is possible. For instance, a group derived from pyrrole includes both pyrrol-1-yl (*N*-attached) or pyrrol-3-yl (C-attached). Further, a group derived from imidazole includes imidazol-1-yl or imidazol-3-yl (both *N*-attached) or imidazol-2-yl, imidazol-4-yl or imidazol-5-yl (all C-attached). The heterocyclic groups include benzo-fused ring systems. Non-aromatic heterocycles are optionally substituted with one or two oxo (=O) moieties, such as pyrrolidin-2-one. In some embodiments, at least one of the two rings of a bicyclic heterocycle is aromatic. In some embodiments, both rings of a bicyclic heterocycle are aromatic.

The term "heteroaryl" refers to an aryl group that includes one or more ring heteroatoms selected from nitrogen, oxygen, and sulfur. The heteroaryl can be monocyclic or bicyclic. Illustrative examples of monocyclic heteroaryls include pyridinyl, imidazolyl, pyrimidinyl, pyrazolyl, triazolyl, pyrazinyl, tetrazolyl, furyl, thienyl, isoxazolyl, thiazolyl, oxazolyl, isothiazolyl, pyrrolyl, pyridazinyl, triazinyl, oxadiazolyl, thiadiazolyl, furazanyl, indolizine, indole, benzofuran, benzothiophene, indazole, benzimidazole, purine, quinolizine, quinoline, isoquinoline, cinnoline, phthalazine, quinazoline, quinoxaline, 1,8-naphthyridine, and pteridine. Illustrative examples of monocyclic heteroaryls include pyridinyl, imidazolyl, pyrimidinyl, pyrazolyl, triazolyl, pyrazinyl, tetrazolyl, furyl, thienyl, isoxazolyl, thiazolyl, oxazolyl, isothiazolyl, pyrrolyl, pyridazinyl, triazinyl, oxadiazolyl, thiadiazolyl, and furazanyl. Illustrative examples of bicyclic heteroaryls include indolizine, indole, benzofuran, benzothiophene, indazole, benzimidazole, purine, quinolizine, quinoline, isoquinoline, cinnoline, phthalazine, quinazoline, quinoxaline, 1,8-naphthyridine, and pteridine. In some embodiments, heteroaryl is pyridinyl, pyrazinyl, pyrimidinyl, thiazolyl, thienyl, thiadiazolyl or furyl. In some embodiments, a heteroaryl contains 0-6 N atoms in the ring. In some embodiments, a heteroaryl contains 1-4 N atoms in the ring. In some embodiments, a heteroaryl contains 4-6 N atoms in the ring. In some embodiments, a heteroaryl contains 0-4 N atoms, 0-1 O atoms, 0-1 P atoms, and 0-1 S atoms in the ring. In some embodiments, a heteroaryl contains 1-4 N atoms, 0-1 O atoms, and 0-1 S atoms in the ring. In some embodiments, heteroaryl is a C₁-C₉ heteroaryl. In some embodiments, monocyclic heteroaryl is a C₁-C₅ heteroaryl. In some embodiments, monocyclic heteroaryl is a 5-membered or 6-membered heteroaryl. In some embodiments, a bicyclic heteroaryl is a C₆-C₉ heteroaryl. In some embodiments, a heteroaryl group is partially reduced to form a heterocycloalkyl group defined herein. In some embodiments, a heteroaryl group is fully reduced to form a heterocycloalkyl group defined herein.

The term "moiety" refers to a specific segment or functional group of a molecule. Chemical moieties are often recognized chemical entities embedded in or appended to a molecule.

The term "optionally substituted" or "substituted" means that the referenced group is optionally substituted with one or more additional group(s) individually and independently selected from D, halogen, -CN, -NH₂, -NH(alkyl), -N(alkyl)₂, -OH, -CO₂H, -CO₂alkyl, -C(=O)NH₂, - C(=O)NH(alkyl), -C(=O)N(alkyl)₂, -S(=O)₂NH₂, -S(=O)₂NH(alkyl), -S(=O)₂N(alkyl)₂, alkyl, cycloalkyl, fluoroalkyl, heteroalkyl, alkoxy, fluoroalkoxy, heterocycloalkyl, aryl, heteroaryl, aryloxy, alkylthio, arylthio, alkylsulfoxide, arylsulfoxide, alkylsulfone, and arylsulfone. In some other embodiments, optional substituents are independently selected from D, halogen, -CN, -NH₂, - NH(CH₃), -N(CH₃)₂, -OH, -CO₂H, -CO₂(C₁-C₄ alkyl), -C(=O)NH₂, -C(=O)NH(C₁-C₄ alkyl), - C(=O)N(C₁-C₄ alkyl)₂, -S(=O)₂NH₂, -S(=O)₂NH(C₁-C₄ alkyl), -S(=O)₂N(C₁-C₄ alkyl)₂, C₁-C₄ alkyl, C₃-C₆ cycloalkyl, C₁-C₄ fluoroalkyl, C₁-C₄ heteroalkyl, C₁-C₄ alkoxy, C₁-C₄ fluoroalkoxy, -SC₁-C₄ alkyl, -S(=O)C₁-C₄ alkyl, and -S(=O)₂(C₁-C₄ alkyl). In some embodiments, optional substituents are independently selected from D, halogen, -CN, -NH₂, -OH, -NH(CH₃), -N(CH₃)₂, - NH(cyclopropyl), -CH₃, -CH₂CH₃, -CF₃, -OCH₃, and -OCF₃. In some embodiments, substituted groups are substituted with one or two of the preceding groups. In some embodiments, an optional substituent on an aliphatic carbon atom (acyclic or cyclic) includes oxo (=O).

The term "tautomer" refers to a proton shift from one atom of a molecule to another atom of the same molecule. The compounds presented herein may exist as tautomers. Tautomers are compounds that are interconvertible by migration of a hydrogen atom, accompanied by a switch of a single bond and adjacent double bond. In bonding arrangements where tautomerization is possible, a chemical equilibrium of the tautomers will exist. All tautomeric forms of the compounds disclosed herein are contemplated. The exact ratio of the tautomers depends on several factors, including temperature, solvent, and pH. Some examples of tautomeric interconversions include:

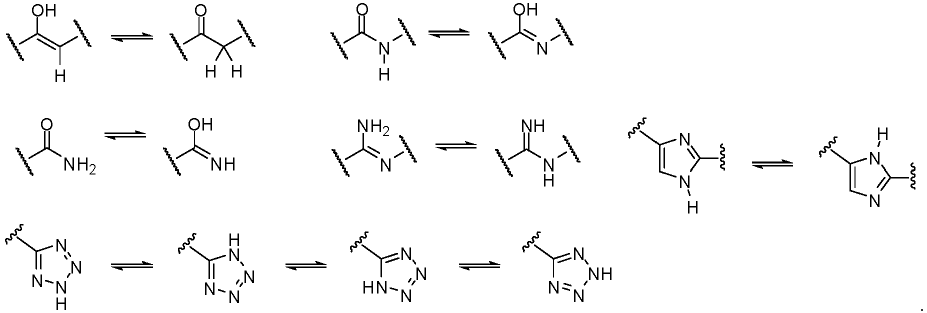

The terms "administer," "administering," "administration," and the like, as used herein, refer to the methods that may be used to enable delivery of compounds or compositions to the desired site of biological action. These methods include but are not limited to oral routes (p.o.), intraduodenal routes (i.d.), parenteral injection (including intravenous (i.v.), subcutaneous (s.c.), intraperitoneal (i.p.), intramuscular (i.m.), intravascular or infusion (inf.)), topical (top.) and rectal (p.r.) administration. Those of skill in the art are familiar with administration techniques that can be employed with the compounds and methods described herein. In some embodiments, the compounds and compositions described herein are administered orally.

The terms "co-administration" or the like, as used herein, are meant to encompass administration of the selected therapeutic agents to a single patient and are intended to include treatment regimens in which the agents are administered by the same or different route of administration or at the same or different time.

The term "subject" or "patient" encompasses mammals. Examples of mammals include, but are not limited to, any member of the mammalian class: humans, non-human primates such as chimpanzees, and other apes and monkey species; farm animals such as cattle, horses, sheep, goats, swine; domestic animals such as rabbits, dogs, and cats; laboratory animals including rodents, such as rats, mice and guinea pigs, and the like. In one aspect, the mammal is a human. The term "animal" as used herein comprises human beings and non-human animals. In one embodiment, a "non-human animal" is a mammal, for example a rodent such as rat or a mouse. In one embodiment, a non-human animal is a mouse.

The term "pharmaceutically acceptable" denotes an attribute of a material which is useful in preparing a pharmaceutical composition that is generally safe, non toxic, and neither biologically nor otherwise undesirable and is acceptable for veterinary as well as human pharmaceutical use. "Pharmaceutically acceptable" can refer a material, such as a carrier or diluent, which does not abrogate the biological activity or properties of the compound, and is relatively nontoxic, *i.e*., the material may be administered to an individual without causing undesirable biological effects or interacting in a deleterious manner with any of the components of the composition in which it is contained.

The terms "pharmaceutically acceptable excipient", "pharmaceutically acceptable carrier" and "therapeutically inert excipient" can be used interchangeably and denote any pharmaceutically acceptable ingredient in a pharmaceutical composition having no therapeutic activity and being nontoxic to the subject administered, such as disintegrators, binders, fillers, solvents, buffers, tonicity agents, stabilizers, antioxidants, surfactants, carriers, diluents, excipients, preservatives or lubricants used in formulating pharmaceutical products.

The term "pharmaceutically acceptable salts" denotes salts which are not biologically or otherwise undesirable. Pharmaceutically acceptable salts include both acid and base addition salts. A "pharmaceutically acceptable salt" can refer to a formulation of a compound that does not cause significant irritation to an organism to which it is administered and/or does not abrogate the biological activity and properties of the compound. In some embodiments, pharmaceutically acceptable salts are obtained by reacting a SMSM compound of the present disclosure with acids. Pharmaceutically acceptable salts are also obtained by reacting a compound of the present disclosure with a base to form a salt.

As used herein, a "small molecular weight compound" can be used interchangeably with "small molecule" or "small organic molecule." Small molecules refer to compounds other than peptides or oligonucleotides; and typically have molecular weights of less than about 2000 Daltons, *e.g.,* less than about 900 Daltons.

### Small Molecule Splicing Modulators (SMSMs)

It has now been found that compounds of this disclosure, and pharmaceutically acceptable compositions thereof, are effective as agents for use in treating, preventing, or ameliorating a disease or a condition associated with a target RNA. The present disclosure provides the unexpected discovery that certain small chemical molecules can modify splicing events in pre-mRNA molecules, herein referred to as small molecule splicing modulators (SMSMs). These SMSMs can modulate specific splicing events in specific pre-mRNA molecules. The small molecules of this disclosure are different from and are not related to antisense or antigene oligonucleotides.

In one aspect, a SMSM described herein is a compound of Formula (I), or a pharmaceutically acceptable salt thereof: 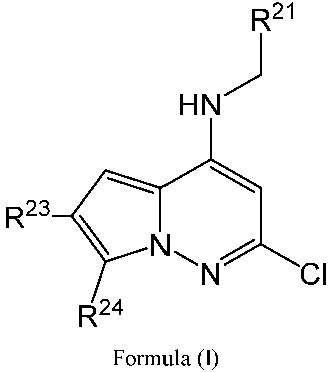 wherein,
- R²¹ is furanyl or thiazolyl, each of which is unsubstituted or substituted with 1, 2, or 3, independently selected R^{1A} groups; each R^{1A} is independently selected from halo, CN, NO₂, C₁₋₆ alkyl, C₂₋₆ alkenyl, C₂₋₆ alkynyl, C₁₋₆ haloalkyl, C₁₋₆ alkoxy, -C(=O)OH, -C(=O)C₁₋₆ alkyl, - C(=O)C₁₋₆ haloalkyl, and -C(=O)C₁₋₆ alkoxy;
- R²³ is selected from the group consisting of H, azido, halo, CN, NO₂, C₁₋₆alkyl, C₂₋₆ alkenyl, C₂₋₆ alkynyl, C₁₋₆heteroalkyl, -(C₁₋₆ alkylene)-C₃₋₁₀ cycloalkyl, -(C₁₋₆ alkylene)-4-10 membered heterocycloalkyl, -(C₁₋₆ heteroalkylene)-C₃₋₁₀ cycloalkyl, -(C₁₋₆ heteroalkylene)-4-10 membered heterocycloalkyl, C₃₋₁₀ cycloalkyl, C₆₋₁₀ aryl, 5-10 membered heteroaryl, 4- 10 membered heterocycloalkyl, OR^{a3}, SR^{a3}, C(=O)R^{b3}, C(=O)OR^{b3}, NR^{c3}R^{d3}, C(=O)NR^{c3}R^{d3}, - OC(=O)NR^{c3}R^{d3}, NR^{c3}C(=O)R^{b3}, NR^{c3}C(=O)OR^{b3}, NR^{c3}C(=O)NR^{c3}R^{d3}, NR^{c3}S(=O)₂R^{b3}, NR^{c3}S(=O)₂NR^{c3}R^{d3}, S(O)NR^{c3}R^{d3}, and S(O)₂NR^{c3}R^{d3}, wherein the C₁₋₆ alkyl, C₂₋₆ alkenyl, C₂₋₆ alkynyl, C₁₋₆heteroalkyl, C₁₋₆ alkylene, C₁₋₆ heteroalkylene, C₃₋₁₀ cycloalkyl, C₆₋₁₀ aryl, 5-10 membered heteroaryl, and 4-10 membered heterocycloalkyl are each optionally substituted by 1, 2, 3, or 4 independently selected R²⁰ groups;
- R²⁴ is selected from the group consisting of H, azido, halo, CN, NO₂, C₁₋₆ alkyl, C₂₋₆ alkenyl, C₂₋₆ alkynyl, C₃₋₁₀ cycloalkyl, C₆₋₁₀ aryl, 5-10 membered heteroaryl, 4- 10 membered heterocycloalkyl, OR^{a4}, C(=O)R^{b4}, C(=O)OR^{b4}, NR^{c4}R^{d4}, C(=O)NR^{c4}R^{d4}, - OC(=O)NR^{c4}R^{d4}, NR^{c4}C(=O)R^{b4}, NR^{c4}C(=O)OR^{b4}, NR^{c4}C(=O)NR^{c4}R^{d4}, NR^{c4}S(=O)₂R^{b4}, NR^{c4}S(=O)₂NR^{c4}R^{d4}, S(O)NR^{c4}R^{d4}, and S(O)₂NR^{c4}R^{d4}, wherein the C₁₋₆ alkyl, C₃₋₁₀ cycloalkyl, C₂₋₆ alkenyl, C₂₋₆ alkynyl, C₆₋₁₀ aryl, 5-10 membered heteroaryl, and 4-10 membered heterocycloalkyl are each optionally substituted by 1, 2, 3, or 4 independently selected R²⁰ groups;
- each R^{a3}, R^{b3}, R^{c3}, R^{d3}, R^{a4}, R^{b4}, R^{c4}, and R^{d4}, is independently selected from the group consisting of H, C₁₋₆ alkyl, C₂₋₆ alkenyl, C₂₋₆ alkynyl, C₁₋₆ hydroxyalkyl, C₁₋₆ haloalkyl, C₁₋₆ alkoxy, - (C₁₋₆ alkylene)-C₁₋₆ alkoxy, C₃₋₁₀ cycloalkyl, -(C₁₋₆ alkylene)-C₃₋₁₀ cycloalkyl, C₆₋₁₀ aryl, 5-10 membered heteroaryl, and 4-10 membered heterocycloalkyl, wherein the C₁₋₆ alkyl, C₂₋₆ alkenyl, C₂₋₆ alkynyl, C₃₋₁₀ cycloalkyl, -(C₁₋₆ alkylene)-C₃₋₁₀ cycloalkyl, C₆₋₁₀ aryl, 5-10 membered heteroaryl, and 4-10 membered heterocycloalkyl are each optionally substituted by 1, 2, 3, or 4 independently selected R²⁰ groups;
   or R^{c3} and R^{d3} together with the N atom to which they are connected, come together to form a 5-10 membered heteroaryl or 4-10 membered heterocycloalkyl ring, each optionally substituted by 1, 2, 3, or 4 independently selected R²⁰ groups;
   or R^{c4} and R^{d4} together with the N atom to which they are connected, come together to form a 5-10 membered heteroaryl or 4-10 membered heterocycloalkyl ring, each optionally substituted by 1, 2, 3, or 4 independently selected R²⁰ groups;
      and
- each R²⁰ is independently selected from the group consisting of OH, SH, CN, NO₂, halo, oxo, C₁₋₄ alkyl, C₂₋₄ alkenyl, C₂₋₄ alkynyl, C₁₋₄ haloalkyl, C₁₋₄ cyanoalkyl, C₁₋₄ hydroxyalkyl, C₁₋₄ alkoxy, -(C₁₋₄ alkyl)-(C₁₋₄ alkoxy), -(C₁₋₄ alkoxy)-(C₁₋₄ alkoxy), C₁₋₄ haloalkoxy, C₃₋₆ cycloalkyl, phenyl, 5-6 membered heteroaryl, 4-6 membered heterocycloalkyl, amino, C₁₋₄ alkylamino, di(C₁₋₄ alkyl)amino, carbamyl, C₁₋₄ alkylcarbamyl, di(C₁₋₄ alkyl)carbamyl, carbamoyl, C₁₋₄ alkylcarbamoyl, di(C₁₋₄ alkyl)carbamoyl, C₁₋₄ alkylcarbonyl, C₁₋₄ alkoxycarbonyl, C₁₋₄ alkylcarbonylamino, C₁₋₄ alkylsulfonylamino, aminosulfonyl, C₁₋₄ alkylaminosulfonyl, di(C₁₋₄ alkyl)aminosulfonyl, aminosulfonylamino, C₁₋₄ alkylaminosulfonylamino, di(C₁₋₄ alkyl)aminosulfonylamino, aminocarbonylamino, C₁₋₄ alkylaminocarbonylamino, di(C₁₋₄ alkyl)aminocarbonylamino, and amidinyl.

In some embodiments of a compound of Formula (I) or a pharmaceutically acceptable salt thereof,
- R²¹ is furanyl or thiazolyl, each of which is unsubstituted or substituted with 1, 2, or 3, independently selected R^{1A} groups; wherein each R^{1A} is independently selected from halo, CN, NO₂, alkyl, alkenyl, C₂₋₆ alkynyl, alkoxy, -C(=O)OH, an ether group, or an ester group, each of which is unsubstituted or substituted;
- R²³ is selected from the group consisting of H, oxo, azido, halo, CN, NO₂, alkyl, heteroalkyl, alkenyl, alkynyl, cycloalkyl, aryl, heteroaryl, heterocycloalkyl, OR^{a3}, SR^{a3}, C(=O)R^{b3}, C(=O)OR^{b3}, NR^{c3}R^{d3}, C(=O)NR^{c3}R^{d3}, -OC(=O)NR^{c3}R^{d3}, NR^{c3}C(=O)R^{b3}, NR^{c3}C(=O)OR^{b3}, NR^{c3}C(=O)NR^{c3}R^{d3}, NR^{c3}S(=O)₂R^{b3}, NR^{c3}S(=O)₂NR^{c3}R^{d3}, S(O)NR^{c3}R^{d3}, and S(O)₂NR^{c3}R^{d3}, wherein the alkyl, cycloalkyl, aryl, heteroaryl, and heterocycloalkyl are each unsubstituted or substituted with 1, 2, 3, or 4 independently selected R²⁰ groups;
- R²⁴ is selected from the group consisting of H, oxo, azido, halo, CN, NO₂, alkyl, heteroalkyl, alkenyl, alkynyl, cycloalkyl, aryl, heteroaryl, heterocycloalkyl, OR^{a4}, C(=O)R^{b4}, C(=O)OR^{b4}, NR^{c4}R^{d4}, C(=O)NR^{c4}R^{d4}, - OC(=O)NR^{c4}R^{d4}, NR^{c4}C(=O)R^{b4}, NR^{c4}C(=O)OR^{b4}, NR^{c4}C(=O)NR^{c4}R^{d4}, NR^{c4}S(=O)₂R^{b4}, NR^{c4}S(=O)₂NR^{c4}R^{d4}, S(O)NR^{c4}R^{d4}, and S(O)₂NR^{c4}R^{d4}, wherein the alkyl, heteroalkyl, alkenyl, alkynyl, cycloalkyl, aryl, heteroaryl, and heterocycloalkyl are each unsubstituted or substituted with 1, 2, 3, or 4 independently selected R²⁰ groups;
- each R^{a3}, R^{b3}, R^{c3}, R^{d3}, R^{a4}, R^{b4}, R^{c4}, and R^{d4}, is independently selected from the group consisting of H, alkyl, alkenyl, alkynyl, alkoxy, cycloalkyl, aryl, heteroaryl, and heterocycloalkyl, each of which is unsubstituted or substituted with 1, 2, 3, or 4 independently selected R²⁰ groups;
   each R^{c3} and R^{d3} together with the N atom to which they are connected, come together to form a heteroaryl or heterocycloalkyl ring, each of which is unsubstituted or substituted with 1, 2, 3, or 4 independently selected R²⁰ groups;
   or each R^{c4} and R^{d4} together with the N atom to which they are connected, come together to form a heteroaryl or heterocycloalkyl ring, each of which is unsubstituted or substituted with 1, 2, 3, or 4 independently selected R²⁰ groups; and
- each R²⁰ is independently selected from the group consisting of OH, SH, CN, NO₂, halo, oxo, alkyl, alkenyl, alkynyl, alkoxy, cycloalkyl, aryl, heteroaryl, heterocycloalkyl, amino, carbamyl, or carbamoyl.

In some embodiments, R²⁴ is selected from the group consisting of H, azido, halo, CN, NO₂, C₁₋₆ alkyl, C₂₋₆ alkenyl, C₂₋₆ alkynyl, C₃₋₁₀ cycloalkyl, C₆₋₁₀ aryl, 5-10 membered heteroaryl, 4- 10 membered heterocycloalkyl, OR^{a4}, C(=O)R^{b4}, C(=O)OR^{b4}, NR^{c4}R^{d4}, C(=O)NR^{c4}R^{d4}, - OC(=O)NR^{c4}R^{d4}, NR^{c4}C(=O)R^{b4}, NR^{c4}C(=O)OR^{b4}, NR^{c4}C(=O)NR^{c4}R^{d4}, NR^{c4}S(=O)₂R^{b4}, NR^{c4}S(=O)₂NR^{c4}R^{d4}, S(O)NR^{c4}R^{d4}, and S(O)₂NR^{c4}R^{d4}, wherein the C₁₋₆ alkyl, C₃₋₁₀ cycloalkyl, C₆₋₁₀ aryl, 5-10 membered heteroaryl, and 4-10 membered heterocycloalkyl are each optionally substituted by 1, 2, 3, or 4 independently selected R²⁰ groups. In some embodiments, R²⁴ is selected from the group consisting of H, halo, CN, NO₂, C₁₋₆ alkyl, C₂₋₆ alkenyl, C₂₋₆ alkynyl, C₃₋₁₀ cycloalkyl, 4-10 membered heterocycloalkyl, OH, C₁₋₆ alkoxyl, and C₁₋₆ haloalkyl. In some embodiments, R²⁴ is selected from the group consisting of H, halo, CN, C₁₋₆ alkyl, C₂₋₆ alkynyl, C₃₋₆ cycloalkyl, 4-6 membered heterocycloalkyl, OH, C₁₋₆ alkoxyl, and C₁₋₆ haloalkyl. In some embodiments, R²⁴ is selected from the group consisting of hydrogen, OH, halo, CN, substituted or unsubstituted C₁₋₆ alkyl, substituted or unsubstituted C₁₋₆ alkoxyl, substituted or unsubstituted C₃₋₆ cycloalkyl, substituted or unsubstituted C₂₋₄ alkenyl, and substituted or unsubstituted C₂₋₄ alkynyl. In some embodiments, R²⁴ is hydrogen. In some embodiments, R²⁴ is halogen. In some embodiments, R²⁴ is -Br. In some embodiments, R²⁴ is -F. In some embodiments, R²⁴ is -Cl. In some embodiments, R²⁴ is -CN. In some embodiments, R²⁴ is OH. In some embodiments, R²⁴ is C₁₋₄ alkyl. In some embodiments, R²⁴ is C₁₋₄ haloalkyl. In some embodiments, R²⁴ is C₁₋₄ alkoxyl. In some embodiments, R²⁴ is methyl. In some embodiments, R²⁴ is ethyl. In some embodiments, R²⁴ is cycloalkyl. In some embodiments, R²⁴ is cyclopropyl. In some embodiments, R²⁴ is C₂₋₄ alkenyl. In some embodiments, R²⁴ is C₂₋₄ alkynyl. In some embodiments, R²⁴ is ethynyl. In some embodiment, R²⁴ is propynyl.

In some embodiments, R²¹ is unsubstituted or substituted furanyl or unsubstituted or substituted thiazolyl.

In some embodiments, R²¹ is unsubstituted or substituted furanyl. In some embodiments, R²¹ is unsubstituted furanyl. In some embodiments, R²¹ is substituted furanyl. In some embodiments, R²¹ is furanyl, which is substituted with 1, 2, or 3, independently selected R^{1A} groups; wherein each R^{1A} is independently selected from halo, CN, NO₂, alkyl, alkenyl, C₂₋₆ alkynyl, alkoxy, -C(=O)OH, an ether group, or an ester group, each of which is unsubstituted or substituted. In some embodiments, R²¹ is furanyl, which is substituted with 1, 2, or 3 substituents independently selected R^{1A} groups; wherein each R^{1A} is independently selected from halo, C₁₋₆alkyl, C₁₋₆haloalkyl, and C₁₋₆alkoxy. In some embodiments, R²¹ is furanyl, which is substituted with 1, 2, or 3 substituents independently selected R^{1A} groups; wherein each R^{1A} is independently selected from halo, C₁₋₃alkyl, C₁₋₃haloalkyl, and C₁₋₃alkoxy.

In some embodiments, R²¹ is unsubstituted or substituted thiazolyl. In some embodiments, R²¹ is unsubstituted thiazolyl. In some embodiments, R²¹ is substituted thiazolyl. In some embodiments, the thiazolyl is 1,3-thiazolyl. In some embodiments, the thiazolyl is 1,2-thiazolyl (or isothiazolyl). In some embodiments, R²¹ is thiazolyl, which is substituted with 1, 2, or 3, independently selected R^{1A} groups; wherein each R^{1A} is independently selected from halo, CN, NO₂, alkyl, alkenyl, C₂₋₆ alkynyl, alkoxy, -C(=O)OH, an ether group, or an ester group, each of which is unsubstituted or substituted. In some embodiments, R²¹ is thiazolyl, which is substituted with 1, 2, or 3 substituents independently selected R^{1A} groups; wherein each R^{1A} is independently selected from halo, C₁₋₆alkyl, C₁₋₆haloalkyl, and C₁₋₆alkoxy. In some embodiments, R²¹ is thiazolyl, which is substituted with 1, 2, or 3 substituents independently selected R^{1A} groups; wherein each R^{1A} is independently selected from halo, C₁₋₃alkyl, C₁₋₃haloalkyl, and C₁₋₃alkoxy.

**In** some embodiments, each R^{1A} is independently selected from halo, CN, NO₂, C₁₋₃alkyl, C₁₋₃haloalkyl, and C₁₋₃alkoxy. In some embodiments, each R^{1A} is independently selected from halo, C₁₋₃alkyl, C₁₋₃haloalkyl, and C₁₋₃alkoxy. In some embodiments, each R^{1A} is independently selected from halo, C₁₋₃alkyl, and C₁₋₃haloalkyl. In some embodiments, R^{1A} is halo. In some embodiments, R^{1A} is fluoro, chloro, bromo, or iodo. In some embodiments, R^{1A} is fluoro. In some embodiments, R^{1A} is chloro. In some embodiments, R^{1A} is bromo. In some embodiments, R^{1A} is iodo.

In some embodiments, R²¹ is selected from the group consisting of 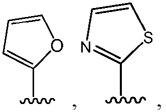 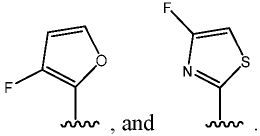 In some embodiments, R²¹ is 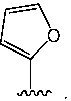 In some embodiments, R²¹ is 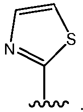 In some embodiments, R²¹ is 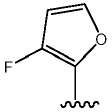 . In some embodiments, R²¹ is 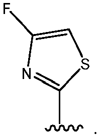 In some embodiments, R²¹ is 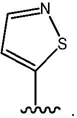 In some embodiments, R²¹ is

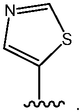

In some embodiments, R²³ is H.

In some embodiments, R²³ is substituted with 1, 2, or 3 independently selected R²⁰ groups, wherein each R²⁰ group is independently selected from the group consisting of OH, SH, CN, NO₂, halo, oxo, amino, C₁₋₃alkyl, C₁₋₃alkoxy, cycloalkyl, aryl, heteroaryl, heterocycloalkyl, carbamyl, and carbamoyl. In some embodiments, R²³ is substituted with 1, 2, or 3 independently selected R²⁰ groups, wherein each R²⁰ group is independently selected from the group consisting of OH, halo, and C₁₋₃alkoxy. In some embodiments, R²³ is substituted with 1, 2, or 3 independently selected R²⁰ groups, wherein each R²⁰ group is independently selected from the group consisting of OH, halo, C₁₋₃alkyl, C₁₋₃haloalkyl, amino, and C₁₋₃alkoxy.In some embodiments, R²³ is substituted with 1, 2, or 3 independently selected R²⁰ groups, wherein each R²⁰ group is independently selected from the group consisting of OH, halo, amino, and C₁₋₃alkoxy. In some embodiments, R²⁰ group is OH. In some embodiments, R²⁰ group is halo. In some embodiments, R²⁰ group is amino. In some embodiments, R²⁰ group is C₁₋₃alkoxy.

In some embodiments, R²³ is substituted or unsubstituted C₁₋₆ alkyl. In some embodiments, R²³ is C₁₋₆ alkyl, wherein C₁₋₆ alkyl is substituted with 1, 2, or 3 independently selected R²⁰ groups. In some embodiments, R²³ is C₁₋₆ alkyl, wherein C₁₋₆ alkyl is substituted with 1, 2, or 3 independently selected R²⁰ groups, wherein each R²⁰ group is independently selected from the group consisting of OH, SH, CN, NO₂, halo, oxo, amino, C₁₋₃alkyl, C₁₋₃alkoxy, cycloalkyl, aryl, heteroaryl, heterocycloalkyl, carbamyl, and carbamoyl. In some embodiments, R²³ is C₁₋₆ alkyl, wherein C₁₋₆ alkyl is substituted with 1, 2, or 3 independently selected R²⁰ groups, wherein each R²⁰ group is independently selected from the group consisting of OH, halo, and C₁₋₃alkoxy.

In some embodiments, R²³ is substituted or unsubstituted C₁₋₆ alkenyl. In some embodiments, R²³ is C₁₋₆ alkenyl, wherein C₁₋₆ alkenyl is substituted with 1, 2, or 3 independently selected R²⁰ groups.

In some embodiments, R²³ is substituted or unsubstituted C₁₋₆ alkynyl. In some embodiments, R²³ is C₁₋₆ alkynyl, wherein C₁₋₆ alkynyl is substituted with 1, 2, or 3 independently selected R²⁰ groups.

In some embodiments, R²³ is substituted or unsubstituted C₁₋₆ alkyl or substituted or unsubstituted C₁₋₆ heteroalkyl. In some embodiments, R²³ is substituted or unsubstituted C₁₋₆ heteroalkyl. In some embodiments, the C₁₋₆ heteroalkyl is -CH₂CH(NH₂)CH₂-S(=O)₂-CH₃ or - CH₂CH(NH₂)CH₂-S(=O)-CH₃. In some embodiments, R²³ is -CH₂CH(NH₂)CH₂-S(=O)₂-CH₃. In some embodiments, R²³ is -CH₂CH(NH₂)CH₂-S(=O)-CH₃. In some embodiments, R²³ is CH₂CHNH₂CH₃. In some embodiments, R²³ is CH₂CHNH₂CH₂OH. In some embodiments, R²³ is CH₂CHNH₂CH₂CH₃. In some embodiments, R²³ is CH₂CHNH₂CH₂CH₂OH. In some embodiments, R²³ is CH₂CHNH₂CH₂CH₂F. In some embodiments, R²³ is CH₂CHNH₂CH₂CHF₂. In some embodiments, R²³ is CH₂CHNH₂CH₂CH(CH₃)₂. In some embodiments, R²³ is CH₂CHNH₂CHFCH₃. In some embodiments, R²³ is CH₂CHNH₂CH₂F. In some embodiments, R²³ is CH₂CHNH₂CH₂OCH₃. In some embodiments, R²³ is CH₂CHNH₂CH₂OCD₃.

In some embodiments, R²³ is substituted or unsubstituted C₁₋₆ heteroalkyl. In some embodiments, R²³ is C₁₋₆ heteroalkyl, wherein the C₁₋₆ heteroalkyl is substituted with 1, 2, or 3 independently selected R²⁰ groups. In some embodiments, R²³ is C₁₋₆ heteroalkyl, wherein the C₁₋₆ heteroalkyl is substituted with 1, 2, or 3 independently selected R²⁰ groups, wherein each R²⁰ group is independently selected from the group consisting of OH, SH, CN, NO₂, halo, oxo, amino, C₁₋₃alkyl, C₁₋₃alkoxy, cycloalkyl, aryl, heteroaryl, heterocycloalkyl, carbamyl, and carbamoyl. In some embodiments, R²³ is C₁₋₆ heteroalkyl, wherein the C₁₋₆ heteroalkyl is substituted with 1, 2, or 3 independently selected R²⁰ groups, wherein each R²⁰ group is independently selected from the group consisting of OH, halo, and C₁₋₃alkoxy. In some embodiments, R²³ is C₁₋₆ alkyl, wherein C₁₋₆ alkyl is substituted with 1, 2, or 3 independently selected R²⁰ groups, wherein each R²⁰ group is independently selected from the group consisting of OH, halo, amino, and C₁₋₃alkoxy.

**In** some embodiments, R²³ is substituted or unsubstituted -(C₁₋₆ alkylene)-C₃₋₁₀ cycloalkyl. In some embodiments, R²³ is -(C₁₋₆ alkylene)-C₃₋₁₀ cycloalkyl, wherein -(C₁₋₆ alkylene)-C₃₋₁₀ cycloalkyl is substituted with 1, 2, or 3 independently selected R²⁰ groups. In some embodiments, the C₁₋₆ alkylene is C₁₋₃ alkylene. In some embodiments, the C₁₋₆ alkylene is CH₂. In some embodiments, the C₃₋₁₀ cycloalkyl is an optionally substituted 3-6 membered ring. In some embodiments, the C₃₋₁₀ cycloalkyl is an optionally substituted 3 membered ring. In some embodiments, the C₃₋₁₀ cycloalkyl is an optionally substituted 4 membered ring. In some embodiments, the C₃₋₁₀ cycloalkyl is an optionally substituted 5 membered ring. In some embodiments, the C₃₋₁₀ cycloalkyl is an optionally substituted 6 membered ring. In some embodiments, the C₃₋₁₀ cycloalkyl is 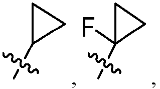 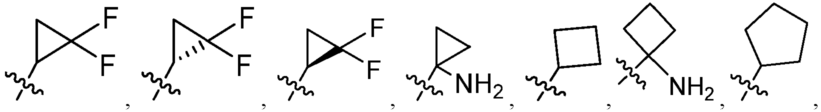 or

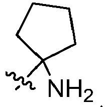

In some embodiments, R²³ is substituted or unsubstituted -(C₁₋₆ alkylene)-4-10 membered heterocycloalkyl. In some embodiments, R²³ is -(C₁₋₆ alkylene)-4-10 membered heterocycloalkyl, wherein -(C₁₋₆ alkylene)-4-10 membered heterocycloalkyl is substituted with 1, 2, or 3 independently selected R²⁰ groups. In some embodiments, the C₁₋₆ alkylene is C₁₋₃ alkylene. In some embodiments, the C₁₋₆ alkylene is CH₂. In some embodiments, the 4-10 membered heterocycloalkyl is an optionally substituted 4-6 membered ring. In some embodiments, the 4-10 membered heterocycloalkyl is an optionally substituted 4 membered ring. In some embodiments, the 4-10 membered heterocycloalkyl is an optionally substituted 5 membered ring. In some embodiments, the 4-10 membered heterocycloalkyl is an optionally substituted 6 membered ring. In some embodiments, the 4-10 membered heterocycloalkyl contains 0-1 oxygen and 0-2 nitrogen atoms. In some embodiments, the 4-10 membered heterocycloalkyl is 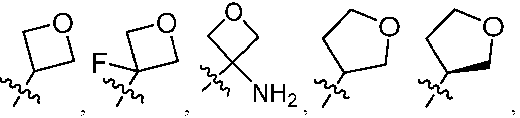 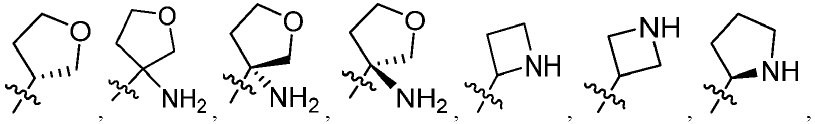 or

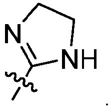

**In** some embodiments, R²³ is substituted or unsubstituted -(C₁₋₆ heteroalkylene)-C₃₋₁₀ cycloalkyl. In some embodiments, R²³ is -(C₁₋₆ heteroalkylene)-C₃₋₁₀ cycloalkyl, wherein -(C₁₋₆ heteroalkylene)-C₃₋₁₀ cycloalkyl is substituted with 1, 2, or 3 independently selected R²⁰ groups. In some embodiments, the heteroalkylene is C₁₋₃ heteroalkylene. In some embodiments, the C₃₋₁₀ cycloalkyl is an optionally substituted 3-6 membered ring. In some embodiments, the C₃₋₁₀ cycloalkyl is an optionally substituted 3 membered ring. In some embodiments, the C₃₋₁₀ cycloalkyl is an optionally substituted 4 membered ring. In some embodiments, the C₃₋₁₀ cycloalkyl is an optionally substituted 5 membered ring. In some embodiments, the C₃₋₁₀ cycloalkyl is an optionally substituted 6 membered ring. In some embodiments, the heteroalkylene is C₁₋₃ heteroalkylene. In some embodiments, the C₃₋₁₀ cycloalkyl is

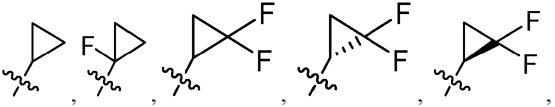

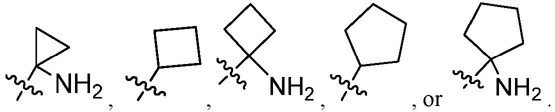

In some embodiments, R²³ is substituted or unsubstituted -(C₁₋₆ heteroalkylene)-4-10 membered heterocycloalkyl. In some embodiments, R²³ is -(C₁₋₆ heteroalkylene)-4-10 membered heterocycloalkyl, wherein -(C₁₋₆ heteroalkylene)-4-10 membered heterocycloalkyl is substituted with 1, 2, or 3 independently selected R²⁰ groups. In some embodiments, the heteroalkylene is C₁₋₃ heteroalkylene. In some embodiments, the 4-10 membered heterocycloalkyl is an optionally substituted 4-6 membered ring. In some embodiments, the 4-10 membered heterocycloalkyl is an optionally substituted 4 membered ring. In some embodiments, the 4-10 membered heterocycloalkyl is an optionally substituted 5 membered ring. In some embodiments, the 4-10 membered heterocycloalkyl is an optionally substituted 6 membered ring. In some embodiments, the 4-10 membered heterocycloalkyl contains 0-1 oxygen and 0-2 nitrogen atoms. In some embodiments, the 4-10 membered heterocycloalkyl is 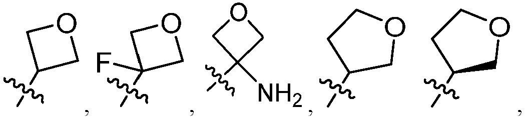 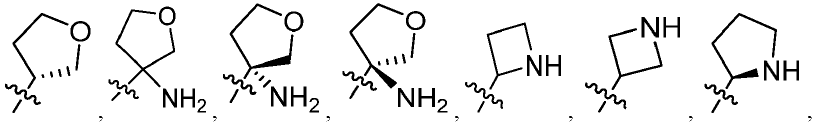 or

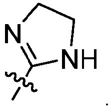

In some embodiments, R²³ is any one selected from the group consisting of: 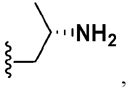 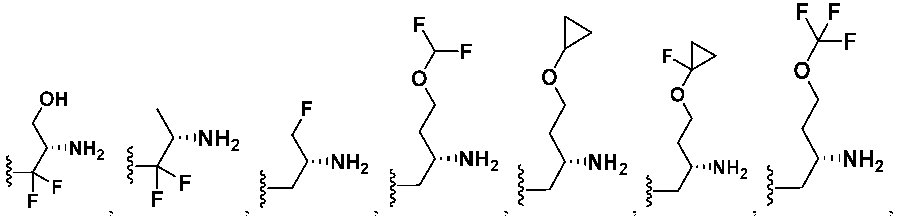 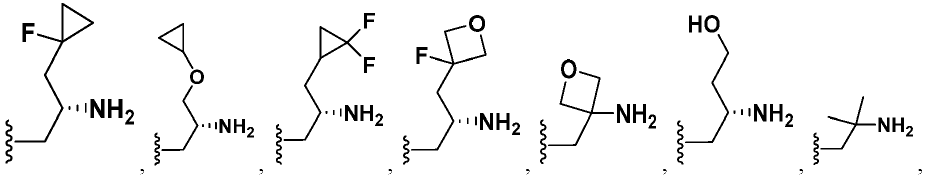 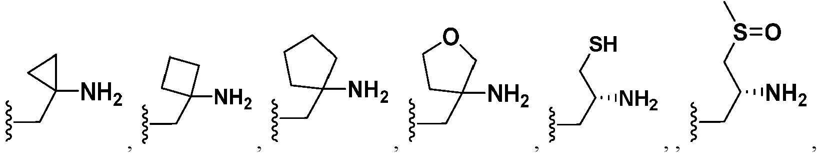 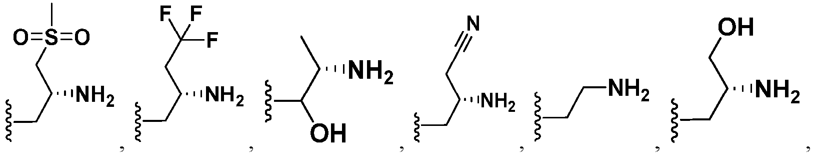 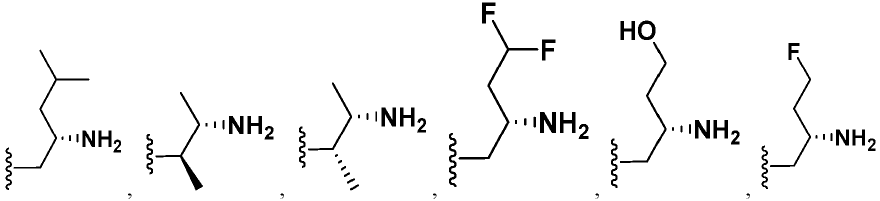 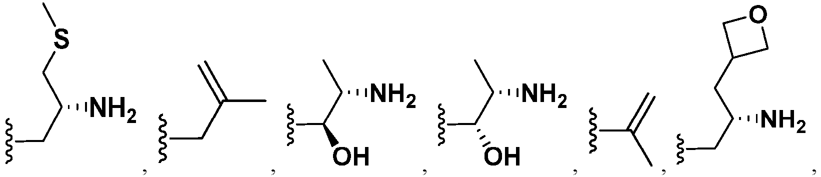 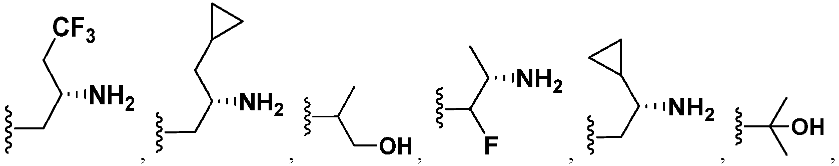 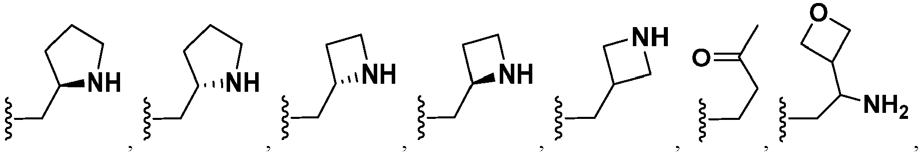 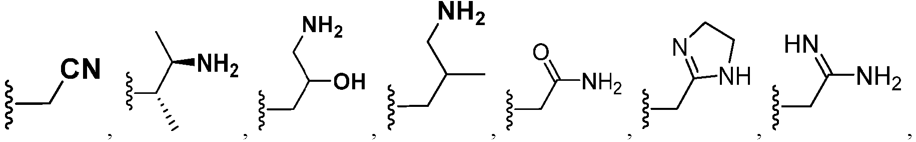 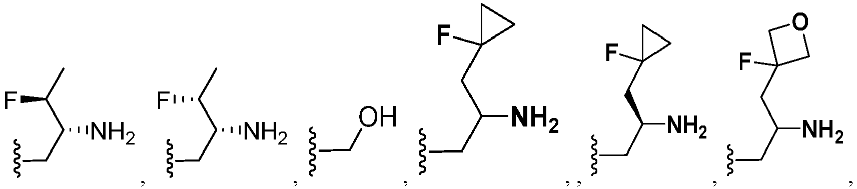 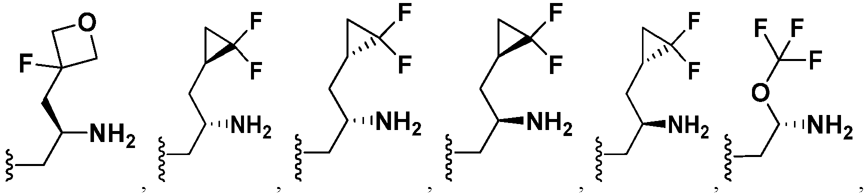 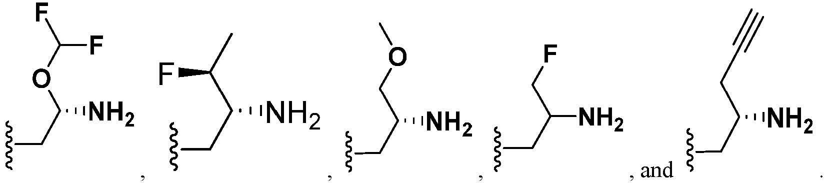 In some embodiments, R²³ is any one selected from the group consisting of: 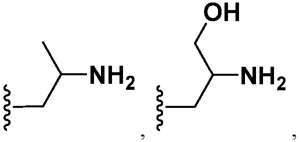 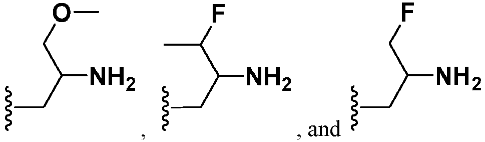 In some embodiments, R²³ is 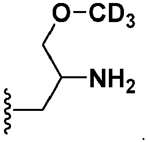 In some embodiments, R²³ is 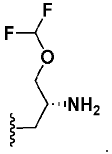 In some embodiments, R²³ is

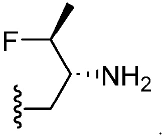

In some embodiments, each R²⁰ is independently selected from the group consisting of OH, SH, CN, NO₂, halo, oxo, C₁₋₄ alkyl, C₂₋₄ alkenyl, C₂₋₄ alkynyl, C₁₋₄ haloalkyl, C₁₋₄ cyanoalkyl, C₁₋₄ hydroxyalkyl, C₁₋₄ alkoxy, -(C₁₋₄ alkyl)-(C₁₋₄ alkoxy), -(C₁₋₄ alkoxy)-(C₁₋₄ alkoxy), C₁₋₄ haloalkoxy, C₃₋₆ cycloalkyl, phenyl, 5-6 membered heteroaryl, 4-6 membered heterocycloalkyl, amino, C₁₋₄ alkylamino, di(C₁₋₄ alkyl)amino, carbamyl, C₁₋₄ alkylcarbamyl, di(C₁₋₄ alkyl)carbamyl, carbamoyl, C₁₋₄ alkylcarbamoyl, di(C₁₋₄ alkyl)carbamoyl, C₁₋₄ alkylcarbonyl, C₁₋₄ alkoxycarbonyl, C₁₋₄ alkylcarbonylamino, C₁₋₄ alkylsulfonylamino, aminosulfonyl, C₁₋₄ alkylaminosulfonyl, di(C₁₋₄ alkyl)aminosulfonyl, aminosulfonylamino, C₁₋₄ alkylaminosulfonylamino, di(C₁₋₄ alkyl)aminosulfonylamino, aminocarbonylamino, C₁₋₄ alkylaminocarbonylamino, di(C₁₋₄ alkyl)aminocarbonylamino, and amidinyl. In some embodiments, each R²⁰ is independently selected from the group consisting of OH, SH, CN, NO₂, halo, oxo, C₁₋₄ alkyl, C₂₋₄ alkenyl, C₂₋₄ alkynyl, C₁₋₄ haloalkyl, C₁₋₄ cyanoalkyl, C₁₋₄ hydroxyalkyl, C₁₋₄ alkoxy, -C₁₋₄ haloalkoxy, C₃₋₆ cycloalkyl, 4-6 membered heterocycloalkyl, amino, C₁₋₄ alkylamino, di(C₁₋₄ alkyl)amino, carbamyl, and amidinyl. In some embodiments, each R²⁰ is independently selected from the group consisting of OH, SH, CN, NO₂, halo, oxo, C₁₋₄ alkyl, C₁₋₄ haloalkyl, C₁₋₄ hydroxyalkyl, C₁₋₄ alkoxy, -C₁₋₄ haloalkoxy, C₃₋₆ cycloalkyl, 4-6 membered heterocycloalkyl, amino, carbamyl C₁₋₄ alkylamino, di(C₁₋₄ alkyl)amino, and amidinyl. In some embodiments, R²⁰ is OH. In some embodiments, R²⁰ is NH₂. In some embodiments, R²⁰ is SH. In some embodiments, R²⁰ is CN. In some embodiments, R²⁰ is F. In some embodiments, R²⁰ is carbamyl.

In some embodiments, R²⁴ is selected from the group consisting of halo, CN, and substituted or unsubstituted C₁₋₆ alkyl. In some embodiments, R²⁴ is selected from the group consisting of hydrogen, OH, halo, CN, substituted or unsubstituted C₁₋₆ alkyl, substituted or unsubstituted C₁₋₆ alkoxyl, substituted or unsubstituted C₃₋₁₀ cycloalkyl, substituted or unsubstituted C₂₋₄ alkenyl, and substituted or unsubstituted C₂₋₄ alkynyl.

In some embodiments, each R^{a3}, R^{b3}, R^{c3}, R^{d3}, R^{a4}, R^{b4}, R^{c4}, and R^{d4}, is independently selected from the group consisting of H, C₁₋₆ alkyl, C₁₋₆ hydroxyalkyl, and C₁₋₆ haloalkyl. In some embodiments, each R^{a3}, R^{b3}, R^{c3}, R^{d3}, R^{a4}, R^{b4}, R^{c4}, and R^{d4}, is independently selected from the group consisting of H and C₁₋₆ alkyl. In some embodiments, each R^{a3}, R^{b3}, R^{c3}, R^{d3}, R^{a4}, R^{b4}, R^{c4}, and R^{d4}, is independently selected from the group consisting of H and C₁₋₃ alkyl. In some embodiments, each R^{a3}, R^{b3}, R^{c3}, R^{d3}, R^{a4}, R^{b4}, R^{c4}, and R^{d4}, is hydrogen.

In some embodiments, the compound is of the Formula (IIa): 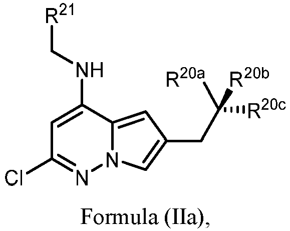 wherein
R²¹ has the meaning defined in Formula (I);
each R^{20a}, R^{20b}, and R^{20c} is independently selected from the group consisting of H, OH, SH, CN, NO₂, halo, C₁₋₄ alkyl, C₂₋₄ alkenyl, C₂₋₄ alkynyl, C₁₋₄ haloalkyl, C₁₋₄ cyanoalkyl, C₁₋₄ hydroxyalkyl, C₁₋₄ alkoxy, -(C₁₋₄ alkyl)-(C₁₋₄ alkoxy), -(C₁₋₄ alkoxy)-(C₁₋₄ alkoxy), C₁₋₄ haloalkoxy, C₃₋₆ cycloalkyl, C₁₋₄ heteroalkyl, phenyl, 5-6 membered heteroaryl, 4-6 membered heterocycloalkyl, - (C₁₋₃ alkylene)-C₃₋₁₀ cycloalkyl, -(C₁₋₃ alkylene)-4-10 membered heterocycloalkyl, -(C₁₋₃ heteroalkylene)-C₃₋₁₀ cycloalkyl, -(C₁₋₃ heteroalkylene)-4-10 membered heterocycloalkyl, amidinyl, amino, C₁₋₄ alkylamino, di(C₁₋₄ alkyl)amino, carbamyl, C₁₋₄ alkylcarbamyl, di(C₁₋₄ alkyl)carbamyl, carbamoyl, C₁₋₄ alkylcarbamoyl, di(C₁₋₄ alkyl)carbamoyl, C₁₋₄ alkylcarbonyl, C₁₋₄ alkoxycarbonyl, C₁₋₄ alkylcarbonylamino, C₁₋₄ alkylsulfonylamino, aminosulfonyl, C₁₋₄ alkylaminosulfonyl, di(C₁₋₄ alkyl)aminosulfonyl, aminosulfonylamino, C₁₋₄ alkylaminosulfonylamino, di(C₁₋₄ alkyl)aminosulfonylamino, aminocarbonylamino, C₁₋₄ alkylaminocarbonylamino, and di(C₁₋₄ alkyl)aminocarbonylamino, wherein:
   each of the cycloalkyl and heterocycloalkyl is optionally substituted by 1, 2, 3, or 4 substituents independently selected from halo, CN, SH, -CN, oxo, NO₂, OH, C₁₋₄ alkyl, C₂₋₄ alkenyl, C₂₋₄ alkynyl, C₁₋₄ haloalkyl, C₁₋₄ cyanoalkyl, C₁₋₄ aminoalkyl, C₁₋₄ hydroxyalkyl, C₁₋₄ alkoxy, and amino,
   or R^{20a} and R^{20b} are taken together to form a =NH or =N(C₁₋₄ alkyl),
or a pharmaceutically acceptable salt thereof.

In some embodiments of the compound of the Formula (IIa) or a pharmaceutically acceptable salt thereof, each R^{20a}, R^{20b}, and R^{20c} is independently selected from the group consisting of H, OH, SH, CN, NO₂, halo, C₁₋₄ alkyl, C₂₋₄ alkenyl, C₂₋₄ alkynyl, C₁₋₄ haloalkyl, C₁₋₄ cyanoalkyl, C₁₋₄ hydroxyalkyl, C₁₋₄ alkoxy, -(C₁₋₄ alkyl)-(C₁₋₄ alkoxy), -(C₁₋₄ alkoxy)-(C₁₋₄ alkoxy), C₁₋₄ haloalkoxy, C₃₋₆ cycloalkyl, phenyl, 5-6 membered heteroaryl, 5-6 membered heterocycloalkyl, amino, C₁₋₄ alkylamino, di(C₁₋₄ alkyl)amino, carbamyl, C₁₋₄ alkylcarbamyl, di(C₁₋₄ alkyl)carbamyl, carbamoyl, C₁₋₄ alkylcarbamoyl, di(C₁₋₄ alkyl)carbamoyl, C₁₋₄ alkylcarbonyl, C₁₋₄ alkoxycarbonyl, C₁₋₄ alkylcarbonylamino, C₁₋₄ alkylsulfonylamino, aminosulfonyl, C₁₋₄ alkylaminosulfonyl, di(C₁₋₄ alkyl)aminosulfonyl, aminosulfonylamino, C₁₋₄ alkylaminosulfonylamino, di(C₁₋₄ alkyl)aminosulfonylamino, aminocarbonylamino, C₁₋₄ alkylaminocarbonylamino, and di(C₁₋₄ alkyl)aminocarbonylamino.

In some embodiments, R^{20a} is methyl. In some embodiments, R^{20a} is ethyl. In some embodiments, R^{20a} is CH₂OH. In some embodiments, R^{20a} is CH₂CH₂OH. In some embodiments, R^{20a} is CH₂CH₂F. In some embodiments, R^{20a} is CH₂CHF₂. In some embodiments, R^{20a} is CH₂CH(CH₃)₂. In some embodiments, R^{20c} is NH₂. In some embodiments, R^{20b} is hydrogen. In some embodiments, R^{20a} and R^{20b} are taken together to form a =NH or =N(C₁₋₄ alkyl). In some embodiments, R^{20a} is 4-6 membered heterocycloalkyl. In some embodiments, R^{20a} is -(C₁₋₃ alkylene)-C₃₋₁₀ cycloalkyl. In some embodiments, R^{20a} is -(C₁₋₃ alkylene)-4-10 membered heterocycloalkyl. In some embodiments, R^{20a} is -(C₁₋₃ heteroalkylene)-C₃₋₁₀ cycloalkyl. In some embodiments, R^{20a} is -(C₁₋₃ heteroalkylene)-4-10 membered heterocycloalkyl. In some embodiments, the C₃₋₁₀ cycloalkyl is 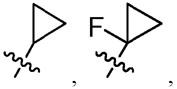 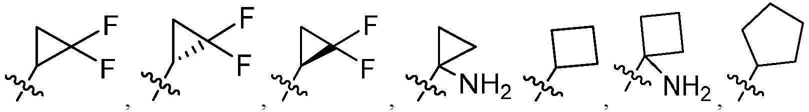 , or 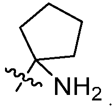 In some embodiments, the C₃₋₁₀ cycloalkyl is a 3-5 membered ring, which is optionally substituted. In some embodiments, the 4-10 membered heterocycloalkyl is 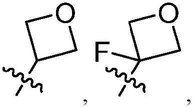 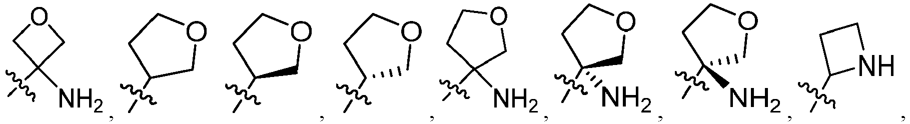 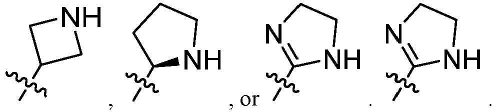 In some embodiments, the 4-10 membered heterocycloalkyl is a 4-5 membered ring, which is optionally substituted. In some embodiments, R^{20a} is C₁₋₄heteroalkyl.

In some embodiments, the compound is of the Formula (IIb): 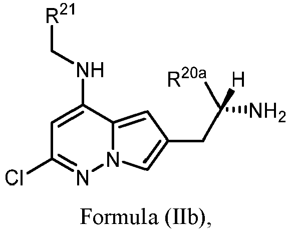 wherein
R²¹ has the meaning defined in Formula (I);
R^{20a} is selected from the group consisting of H, OH, SH, CN, NO₂, halo, oxo, C₁₋₄ alkyl, C₂₋₄ alkenyl, C₂₋₄ alkynyl, C₁₋₄ haloalkyl, C₁₋₄ cyanoalkyl, C₁₋₄ hydroxyalkyl, C₁₋₄ alkoxy, -(C₁₋₄ alkyl)-(C₁₋₄ alkoxy), -(C₁₋₄ alkoxy)-(C₁₋₄ alkoxy), C₁₋₄ haloalkoxy, C₃₋₆ cycloalkyl, C₁₋₄ heteroalkyl, phenyl, 5-6 membered heteroaryl, 4-6 membered heterocycloalkyl, -(C₁₋₃ alkylene)-C₃₋₁₀ cycloalkyl, -(C₁₋₃ alkylene)-4-10 membered heterocycloalkyl, -(C₁₋₃ heteroalkylene)-C₃₋₁₀ cycloalkyl, -(C₁₋₃ heteroalkylene)-4-10 membered heterocycloalkyl, amidinyl, amino, C₁₋₄ alkylamino, di(C₁₋₄ alkyl)amino, carbamyl, C₁₋₄ alkylcarbamyl, di(C₁₋₄ alkyl)carbamyl, carbamoyl, C₁₋₄ alkylcarbamoyl, di(C₁₋₄ alkyl)carbamoyl, C₁₋₄ alkylcarbonyl, C₁₋₄ alkoxycarbonyl, C₁₋₄ alkylcarbonylamino, C₁₋₄ alkylsulfonylamino, aminosulfonyl, C₁₋₄ alkylaminosulfonyl, di(C₁₋₄ alkyl)aminosulfonyl, aminosulfonylamino, C₁₋₄ alkylaminosulfonylamino, di(C₁₋₄ alkyl)aminosulfonylamino, aminocarbonylamino, C₁₋₄ alkylaminocarbonylamino, and di(C₁₋₄ alkyl)aminocarbonylamino, wherein each of the cycloalkyl and heterocycloalkyl is optionally substituted by 1, 2, 3, or 4 substituents independently selected from halo, CN, SH, -CN, oxo, NO₂, OH, C₁₋₄ alkyl, C₂₋₄ alkenyl, C₂₋₄ alkynyl, C₁₋₄ haloalkyl, C₁₋₄ cyanoalkyl, C₁₋₄ aminoalkyl, C₁₋₄ hydroxyalkyl, C₁₋₄ alkoxy, and amino, or a pharmaceutically acceptable salt thereof.

In some embodiments of the compound of the Formula (IIb) or a pharmaceutically acceptable salt thereof: R^{20a} is selected from the group consisting of OH, SH, CN, NO₂, halo, oxo, C₁₋₄ alkyl, C₂₋₄ alkenyl, C₂₋₄ alkynyl, C₁₋₄ haloalkyl, C₁₋₄ cyanoalkyl, C₁₋₄ hydroxyalkyl, C₁₋₄ alkoxy, -(C₁₋₄ alkyl)-(C₁₋₄ alkoxy), -(C₁₋₄ alkoxy)-(C₁₋₄ alkoxy), C₁₋₄ haloalkoxy, C₃₋₆ cycloalkyl, phenyl, 5-6 membered heteroaryl, 5-6 membered heterocycloalkyl, amino, C₁₋₄ alkylamino, di(C₁₋₄ alkyl)amino, carbamyl, C₁₋₄ alkylcarbamyl, di(C₁₋₄ alkyl)carbamyl, carbamoyl, C₁₋₄ alkylcarbamoyl, di(C₁₋₄ alkyl)carbamoyl, C₁₋₄ alkylcarbonyl, C₁₋₄ alkoxycarbonyl, C₁₋₄ alkylcarbonylamino, C₁₋₄ alkylsulfonylamino, aminosulfonyl, C₁₋₄ alkylaminosulfonyl, di(C₁₋₄ alkyl)aminosulfonyl, aminosulfonylamino, C₁₋₄ alkylaminosulfonylamino, di(C₁₋₄ alkyl)aminosulfonylamino, aminocarbonylamino, C₁₋₄ alkylaminocarbonylamino, and di(C₁₋₄ alkyl)aminocarbonylamino.

In some embodiments, R^{20a} is methyl. In some embodiments, R^{20a} is ethyl. In some embodiments, R^{20a} is CH₂OH. In some embodiments, R^{20a} is CH₂CH₂OH. In some embodiments, R^{20a} is CH₂CH₂F. In some embodiments, R^{21a} is CH₂CHF₂. In some embodiments, R^{21a} is CH₂CH(CH₃)₂. In some embodiments, R^{21a} is 4-6 membered heterocycloalkyl. In some embodiments, R^{20a} is -(C₁₋₃ alkylene)-C₃₋₁₀ cycloalkyl. In some embodiments, R^{20a} is -(C₁₋₃ alkylene)-4-10 membered heterocycloalkyl. In some embodiments, R^{20a} is -(C₁₋₃ heteroalkylene)-C₃₋₁₀ cycloalkyl. In some embodiments, R^{20a} is -(C₁₋₃ heteroalkylene)-4-10 membered heterocycloalkyl. In some embodiments, the C₃₋₁₀ cycloalkyl is 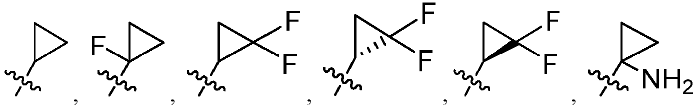 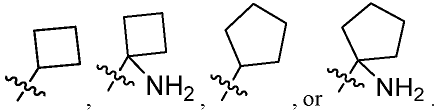 In some embodiments, the C₃₋₁₀ cycloalkyl is a 3-5 membered ring, which is optionally substituted. In some embodiments, the 4-10 membered heterocycloalkyl is 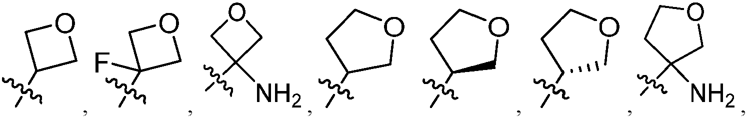 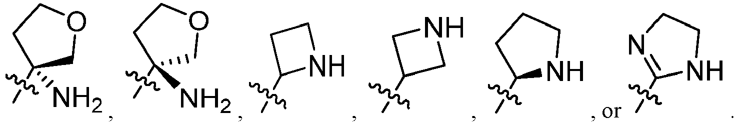 In some embodiments, the 4-10 membered heterocycloalkyl is a 4-5 membered ring, which is optionally substituted. In some embodiments, R^{20a} is C₁₋₄ heteroalkyl. In some embodiments, R^{20a} is C₁₋₄ alkyl. In some embodiments, R^{20a} is optionally substituted C₁₋₄heteroalkyl. In some embodiments, R^{20a} is optionally substituted C₁₋₄ alkyl.

In some embodiments, the compound is of the Formula (IIIa): 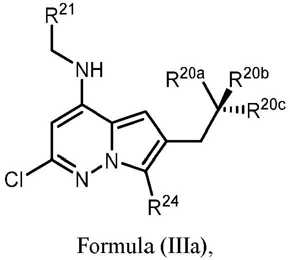 wherein
R²¹ and R²⁴ each has the meaning defined in Formula (I);
each R^{20a}, R^{20b}, and R^{20c} is independently selected from the group consisting of H, OH, SH, CN, NO₂, halo, C₁₋₄ alkyl, C₂₋₄ alkenyl, C₂₋₄ alkynyl, C₁₋₄ haloalkyl, C₁₋₄ cyanoalkyl, C₁₋₄ hydroxyalkyl, C₁₋₄ alkoxy, -(C₁₋₄ alkyl)-(C₁₋₄ alkoxy), -(C₁₋₄ alkoxy)-(C₁₋₄ alkoxy), C₁₋₄ haloalkoxy, C₃₋₆ cycloalkyl, C₁₋₄ heteroalkyl, phenyl, 5-6 membered heteroaryl, 4-6 membered heterocycloalkyl, - (C₁₋₃ alkylene)-C₃₋₁₀ cycloalkyl, -(C₁₋₃ alkylene)-4-10 membered heterocycloalkyl, -(C₁₋₃ heteroalkylene)-C₃₋₁₀ cycloalkyl, -(C₁₋₃ heteroalkylene)-4-10 membered heterocycloalkyl, amidinyl, amino, C₁₋₄ alkylamino, di(C₁₋₄ alkyl)amino, carbamyl, C₁₋₄ alkylcarbamyl, di(C₁₋₄ alkyl)carbamyl, carbamoyl, C₁₋₄ alkylcarbamoyl, di(C₁₋₄ alkyl)carbamoyl, C₁₋₄ alkylcarbonyl, C₁₋₄ alkoxycarbonyl, C₁₋₄ alkylcarbonylamino, C₁₋₄ alkylsulfonylamino, aminosulfonyl, C₁₋₄ alkylaminosulfonyl, di(C₁₋₄ alkyl)aminosulfonyl, aminosulfonylamino, C₁₋₄ alkylaminosulfonylamino, di(C₁₋₄ alkyl)aminosulfonylamino, aminocarbonylamino, C₁₋₄ alkylaminocarbonylamino, and di(C₁₋₄ alkyl)aminocarbonylamino, wherein:
   each of the cycloalkyl and heterocycloalkyl is optionally substituted by 1, 2, 3, or 4 substituents independently selected from halo, CN, SH, -CN, oxo, NO₂, OH, C₁₋₄ alkyl, C₂₋₄ alkenyl, C₂₋₄ alkynyl, C₁₋₄ haloalkyl, C₁₋₄ cyanoalkyl, C₁₋₄ aminoalkyl, C₁₋₄ hydroxyalkyl, C₁₋₄ alkoxy, and amino,
   or R^{20a} and R^{20b} are taken together to form a =NH or =N(C₁₋₄ alkyl),
or a pharmaceutically acceptable salt thereof.

In some embodiments of the compound of the Formula (IIIa) or a pharmaceutically acceptable salt thereof, each R^{20a}, R^{20b}, and R^{20c} is independently selected from the group consisting of H, OH, SH, CN, NO₂, halo, C₁₋₄ alkyl, C₂₋₄ alkenyl, C₂₋₄ alkynyl, C₁₋₄ haloalkyl, C₁₋₄ cyanoalkyl, C₁₋₄ hydroxyalkyl, C₁₋₄ alkoxy, -(C₁₋₄ alkyl)-(C₁₋₄ alkoxy), -(C₁₋₄ alkoxy)-(C₁₋₄ alkoxy), C₁₋₄ haloalkoxy, C₃₋₆ cycloalkyl, phenyl, 5-6 membered heteroaryl, 5-6 membered heterocycloalkyl, amino, C₁₋₄ alkylamino, di(C₁₋₄ alkyl)amino, carbamyl, C₁₋₄ alkylcarbamyl, di(C₁₋₄ alkyl)carbamyl, carbamoyl, C₁₋₄ alkylcarbamoyl, di(C₁₋₄ alkyl)carbamoyl, C₁₋₄ alkylcarbonyl, C₁₋₄ alkoxycarbonyl, C₁₋₄ alkylcarbonylamino, C₁₋₄ alkylsulfonylamino, aminosulfonyl, C₁₋₄ alkylaminosulfonyl, di(C₁₋₄ alkyl)aminosulfonyl, aminosulfonylamino, C₁₋₄ alkylaminosulfonylamino, di(C₁₋₄ alkyl)aminosulfonylamino, aminocarbonylamino, C₁₋₄ alkylaminocarbonylamino, and di(C₁₋₄ alkyl)aminocarbonylamino.

In some embodiments, R^{20a} is methyl. In some embodiments, R^{20a} is ethyl. In some embodiments, R^{20a} is CH₂OH. In some embodiments, R^{20a} is CH₂CH₂OH. In some embodiments, R^{20a} is CH₂CH₂F. In some embodiments, R^{20a} is CH₂CHF₂. In some embodiments, R^{20a} is CH₂CH(CH₃)₂. In some embodiments, R^{20c} is NH₂. In some embodiments, R^{20b} is hydrogen. In some embodiments, R^{20a} is 4-6 membered heterocycloalkyl. In some embodiments, R^{20a} is -(C₁₋₃ alkylene)-C₃₋₁₀ cycloalkyl. In some embodiments, R^{20a} is -(C₁₋₃ alkylene)-4-10 membered heterocycloalkyl. In some embodiments, R^{20a} is -(C₁₋₃ heteroalkylene)-C₃₋₁₀ cycloalkyl. In some embodiments, R^{20a} is -(C₁₋₃ heteroalkylene)-4-10 membered heterocycloalkyl. In some embodiments, the C₃₋₁₀ cycloalkyl is 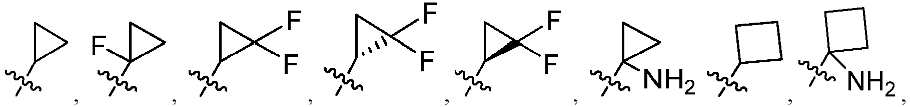 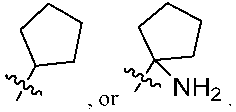 In some embodiments, the C₃₋₁₀ cycloalkyl is a 3-5 membered ring, which is optionally substituted. In some embodiments, the 4-10 membered heterocycloalkyl is 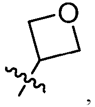 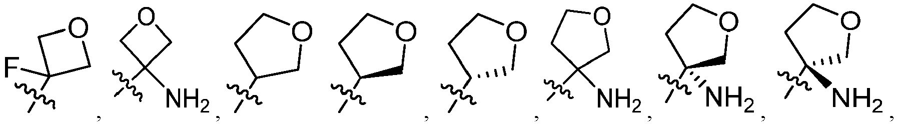 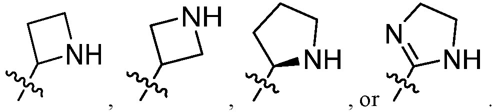 In some embodiments, the 4-10 membered heterocycloalkyl is a 4-5 membered ring, which is optionally substituted. In some embodiments, R^{20a} is C₁₋₄heteroalkyl. In some embodiments, R^{20a} is C₁₋₄alkyl. In some embodiments, R^{20a} is optionally substituted C₁₋₄heteroalkyl. In some embodiments, R^{20a} is optionally substituted C₁₋₄alkyl.

In some embodiments, the compound is of the Formula (IIIb): 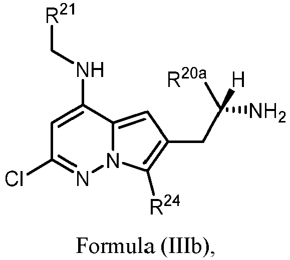 wherein
R²¹ and R²⁴ each has the meaning defined in Formula (I);
R^{20a} is selected from the group consisting of H, OH, SH, CN, NO₂, halo, C₁₋₄ alkyl, C₂₋₄ alkenyl, C₂₋₄ alkynyl, C₁₋₄ haloalkyl, C₁₋₄ cyanoalkyl, C₁₋₄ hydroxyalkyl, C₁₋₄ alkoxy, -(C₁₋₄ alkyl)-(C₁₋₄ alkoxy), -(C₁₋₄ alkoxy)-(C₁₋₄ alkoxy), C₁₋₄ haloalkoxy, C₃₋₆ cycloalkyl, C₁₋₄ heteroalkyl, phenyl, 5-6 membered heteroaryl, 4-6 membered heterocycloalkyl, -(C₁₋₃ alkylene)-C₃₋₁₀ cycloalkyl, -(C₁₋₃ alkylene)-4-10 membered heterocycloalkyl, -(C₁₋₃ heteroalkylene)-C₃₋₁₀ cycloalkyl, -(C₁₋₃ heteroalkylene)-4-10 membered heterocycloalkyl, amidinyl, amino, C₁₋₄ alkylamino, di(C₁₋₄ alkyl)amino, carbamyl, C₁₋₄ alkylcarbamyl, di(C₁₋₄ alkyl)carbamyl, carbamoyl, C₁₋₄ alkylcarbamoyl, di(C₁₋₄ alkyl)carbamoyl, C₁₋₄ alkylcarbonyl, C₁₋₄ alkoxycarbonyl, C₁₋₄ alkylcarbonylamino, C₁₋₄ alkylsulfonylamino, aminosulfonyl, C₁₋₄ alkylaminosulfonyl, di(C₁₋₄ alkyl)aminosulfonyl, aminosulfonylamino, C₁₋₄ alkylaminosulfonylamino, di(C₁₋₄ alkyl)aminosulfonylamino, aminocarbonylamino, C₁₋₄ alkylaminocarbonylamino, and di(C₁₋₄ alkyl)aminocarbonylamino, wherein each of the cycloalkyl and heterocycloalkyl is optionally substituted by 1, 2, 3, or 4 substituents independently selected from halo, CN, SH, -CN, oxo, NO₂, OH, C₁₋₄ alkyl, C₂₋₄ alkenyl, C₂₋₄ alkynyl, C₁₋₄ haloalkyl, C₁₋₄ cyanoalkyl, C₁₋₄ aminoalkyl, C₁₋₄ hydroxyalkyl, C₁₋₄ alkoxy, and amino, or a pharmaceutically acceptable salt thereof.

In some embodiments of the compound of the Formula (IIIb) or a pharmaceutically acceptable salt thereof, R^{20a} is selected from the group consisting of OH, SH, CN, NO₂, halo, C₁₋₄ alkyl, C₂₋₄ alkenyl, C₂₋₄ alkynyl, C₁₋₄ haloalkyl, C₁₋₄ cyanoalkyl, C₁₋₄ hydroxyalkyl, C₁₋₄ alkoxy, -(C₁₋₄ alkyl)-(C₁₋₄ alkoxy), -(C₁₋₄ alkoxy)-(C₁₋₄ alkoxy), C₁₋₄ haloalkoxy, C₃₋₆ cycloalkyl, phenyl, 5-6 membered heteroaryl, 5-6 membered heterocycloalkyl, amino, C₁₋₄ alkylamino, di(C₁₋₄ alkyl)amino, carbamyl, C₁₋₄ alkylcarbamyl, di(C₁₋₄ alkyl)carbamyl, carbamoyl, C₁₋₄ alkylcarbamoyl, di(C₁₋₄ alkyl)carbamoyl, C₁₋₄ alkylcarbonyl, C₁₋₄ alkoxycarbonyl, C₁₋₄ alkylcarbonylamino, C₁₋₄ alkylsulfonylamino, aminosulfonyl, C₁₋₄ alkylaminosulfonyl, di(C₁₋₄ alkyl)aminosulfonyl, aminosulfonylamino, C₁₋₄ alkylaminosulfonylamino, di(C₁₋₄ alkyl)aminosulfonylamino, aminocarbonylamino, C₁₋₄ alkylaminocarbonylamino, and di(C₁₋₄ alkyl)aminocarbonylamino.

In some embodiments, R^{21a} is methyl. In some embodiments, R^{20a} is ethyl. In some embodiments, R^{21a} is CH₂OH. In some embodiments, R^{20a} is CH₂CH₂OH. In some embodiments, R^{20a} is CH₂CH₂F. In some embodiments, R^{21a} is CH₂CHF₂. In some embodiments, R^{21a} is CH₂CH(CH₃)₂. In some embodiments, R^{21a} is 4-6 membered heterocycloalkyl. In some embodiments, R^{20a} is -(C₁₋₃ alkylene)-C₃₋₁₀ cycloalkyl. In some embodiments, R^{20a} is -(C₁₋₃ alkylene)-4-10 membered heterocycloalkyl. In some embodiments, R^{20a} is -(C₁₋₃ heteroalkylene)-C₃₋₁₀ cycloalkyl. In some embodiments, R^{20a} is -(C₁₋₃ heteroalkylene)-4-10 membered heterocycloalkyl. In some embodiments, the C₃₋₁₀ cycloalkyl is 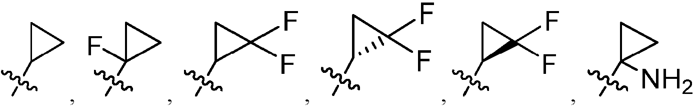 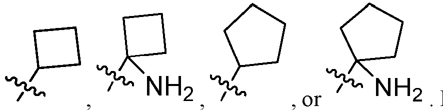 In some embodiments, the C₃₋₁₀ cycloalkyl is a 3-5 membered ring, which is optionally substituted. In some embodiments, the 4-10 membered heterocycloalkyl is 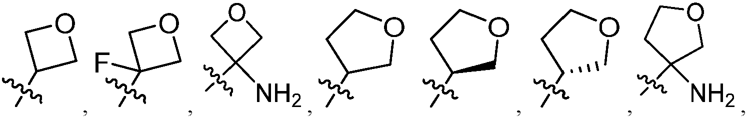 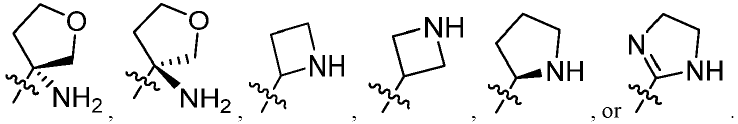 In some embodiments, the 4-10 membered heterocycloalkyl is a 4-5 membered ring, which is optionally substituted. In some embodiments, R^{10a} is C₁₋₄ heteroalkyl. In some embodiments, R^{20a} is C₁₋₄ alkyl. In some embodiments, R^{10a} is optionally substituted C₁₋₄heteroalkyl. In some embodiments, R^{20a} is optionally substituted C₁₋₄alkyl.

In some embodiments, R²⁴ is C₁₋₆ alkyl. In some embodiments, R²⁴ is methyl. In some embodiments, R²⁴ is halo. In some embodiments, R²⁴ is fluoro, bromo, or chloro. In some embodiments, R²⁴ is hydrogen. In some embodiments, R²⁴ is CN. In some embodiments, R²⁴ is C₃₋₁₀ cycloalkyl.

In some embodiments, the compound is of the Formula (IIIc): 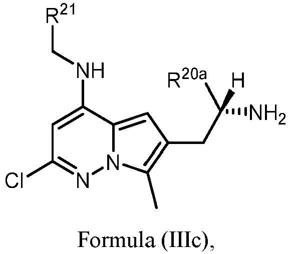 wherein
R²¹ has the meaning defined in Formula (I);
R^{20a} is selected from the group consisting of H, OH, SH, CN, NO₂, halo, C₁₋₄ alkyl, C₂₋₄ alkenyl, C₂₋₄ alkynyl, C₁₋₄ haloalkyl, C₁₋₄ cyanoalkyl, C₁₋₄ hydroxyalkyl, C₁₋₄ alkoxy, -(C₁₋₄ alkyl)-(C₁₋₄ alkoxy), -(C₁₋₄ alkoxy)-(C₁₋₄ alkoxy), C₁₋₄ haloalkoxy, C₃₋₆ cycloalkyl, C₁₋₄ heteroalkyl, phenyl, 5-6 membered heteroaryl, 4-6 membered heterocycloalkyl, -(C₁₋₃ alkylene)-C₃₋₁₀ cycloalkyl, -(C₁₋₃ alkylene)-4-10 membered heterocycloalkyl, -(C₁₋₃ heteroalkylene)-C₃₋₁₀ cycloalkyl, -(C₁₋₃ heteroalkylene)-4-10 membered heterocycloalkyl, amidinyl, amino, C₁₋₄ alkylamino, di(C₁₋₄ alkyl)amino, carbamyl, C₁₋₄ alkylcarbamyl, di(C₁₋₄ alkyl)carbamyl, carbamoyl, C₁₋₄ alkylcarbamoyl, di(C₁₋₄ alkyl)carbamoyl, C₁₋₄ alkylcarbonyl, C₁₋₄ alkoxycarbonyl, C₁₋₄ alkylcarbonylamino, C₁₋₄ alkylsulfonylamino, aminosulfonyl, C₁₋₄ alkylaminosulfonyl, di(C₁₋₄ alkyl)aminosulfonyl, aminosulfonylamino, C₁₋₄ alkylaminosulfonylamino, di(C₁₋₄ alkyl)aminosulfonylamino, aminocarbonylamino, C₁₋₄ alkylaminocarbonylamino, and di(C₁₋₄ alkyl)aminocarbonylamino, wherein each of the cycloalkyl and heterocycloalkyl is optionally substituted by 1, 2, 3, or 4 substituents independently selected from halo, CN, SH, -CN, oxo, NO₂, OH, C₁₋₄ alkyl, C₂₋₄ alkenyl, C₂₋₄ alkynyl, C₁₋₄ haloalkyl, C₁₋₄ cyanoalkyl, C₁₋₄ aminoalkyl, C₁₋₄ hydroxyalkyl, C₁₋₄ alkoxy, and amino, or a pharmaceutically acceptable salt thereof.

In some embodiments of the compound of the Formula (IIIc) or a pharmaceutically acceptable salt thereof, R^{20a} is selected from the group consisting of OH, SH, CN, NO₂, halo, C₁₋₄ alkyl, C₂₋₄ alkenyl, C₂₋₄ alkynyl, C₁₋₄ haloalkyl, C₁₋₄ cyanoalkyl, C₁₋₄ hydroxyalkyl, C₁₋₄ alkoxy, -(C₁₋₄ alkyl)-(C₁₋₄ alkoxy), -(C₁₋₄ alkoxy)-(C₁₋₄ alkoxy), C₁₋₄ haloalkoxy, C₃₋₆ cycloalkyl, phenyl, 5-6 membered heteroaryl, 5-6 membered heterocycloalkyl, amino, C₁₋₄ alkylamino, di(C₁₋₄ alkyl)amino, carbamyl, C₁₋₄ alkylcarbamyl, di(C₁₋₄ alkyl)carbamyl, carbamoyl, C₁₋₄ alkylcarbamoyl, di(C₁₋₄ alkyl)carbamoyl, C₁₋₄ alkylcarbonyl, C₁₋₄ alkoxycarbonyl, C₁₋₄ alkylcarbonylamino, C₁₋₄ alkylsulfonylamino, aminosulfonyl, C₁₋₄ alkylaminosulfonyl, di(C₁₋₄ alkyl)aminosulfonyl, aminosulfonylamino, C₁₋₄ alkylaminosulfonylamino, di(C₁₋₄ alkyl)aminosulfonylamino, aminocarbonylamino, C₁₋₄ alkylaminocarbonylamino, and di(C₁₋₄ alkyl)aminocarbonylamino.

In some embodiments, R^{20a} is methyl. In some embodiments, R^{20a} is ethyl. In some embodiments, R^{20a} is CH₂OH. In some embodiments, R^{20a} is CH₂CH₂OH. In some embodiments, R^{20a} is CH₂CH₂F. In some embodiments, R^{20a} is CH₂CHF₂. In some embodiments, R^{20a} is CH₂CH(CH₃)₂. In some embodiments, R^{20a} is 4-6 membered heterocycloalkyl. In some embodiments, R^{20a} is -(C₁₋₃ alkylene)-C₃₋₁₀ cycloalkyl. In some embodiments, R^{20a} is -(C₁₋₃ alkylene)-4-10 membered heterocycloalkyl. In some embodiments, R^{20a} is -(C₁₋₃ heteroalkylene)-C₃₋₁₀ cycloalkyl. In some embodiments, R^{20a} is -(C₁₋₃ heteroalkylene)-4-10 membered heterocycloalkyl. In some embodiments, the C₃₋₁₀ cycloalkyl is 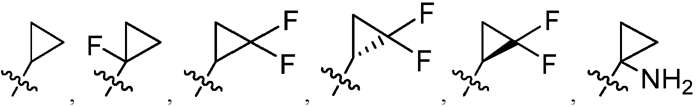 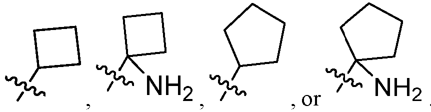 In some embodiments, the C₃₋₁₀ cycloalkyl is a 3-5 membered ring, which is optionally substituted. In some embodiments, the 4-10 membered heterocycloalkyl is 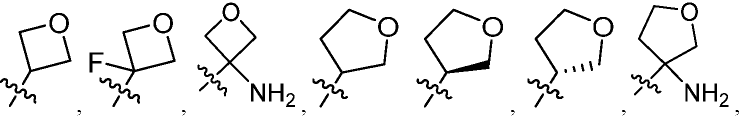 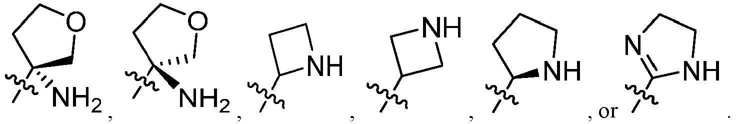 In some embodiments, the 4-10 membered heterocycloalkyl is a 4-5 membered ring, which is optionally substituted. In some embodiments, R^{20a} is C₁₋₄ heteroalkyl. In some embodiments, R^{20a} is C₁₋₄ alkyl. In some embodiments, R^{20a} is optionally substituted C₁₋₄heteroalkyl. In some embodiments, R^{20a} is optionally substituted C₁₋₄ alkyl.

In some embodiments, the compound is of the Formula (IIId): 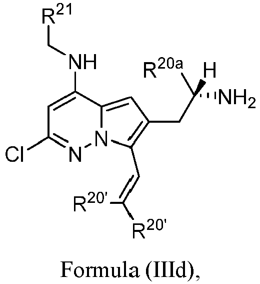 wherein
R²¹ has the meaning defined in Formula (I);
R^{20a} is selected from the group consisting of H, OH, SH, CN, NO₂, halo, C₁₋₄ alkyl, C₂₋₄ alkenyl, C₂₋₄ alkynyl, C₁₋₄ haloalkyl, C₁₋₄ cyanoalkyl, C₁₋₄ hydroxyalkyl, C₁₋₄ alkoxy, -(C₁₋₄ alkyl)-(C₁₋₄ alkoxy), -(C₁₋₄ alkoxy)-(C₁₋₄ alkoxy), C₁₋₄ haloalkoxy, C₃₋₆ cycloalkyl, C₁₋₄ heteroalkyl, phenyl, 5-6 membered heteroaryl, 4-6 membered heterocycloalkyl, -(C₁₋₃ alkylene)-C₃₋₁₀ cycloalkyl, -(C₁₋₃ alkylene)-4-10 membered heterocycloalkyl, -(C₁₋₃ heteroalkylene)-C₃₋₁₀ cycloalkyl, -(C₁₋₃ heteroalkylene)-4-10 membered heterocycloalkyl, amidinyl, amino, C₁₋₄ alkylamino, di(C₁₋₄ alkyl)amino, carbamyl, C₁₋₄ alkylcarbamyl, di(C₁₋₄ alkyl)carbamyl, carbamoyl, C₁₋₄ alkylcarbamoyl, di(C₁₋₄ alkyl)carbamoyl, C₁₋₄ alkylcarbonyl, C₁₋₄ alkoxycarbonyl, C₁₋₄ alkylcarbonylamino, C₁₋₄ alkylsulfonylamino, aminosulfonyl, C₁₋₄ alkylaminosulfonyl, di(C₁₋₄ alkyl)aminosulfonyl, aminosulfonylamino, C₁₋₄ alkylaminosulfonylamino, di(C₁₋₄ alkyl)aminosulfonylamino, aminocarbonylamino, C₁₋₄ alkylaminocarbonylamino, and di(C₁₋₄ alkyl)aminocarbonylamino, wherein each of the cycloalkyl and heterocycloalkyl is optionally substituted by 1, 2, 3, or 4 substituents independently selected from halo, CN, SH, -CN, oxo, NO₂, OH, C₁₋₄ alkyl, C₂₋₄ alkenyl, C₂₋₄ alkynyl, C₁₋₄ haloalkyl, C₁₋₄ cyanoalkyl, C₁₋₄ aminoalkyl, C₁₋₄ hydroxyalkyl, C₁₋₄ alkoxy, and amino,
wherein each of R^{20'} is independently H or R²⁰ as disclosed herein,
or a pharmaceutically acceptable salt thereof.

In some embodiments, R^{20a} is methyl. In some embodiments, R^{20a} is ethyl. In some embodiments, R^{20a} is CH₂OH. In some embodiments, R^{20a} is CH₂CH₂OH. In some embodiments, R^{20a} is CH₂CH₂F. In some embodiments, R^{20a} is CH₂CHF₂. In some embodiments, R^{20a} is CH₂CH(CH₃)₂. In some embodiments, R^{20a} is 4-6 membered heterocycloalkyl. In some embodiments, R^{20a} is -(C₁₋₃ alkylene)-C₃₋₁₀ cycloalkyl. In some embodiments, R^{20a} is -(C₁₋₃ alkylene)-4-10 membered heterocycloalkyl. In some embodiments, R^{20a} is -(C₁₋₃ heteroalkylene)-C₃₋₁₀ cycloalkyl. In some embodiments, R^{20a} is -(C₁₋₃ heteroalkylene)-4-10 membered heterocycloalkyl. In some embodiments, the C₃₋₁₀ cycloalkyl is 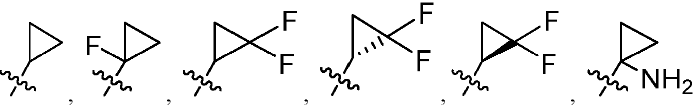 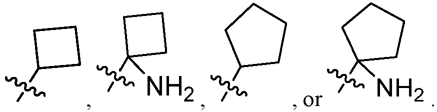 In some embodiments, the C₃₋₁₀ cycloalkyl is a 3-5 membered ring, which is optionally substituted. In some embodiments, the 4-10 membered heterocycloalkyl is 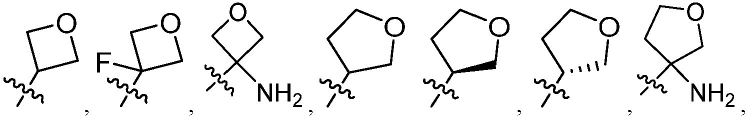 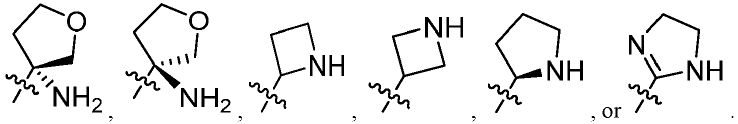 In some embodiments, the 4-10 membered heterocycloalkyl is a 4-5 membered ring, which is optionally substituted. In some embodiments, R^{20a} is C₁₋₄ heteroalkyl. In some embodiments, R^{20a} is C₁₋₄ alkyl. In some embodiments, R^{20a} is optionally substituted C₁₋₄heteroalkyl. In some embodiments, R^{20a} is optionally substituted C₁₋₄ alkyl.

In some embodiments, the compound is of the Formula (IIIe): 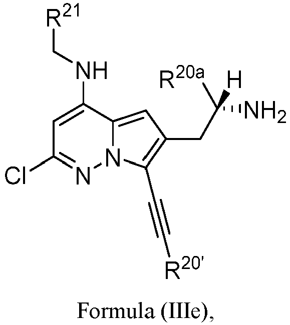 wherein
R²¹ has the meaning defined in Formula (I);
R^{20a} is selected from the group consisting of H, OH, SH, CN, NO₂, halo, C₁₋₄ alkyl, C₂₋₄ alkenyl, C₂₋₄ alkynyl, C₁₋₄ haloalkyl, C₁₋₄ cyanoalkyl, C₁₋₄ hydroxyalkyl, C₁₋₄ alkoxy, -(C₁₋₄ alkyl)-(C₁₋₄ alkoxy), -(C₁₋₄ alkoxy)-(C₁₋₄ alkoxy), C₁₋₄ haloalkoxy, C₃₋₆ cycloalkyl, C₁₋₄ heteroalkyl, phenyl, 5-6 membered heteroaryl, 4-6 membered heterocycloalkyl, -(C₁₋₃ alkylene)-C₃₋₁₀ cycloalkyl, -(C₁₋₃ alkylene)-4-10 membered heterocycloalkyl, -(C₁₋₃ heteroalkylene)-C₃₋₁₀ cycloalkyl, -(C₁₋₃ heteroalkylene)-4-10 membered heterocycloalkyl, amidinyl, amino, C₁₋₄ alkylamino, di(C₁₋₄ alkyl)amino, carbamyl, C₁₋₄ alkylcarbamyl, di(C₁₋₄ alkyl)carbamyl, carbamoyl, C₁₋₄ alkylcarbamoyl, di(C₁₋₄ alkyl)carbamoyl, C₁₋₄ alkylcarbonyl, C₁₋₄ alkoxycarbonyl, C₁₋₄ alkylcarbonylamino, C₁₋₄ alkylsulfonylamino, aminosulfonyl, C₁₋₄ alkylaminosulfonyl, di(C₁₋₄ alkyl)aminosulfonyl, aminosulfonylamino, C₁₋₄ alkylaminosulfonylamino, di(C₁₋₄ alkyl)aminosulfonylamino, aminocarbonylamino, C₁₋₄ alkylaminocarbonylamino, and di(C₁₋₄ alkyl)aminocarbonylamino, wherein each of the cycloalkyl and heterocycloalkyl is optionally substituted by 1, 2, 3, or 4 substituents independently selected from halo, CN, SH, -CN, oxo, NO₂, OH, C₁₋₄ alkyl, C₂₋₄ alkenyl, C₂₋₄ alkynyl, C₁₋₄ haloalkyl, C₁₋₄ cyanoalkyl, C₁₋₄ aminoalkyl, C₁₋₄ hydroxyalkyl, C₁₋₄ alkoxy, and amino,
wherein R^{20'} is H or R²⁰ as disclosed herein,
or a pharmaceutically acceptable salt thereof.

In some embodiments, R^{20a} is methyl. In some embodiments, R^{20a} is ethyl. In some embodiments, R^{20a} is CH₂OH. In some embodiments, R^{20a} is CH₂CH₂OH. In some embodiments, R^{20a} is CH₂CH₂F. In some embodiments, R^{20a} is CH₂CHF₂. In some embodiments, R^{20a} is CH₂CH(CH₃)₂. In some embodiments, R^{20a} is 4-6 membered heterocycloalkyl. In some embodiments, R^{20a} is -(C₁₋₃ alkylene)-C₃₋₁₀ cycloalkyl. In some embodiments, R^{20a} is -(C₁₋₃ alkylene)-4-10 membered heterocycloalkyl. In some embodiments, R^{20a} is -(C₁₋₃ heteroalkylene)-C₃₋₁₀ cycloalkyl. In some embodiments, R^{20a} is -(C₁₋₃ heteroalkylene)-4-10 membered heterocycloalkyl. In some embodiments, the C₃₋₁₀ cycloalkyl is 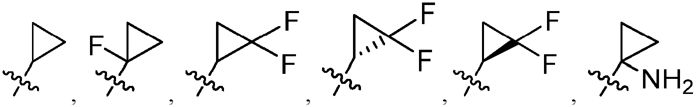 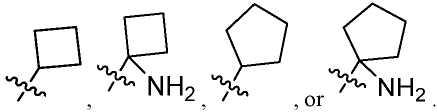 In some embodiments, the C₃₋₁₀ cycloalkyl is a 3-5 membered ring, which is optionally substituted. In some embodiments, the 4-10 membered heterocycloalkyl is 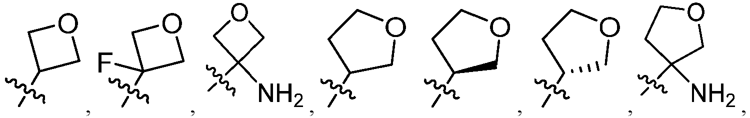 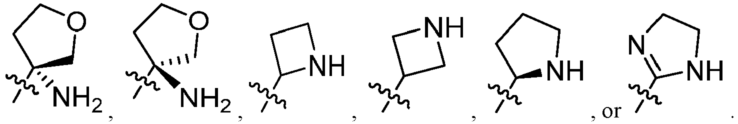 In some embodiments, the 4-10 membered heterocycloalkyl is a 4-5 membered ring, which is optionally substituted. In some embodiments, R^{20a} is C₁₋₄ heteroalkyl. In some embodiments, R^{20a} is C₁₋₄ alkyl. In some embodiments, R^{10a} is optionally substituted C₁₋₄heteroalkyl. In some embodiments, R^{20a} is optionally substituted C₁₋₄ alkyl.

In some embodiments, the compound is of the Formula (IIIf): 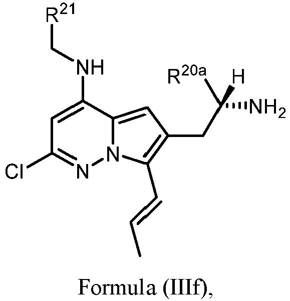 wherein
R²¹ has the meaning defined in Formula (I);
R^{20a} is selected from the group consisting of H, OH, SH, CN, NO₂, halo, C₁₋₄ alkyl, C₂₋₄ alkenyl, C₂₋₄ alkynyl, C₁₋₄ haloalkyl, C₁₋₄ cyanoalkyl, C₁₋₄ hydroxyalkyl, C₁₋₄ alkoxy, -(C₁₋₄ alkyl)-(C₁₋₄ alkoxy), -(C₁₋₄ alkoxy)-(C₁₋₄ alkoxy), C₁₋₄ haloalkoxy, C₃₋₆ cycloalkyl, C₁₋₄ heteroalkyl, phenyl, 5-6 membered heteroaryl, 4-6 membered heterocycloalkyl, -(C₁₋₃ alkylene)-C₃₋₁₀ cycloalkyl, -(C₁₋₃ alkylene)-4-10 membered heterocycloalkyl, -(C₁₋₃ heteroalkylene)-C₃₋₁₀ cycloalkyl, -(C₁₋₃ heteroalkylene)-4-10 membered heterocycloalkyl, amidinyl, amino, C₁₋₄ alkylamino, di(C₁₋₄ alkyl)amino, carbamyl, C₁₋₄ alkylcarbamyl, di(C₁₋₄ alkyl)carbamyl, carbamoyl, C₁₋₄ alkylcarbamoyl, di(C₁₋₄ alkyl)carbamoyl, C₁₋₄ alkylcarbonyl, C₁₋₄ alkoxycarbonyl, C₁₋₄ alkylcarbonylamino, C₁₋₄ alkylsulfonylamino, aminosulfonyl, C₁₋₄ alkylaminosulfonyl, di(C₁₋₄ alkyl)aminosulfonyl, aminosulfonylamino, C₁₋₄ alkylaminosulfonylamino, di(C₁₋₄ alkyl)aminosulfonylamino, aminocarbonylamino, C₁₋₄ alkylaminocarbonylamino, and di(C₁₋₄ alkyl)aminocarbonylamino, wherein each of the cycloalkyl and heterocycloalkyl is optionally substituted by 1, 2, 3, or 4 substituents independently selected from halo, CN, SH, -CN, oxo, NO₂, OH, C₁₋₄ alkyl, C₂₋₄ alkenyl, C₂₋₄ alkynyl, C₁₋₄ haloalkyl, C₁₋₄ cyanoalkyl, C₁₋₄ aminoalkyl, C₁₋₄ hydroxyalkyl, C₁₋₄ alkoxy, and amino,
or a pharmaceutically acceptable salt thereof.

In some embodiments, R^{20a} is methyl. In some embodiments, R^{20a} is ethyl. In some embodiments, R^{20a} is CH₂OH. In some embodiments, R^{20a} is CH₂CH₂OH. In some embodiments, R^{20a} is CH₂CH₂F. In some embodiments, R^{20a} is CH₂CHF₂. In some embodiments, R^{20a} is CH₂CH(CH₃)₂. In some embodiments, R^{20a} is 4-6 membered heterocycloalkyl. In some embodiments, R^{20a} is -(C₁₋₃ alkylene)-C₃₋₁₀ cycloalkyl. In some embodiments, R^{20a} is -(C₁₋₃ alkylene)-4-10 membered heterocycloalkyl. In some embodiments, R^{20a} is -(C₁₋₃ heteroalkylene)-C₃₋₁₀ cycloalkyl. In some embodiments, R^{20a} is -(C₁₋₃ heteroalkylene)-4-10 membered heterocycloalkyl. In some embodiments, the C₃₋₁₀ cycloalkyl is 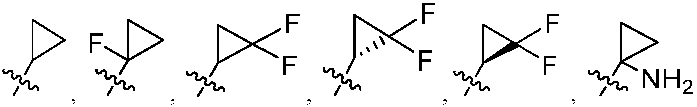 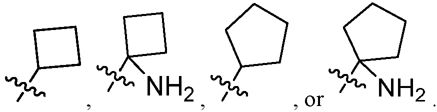 In some embodiments, the C₃₋₁₀ cycloalkyl is a 3-5 membered ring, which is optionally substituted. In some embodiments, the 4-10 membered heterocycloalkyl is 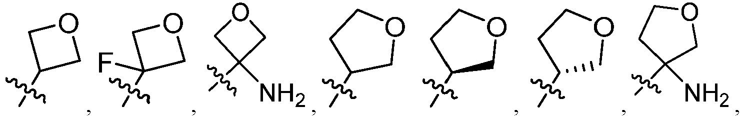 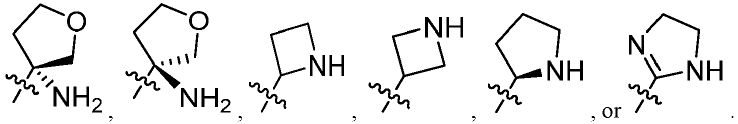 In some embodiments, the 4-10 membered heterocycloalkyl is a 4-5 membered ring, which is optionally substituted. In some embodiments, R^{20a} is C₁₋₄ heteroalkyl. In some embodiments, R^{20a} is C₁₋₄ alkyl. In some embodiments, R^{20a} is optionally substituted C₁₋₄heteroalkyl. In some embodiments, R^{20a} is optionally substituted C₁₋₄ alkyl.

In some embodiments, the compound is of the Formula (IIIg): 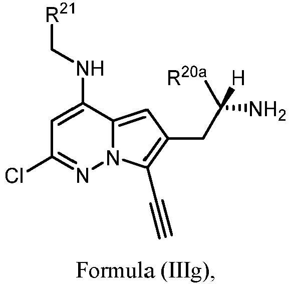 wherein
R²¹ has the meaning defined in Formula (I);
R^{20a} is selected from the group consisting of H, OH, SH, CN, NO₂, halo, C₁₋₄ alkyl, C₂₋₄ alkenyl, C₂₋₄ alkynyl, C₁₋₄ haloalkyl, C₁₋₄ cyanoalkyl, C₁₋₄ hydroxyalkyl, C₁₋₄ alkoxy, -(C₁₋₄ alkyl)-(C₁₋₄ alkoxy), -(C₁₋₄ alkoxy)-(C₁₋₄ alkoxy), C₁₋₄ haloalkoxy, C₃₋₆ cycloalkyl, C₁₋₄ heteroalkyl, phenyl, 5-6 membered heteroaryl, 4-6 membered heterocycloalkyl, -(C₁₋₃ alkylene)-C₃₋₁₀ cycloalkyl, -(C₁₋₃ alkylene)-4-10 membered heterocycloalkyl, -(C₁₋₃ heteroalkylene)-C₃₋₁₀ cycloalkyl, -(C₁₋₃ heteroalkylene)-4-10 membered heterocycloalkyl, amidinyl, amino, C₁₋₄ alkylamino, di(C₁₋₄ alkyl)amino, carbamyl, C₁₋₄ alkylcarbamyl, di(C₁₋₄ alkyl)carbamyl, carbamoyl, C₁₋₄ alkylcarbamoyl, di(C₁₋₄ alkyl)carbamoyl, C₁₋₄ alkylcarbonyl, C₁₋₄ alkoxycarbonyl, C₁₋₄ alkylcarbonylamino, C₁₋₄ alkylsulfonylamino, aminosulfonyl, C₁₋₄ alkylaminosulfonyl, di(C₁₋₄ alkyl)aminosulfonyl, aminosulfonylamino, C₁₋₄ alkylaminosulfonylamino, di(C₁₋₄ alkyl)aminosulfonylamino, aminocarbonylamino, C₁₋₄ alkylaminocarbonylamino, and di(C₁₋₄ alkyl)aminocarbonylamino, wherein each of the cycloalkyl and heterocycloalkyl is optionally substituted by 1, 2, 3, or 4 substituents independently selected from halo, CN, SH, -CN, oxo, NO₂, OH, C₁₋₄ alkyl, C₂₋₄ alkenyl, C₂₋₄ alkynyl, C₁₋₄ haloalkyl, C₁₋₄ cyanoalkyl, C₁₋₄ aminoalkyl, C₁₋₄ hydroxyalkyl, C₁₋₄ alkoxy, and amino,
or a pharmaceutically acceptable salt thereof.

In some embodiments, R^{21a} is methyl. In some embodiments, R^{20a} is ethyl. In some embodiments, R^{20a} is CH₂OH. In some embodiments, R^{20a} is CH₂CH₂OH. In some embodiments, R^{20a} is CH₂CH₂F. In some embodiments, R^{20a} is CH₂CHF₂. In some embodiments, R^{20a} is CH₂CH(CH₃)₂. In some embodiments, R^{20a} is 4-6 membered heterocycloalkyl. In some embodiments, R^{20a} is -(C₁₋₃ alkylene)-C₃₋₁₀ cycloalkyl. In some embodiments, R^{20a} is -(C₁₋₃ alkylene)-4-10 membered heterocycloalkyl. In some embodiments, R^{20a} is -(C₁₋₃ heteroalkylene)-C₃₋₁₀ cycloalkyl. In some embodiments, R^{20a} is -(C₁₋₃ heteroalkylene)-4-10 membered heterocycloalkyl. In some embodiments, the C₃₋₁₀ cycloalkyl is 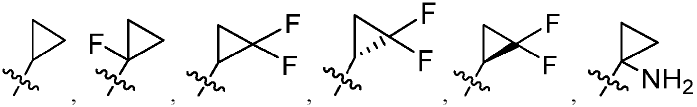 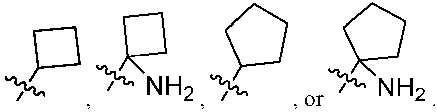 In some embodiments, the C₃₋₁₀ cycloalkyl is a 3-5 membered ring, which is optionally substituted. In some embodiments, the 4-10 membered heterocycloalkyl is 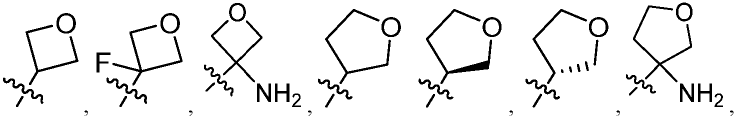 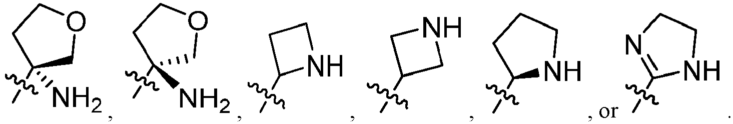 In some embodiments, the 4-10 membered heterocycloalkyl is a 4-5 membered ring, which is optionally substituted. In some embodiments, R^{10a} is C₁₋₄ heteroalkyl. In some embodiments, R^{20a} is C₁₋₄ alkyl. In some embodiments, R^{10a} is optionally substituted C₁₋₄heteroalkyl. In some embodiments, R^{20a} is optionally substituted C₁₋₄ alkyl.

In some embodiments, the compound is selected from Table 1.

In some embodiments, a SMSM described herein, possesses one or more stereocenters and each stereocenter exists independently in either the R or S configuration. The compounds presented herein include all diastereomeric, enantiomeric, and epimeric forms as well as the appropriate mixtures thereof. The compounds and methods provided herein include all cis, trans, syn, anti, entgegen (E), and zusammen (Z) isomers as well as the appropriate mixtures thereof. In certain embodiments, compounds described herein are prepared as their individual stereoisomers by reacting a racemic mixture of the compound with an optically active resolving agent to form a pair of diastereoisomeric compounds/salts, separating the diastereomers and recovering the optically pure enantiomers. In some embodiments, resolution of enantiomers is carried out using covalent diastereomeric derivatives of the compounds described herein. In another embodiment, diastereomers are separated by separation/resolution techniques based upon differences in solubility. In other embodiments, separation of stereoisomers is performed by chromatography or by the forming diastereomeric salts and separation by recrystallization, or chromatography, or any combination thereof (See, for example, Jean Jacques, Andre Collet, Samuel H. Wilen, "Enantiomers, Racemates and Resolutions", John Wiley and Sons, Inc., 1981.) In one aspect, stereoisomers are obtained by stereoselective synthesis.

In some embodiments, compounds described herein are prepared as prodrugs. A "prodrug" refers to an agent that is converted into the parent drug *in vivo.* Prodrugs are often useful because, in some situations, they may be easier to administer than the parent drug. They may, for instance, be bioavailable by oral administration whereas the parent is not. The prodrug may also have improved solubility in pharmaceutical compositions over the parent drug. In some embodiments, the design of a prodrug increases the effective water solubility. An example, without limitation, of a prodrug is a compound described herein, which is administered as an ester (the "prodrug") to facilitate transmittal across a cell membrane where water solubility is detrimental to mobility, but which then is metabolically hydrolyzed to the carboxylic acid, the active entity, once inside the cell where water-solubility is beneficial. A further example of a prodrug might be a short peptide (polyaminoacid) bonded to an acid group where the peptide is metabolized to reveal the active moiety. In certain embodiments, upon *in vivo* administration, a prodrug is chemically converted to the biologically, pharmaceutically or therapeutically active form of the compound. In certain embodiments, a prodrug is enzymatically metabolized by one or more steps or processes to the biologically, pharmaceutically or therapeutically active form of the compound.

In one aspect, prodrugs are designed to alter the metabolic stability or the transport characteristics of a drug, to mask side effects or toxicity, to improve the flavor of a drug or to alter other characteristics or properties of a drug. By virtue of knowledge of pharmacokinetic, pharmacodynamic processes and drug metabolism *in vivo,* once a pharmaceutically active compound is known, the design of prodrugs of the compound is possible. (see, for example, Nogrady (1985) Medicinal Chemistry A Biochemical Approach, Oxford University Press, New York, pages 388-392; Silverman (1992), The Organic Chemistry of Drug Design and Drug Action, Academic Press, Inc., San Diego, pages 352-401, Rooseboom et al., Pharmacological Reviews, 56:53-102, 2004; Aesop Cho, "Recent Advances in Oral Prodrug Discovery", Annual Reports in Medicinal Chemistry, Vol. 41, 395-407, 2006; T. Higuchi and V. Stella, Pro-drugs as Novel Delivery Systems, Vol. 14 of the A.C.S. Symposium Series).

In some cases, some of the herein-described compounds may be a prodrug for another derivative or active compound.

In some embodiments, sites on the aromatic ring portion of compounds described herein are susceptible to various metabolic reactions Therefore incorporation of appropriate substituents on the aromatic ring structures will reduce, minimize or eliminate this metabolic pathway. In specific embodiments, the appropriate substituent to decrease or eliminate the susceptibility of the aromatic ring to metabolic reactions is, by way of example only, a halogen, or an alkyl group.

In another embodiment, the compounds described herein are labeled isotopically (e.g. with a radioisotope) or by other means, including, but not limited to, the use of chromophores or fluorescent moieties, bioluminescent labels, or chemiluminescent labels.

Compounds described herein include isotopically labeled compounds, which are identical to those recited in the various formulae and structures presented herein, but for the fact that one or more atoms are replaced by an atom having an atomic mass or mass number different from the atomic mass or mass number usually found in nature. Examples of isotopes that can be incorporated into the present compounds include isotopes of hydrogen, carbon, nitrogen, oxygen, sulfur, fluorine and chlorine, such as, for example, ²H, ³H, ¹³C, ¹⁴C, ¹⁵N, ¹⁸O, ¹⁷O, ³⁵S, ¹⁸F, and ³⁶Cl. In one aspect, isotopically labeled compounds described herein, for example those into which radioactive isotopes such as ³H and ¹⁴C are incorporated, are useful in drug and/or substrate tissue distribution assays. In one aspect, substitution with isotopes such as deuterium affords certain therapeutic advantages resulting from greater metabolic stability, such as, for example, increased *in vivo* half-life or reduced dosage requirements.

In additional or further embodiments, the compounds described herein are metabolized upon administration to an organism in need to produce a metabolite that is then used to produce a desired effect, including a desired therapeutic effect.

Compounds described herein may be formed as, and/or used as, pharmaceutically acceptable salts. The type of pharmaceutical acceptable salts, include, but are not limited to: (1) acid addition salts, formed by reacting the free base form of the compound with a pharmaceutically acceptable: inorganic acid, such as, for example, hydrochloric acid, hydrobromic acid, sulfuric acid, phosphoric acid, metaphosphoric acid, and the like; or with an organic acid, such as, for example, acetic acid, propionic acid, hexanoic acid, cyclopentanepropionic acid, glycolic acid, pyruvic acid, lactic acid, malonic acid, succinic acid, malic acid, maleic acid, fumaric acid, trifluoroacetic acid, tartaric acid, citric acid, benzoic acid, 3-(4-hydroxybenzoyl)benzoic acid, cinnamic acid, mandelic acid, methanesulfonic acid, ethanesulfonic acid, 1,2-ethanedisulfonic acid, 2-hydroxyethanesulfonic acid, benzenesulfonic acid, toluenesulfonic acid, 2-naphthalenesulfonic acid, 4-methylbicyclo-[2.2.2]oct-2-ene-1-carboxylic acid, glucoheptonic acid, 4,4'-methylenebis-(3-hydroxy-2-ene-1-carboxylic acid), 3-phenylpropionic acid, trimethylacetic acid, tertiary butylacetic acid, lauryl sulfuric acid, gluconic acid, glutamic acid, hydroxynaphthoic acid, salicylic acid, stearic acid, muconic acid, butyric acid, phenylacetic acid, phenylbutyric acid, valproic acid, and the like; (2) salts formed when an acidic proton present in the parent compound is replaced by a metal ion, *e.g.,* an alkali metal ion *(e.g.* lithium, sodium, potassium), an alkaline earth ion (e.g. magnesium, or calcium), or an aluminum ion. In some cases, compounds described herein may coordinate with an organic base, such as, but not limited to, ethanolamine, diethanolamine, triethanolamine, tromethamine, N-methylglucamine, dicyclohexylamine, tris(hydroxymethyl)methylamine. In other cases, compounds described herein may form salts with amino acids such as, but not limited to, arginine, lysine, and the like. Acceptable inorganic bases used to form salts with compounds that include an acidic proton, include, but are not limited to, aluminum hydroxide, calcium hydroxide, potassium hydroxide, sodium carbonate, sodium hydroxide, and the like.

**In** some embodiments, the compounds provided herein can exist in unsolvated as well as solvated forms.

**In** some embodiments, a SMSM has a molecular weight of at most about 2000 Daltons, 1500 Daltons, 1000 Daltons or 900 Daltons. In some embodiments, a SMSM has a molecular weight of at least 100 Daltons, 200 Daltons, 300 Daltons, 400 Daltons or 500 Daltons. In some embodiments, a SMSM does not comprise a phosphodiester linkage. In some embodiments, a SMSM is a compound with a structure set forth in Table 1 below.

**Table 1: Exemplary SMSM compounds**

| **Compound** | **Structure** | **Molecular Weight (g/mol)** |
|---|---|---|
| **1** | 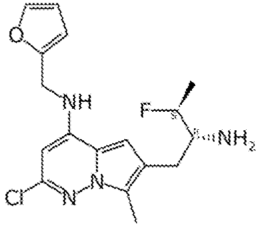 | 350.825 |
| **2** | 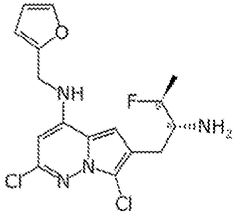 | 371.243 |
| **3** | 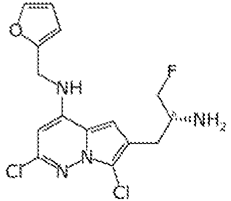 | 357.216 |
| **4** | 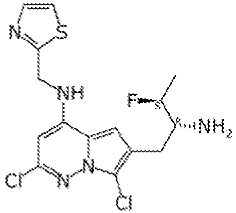 | 388.299 |

### Pharmaceutical Compositions

In some embodiments, the compounds described herein are formulated into pharmaceutical compositions. Pharmaceutical compositions are formulated in a conventional manner using one or more pharmaceutically acceptable inactive ingredients that facilitate processing of the active compounds into preparations that can be used pharmaceutically. Proper formulation is dependent upon the route of administration chosen. A summary of pharmaceutical compositions described herein can be found, for example, in Remington: The Science and Practice of Pharmacy, Nineteenth Ed (Easton, Pa.: Mack Publishing Company, 1995); Hoover, John E., Remington's Pharmaceutical Sciences, Mack Publishing Co., Easton, Pennsylvania 1975; Liberman, H.A. and Lachman, L., Eds., Pharmaceutical Dosage Forms, Marcel Decker, New York, N.Y., 1980; and Pharmaceutical Dosage Forms and Drug Delivery Systems, Seventh Ed. (Lippincott Williams & Wilkins, 1999).

A pharmaceutical composition can be a mixture of a SMSM described herein with one or more other chemical components (*i.e.,* pharmaceutically acceptable ingredients), such as carriers, excipients, binders, filling agents, suspending agents, flavoring agents, sweetening agents, disintegrating agents, dispersing agents, surfactants, lubricants, colorants, diluents, solubilizers, moistening agents, plasticizers, stabilizers, penetration enhancers, wetting agents, anti-foaming agents, antioxidants, preservatives, or one or more combination thereof. The pharmaceutical composition facilitates administration of the compound to an organism.

The compositions described herein can be administered to the subject in a variety of ways, including parenterally, intravenously, intradermally, intramuscularly, colonically, rectally, or intraperitoneally. In some embodiments, the small molecule splicing modulator, or a pharmaceutically acceptable salt thereof is administered by intraperitoneal injection, intramuscular injection, subcutaneous injection, or intravenous injection of the subject. In some embodiments, the pharmaceutical compositions can be administered parenterally, intravenously, intramuscularly or orally. The oral agents comprising a small molecule splicing modulator can be in any suitable form for oral administration, such as liquid, tablets, capsules, or the like. The oral formulations can be further coated or treated to prevent or reduce dissolution in stomach. The compositions of the present disclosure can be administered to a subject using any suitable methods known in the art. Suitable formulations for use in the present disclosure and methods of delivery are generally well known in the art. For example, the small molecule splicing modulators described herein can be formulated as pharmaceutical compositions with a pharmaceutically acceptable diluent, carrier, or excipient. The compositions may contain pharmaceutically acceptable auxiliary substances as required to approximate physiological conditions including pH adjusting and buffering agents, tonicity adjusting agents, wetting agents and the like, such as, for example, sodium acetate, sodium lactate, sodium chloride, potassium chloride, calcium chloride, sorbitan monolaurate, triethanolamine oleate, etc.

In some embodiments, the pharmaceutical formulation is in the form of a tablet. In other embodiments, pharmaceutical formulations containing a SMSM described herein are in the form of a capsule. In one aspect, liquid formulation dosage forms for oral administration are in the form of aqueous suspensions or solutions selected from the group including, but not limited to, aqueous oral dispersions, emulsions, solutions, elixirs, gels, and syrups.

For administration by inhalation, a SMSM described herein can be formulated for use as an aerosol, a mist, or a powder. For buccal or sublingual administration, the compositions may take the form of tablets, lozenges, or gels formulated in a conventional manner. In some embodiments, a SMSM described herein can be prepared as transdermal dosage forms. In some embodiments, a SMSM described herein can be formulated into a pharmaceutical composition suitable for intramuscular, subcutaneous, or intravenous injection. In some embodiments, a SMSM described herein can be administered topically and can be formulated into a variety of topically administrable compositions, such as solutions, suspensions, lotions, gels, pastes, medicated sticks, balms, creams, or ointments. In some embodiments, a SMSM described herein can be formulated in rectal compositions such as enemas, rectal gels, rectal foams, rectal aerosols, suppositories, jelly suppositories, or retention enemas.

In some embodiments, disclosed herein is a pharmaceutical composition comprising a compound of the disclosure or a pharmaceutically acceptable salt thereof, and a pharmaceutically acceptable excipient or carrier.

### Splicing Modulation of Target Gene Products

The present disclosure contemplates use of small molecules with favorable drug properties that modulate the activity of splicing of a target RNA. Provided herein are small molecule splicing modulators (SMSMs) that modulate splicing of a polynucleotide. In some embodiments, the SMSMs bind and modulate target RNA. In some embodiments, provided herein is a library of SMSMs that bind and modulate one or more target RNAs. In some embodiments, the target RNA is mRNA. In some embodiments, the target RNA is a noncoding RNA. In some embodiments, the target RNA is a pre-mRNA. In some embodiments, the target RNA is hnRNA. In some embodiments, the small molecules modulate splicing of the target RNA. In some embodiments, a small molecule provided herein modulates splicing at a sequence of the target RNA. In some embodiments, a small molecule provided herein modulates splicing at a cryptic splice site sequence of the target RNA. In some embodiments, a small molecule provided herein modulates splicing at an alternative splice site sequence of the target RNA. In some embodiments, a small molecule provided herein modulates splicing at a native splice site sequence of the target RNA. In some embodiments, a small molecule provided herein binds to a target RNA. In some embodiments, a small molecule provided herein binds to a splicing complex or a component thereof. In some embodiments, a small molecule provided herein binds to a target RNA and a splicing complex or a component thereof. In some embodiments, a small molecule provided herein modulates binding affinity of a splicing complex component to a target RNA such as a pre-mRNA. In some embodiments, a small molecule provided herein modulates binding affinity of a splicing complex component to a target RNA such as a pre-mRNA at a splice site sequence. In some embodiments, a small molecule provided herein modulates binding affinity of a splicing complex component to a target RNA such as a pre-mRNA upstream of a splice site sequence or downstream of a splice site sequence.

Described herein are compounds modifying splicing of gene products, such as Ataxin 3 pre-mRNA for use in the treatment, prevention, and/or delay of progression of diseases or conditions.

In some embodiments, described herein, is a method of treating, preventing, delaying of progress, or ameliorating symptoms of a disease or a condition associated with Ataxin 3 (ATXN3) expression level or activity level in a subject in need thereof, comprising administering a therapeutically effective amount of a small molecule splicing modulator (SMSM), wherein the SMSM binds to a pre-mRNA encoded by ATXN3 and modulates splicing of the ATXN3 pre-mRNA in a cell of the subject to produce a spliced product of the ATXN3 pre-mRNA.

In some embodiments, described herein is a method of treating, preventing, delaying of progress, or ameliorating symptoms of a disease or a condition associated with Ataxin 3 (ATXN3) expression level or activity level in a subject in need thereof, comprising administering a therapeutically effective amount of a compound or salt of Formula (I). In some embodiments, described herein is a method of modulating splicing of a Ataxin3 (ATXN3) pre-mRNA, comprising contacting a compound or salt of Formula (I) to the ATXN3 pre-mRNA with a splice site sequence or cells comprising the ATXN3 pre-mRNA, wherein the compound binds to the ATXN3 pre-mRNA and modulates splicing of the ATXN3 pre-mRNA in a cell of a subject to produce a spliced product of the ATXN3 pre-mRNA. In some embodiments, described herein is use of a compound of Formula (I), or a pharmaceutically acceptable salt thereof, in the manufacture of a medicament for the treatment of a condition or disease associated with Ataxin 3 (ATXN3) expression level or activity level.

In some embodiments, the spliced product of the ATXN3 pre-mRNA undergoes non-sense mediated decay (NMD) and/or nuclear retention. In some embodiments, the nonsense-mediated decay (NMD) and/or nuclear retention of the spliced product of the ATXN3 pre-mRNA is promoted. In some embodiments, the nonsense-mediated decay (NMD) and/or nuclear retention of the spliced product of the ATXN3 pre-mRNA is increased compared to a spliced product of the ATXN3 pre-mRNA produced in the absence of the SMSM.

In some embodiments, described herein is a method of modulating splicing of a Ataxin3 (ATXN3) pre-mRNA, comprising contacting a small molecule splicing modulator (SMSM) to the ATXN3 pre-mRNA with a splice site sequence or cells comprising the ATXN3 pre-mRNA, wherein the SMSM binds to the ATXN3 pre-mRNA and modulates splicing of the ATXN3 pre-mRNA in a cell of a subject to produce a spliced product of the ATXN3 pre-mRNA.

In some embodiments, described herein, is a method of modulating splicing of Ataxin 3 (ATXN3) pre-mRNA, comprising contacting a small molecule splicing modulator (SMSM) to the ATXN3 pre-mRNA with a splice site sequence or cells comprising the ATXN3 pre-mRNA, wherein the SMSM binds to the ATXN3 pre-mRNA and modulates splicing of the ATXN3 pre-mRNA in a cell of a subject to produce a spliced product of the ATXN3 pre-mRNA, wherein the splice site sequence comprises UCCUAU/guaagauucugu.

In some embodiments, described herein, is a method of treating, preventing, delaying of progress, or ameliorating symptoms of a disease or condition associated with Ataxin 3 (ATXN3) expression level or activity level in a subject in need thereof, comprising administering a therapeutically effective amount of a small molecule splicing modulator (SMSM) to the subject, wherein the SMSM binds to a ATXN3 pre-mRNA with a splice site sequence and modulates splicing of the ATXN3 pre-mRNA in a cell of the subject, wherein a spliced product of the ATXN3 pre-mRNA undergoes nonsense-mediated decay (NMD), and wherein the splice site sequence comprises UCCUAU/guaagauucugu.

In some embodiments, the modulating splicing comprises modulating alternative splicing. In some embodiments, the modulating splicing comprises promoting exon skipping. In some embodiments, the modulating splicing comprises promoting exon inclusion. In some embodiments, the modulating splicing comprises modulating nonsense-mediated mRNA decay (NMD). In some embodiments, the modulating NMD comprises promoting NMD. In some embodiments, the modulating splicing comprises modulating nuclear retention of the spliced product of the pre-mRNA. In some embodiments, the modulating intron retention comprises promoting nuclear retention of the spliced product of the pre-mRNA.

In some embodiments, the splice site sequence is a native splice site sequence. In some embodiments, the native splice site is a canonical splice site. In some embodiments, the native splice site is an alternative splice site. In some embodiments, the alternative splice site comprises a 5' splice site sequence. In some embodiments, the alternative splice site sequence comprises UCCUAU/guaagauucugu. In some embodiments, the SMSM induces splicing at the alternative splice site. In some embodiments, the splicing at the alternative splice site results in a frameshift in a downstream exon in the spliced product. In some embodiments, the downstream exon comprises an in-frame stop codon that is not in frame in the absence of splicing at the alternative splice site. In some embodiments, the in-frame stop codon in the downstream exon is at least 50 or at least 60 base pairs upstream of the 3' end of the downstream exon. In some embodiments, the in-frame stop codon in the downstream exon is at least 50 or at least 60 base pairs upstream of a final exon-exon junction.

In some embodiments, the splicing of the pre-mRNA at the alternative splice site promotes NMD of the spliced product of the ATXN3 pre-mRNA. In some embodiments, the spliced product comprises an alternative exon. In some embodiments, the SMSM promotes inclusion of the alternative exon in the spliced product. In some embodiments, the alternative exon comprises a poison exon. In some embodiments, the SMSM promotes inclusion of the poison exon in the spliced product. In some embodiments, the poison exon comprises an in-frame stop codon. In some embodiments, the in-frame stop codon is a premature termination codon. In some embodiments, the in-frame stop codon is at least 50 or 60 base pairs upstream of the 3' end of the poison exon. In some embodiments, the in-frame stop codon is less than 60 base pairs upstream of the 3' end of the poison exon and wherein the exon immediately downstream of the poison exon is not the last exon in the pre-mRNA. In some embodiments, the sum of (a) the number of base pairs in the exon immediately downstream of the poison exon and (b) the number of base pairs between the premature termination codon in the poison exon and the 3' end of the poison exon is at least 50 or at least 60.

In some embodiments, the cells comprise primary cells. In some embodiments, the cells comprise disease cells. In some embodiments, the SMSM modulates proliferation or survival of the cells. In some embodiments, the SMSM modulates the expression level of a protein encoded by the spliced product of the pre-mRNA in the cells.

**Table 2. Exemplary targets for exon inclusion**

| | |
|---|---|
| Gene | ATXN3 |
| Exon Coordinates | Chr14:92093746-92093831 |
| Splicing Event Region | chr14:92093319-92096092 |
| Strand | - |
| Target site | Exon 4 |
| Exon length | 86 |
| SEQ ID NO: | 1 |
| 5' ss sequence (-6~+12) | UCCUAU/guaagauucugu |
| 5' ss-U1 duplex structure | -1U-C loop |
| Disease | Spinocerebellar Ataxia Type 3 |

### Methods of Treatment

The compositions and methods described herein can be used for treating a human disease or disorder associated with aberrant splicing, such as aberrant pre-mRNA splicing. The compositions and methods described herein can be used for treating a human disease or disorder by modulating mRNA, such as pre-mRNA. In some embodiments, the compositions and methods described herein can be used for treating a human disease or disorder by modulating splicing of a nucleic acid even when that nucleic acid is not aberrantly spliced in the pathogenesis of the disease or disorder being treated.

In some embodiments, an effective amount in the context of the administration of a SMSM or a pharmaceutically acceptable salt thereof, or composition or medicament thereof refers to an amount of a SMSM or a pharmaceutically acceptable salt thereof to a patient which has a therapeutic effect and/or beneficial effect. In certain specific embodiments, an effective amount in the context of the administration of a SMSM or a pharmaceutically acceptable salt thereof, or composition or medicament thereof to a patient results in one, two or more of the following effects: (i) reduces or ameliorates the severity of a disease; (ii) delays onset of a disease; (iii) inhibits the progression of a disease; (iv) reduces hospitalization of a subject; (v) reduces hospitalization length for a subject; (vi) increases the survival of a subject; (vii) improves the quality of life of a subject; (viii) reduces the number of symptoms associated with a disease; (ix) reduces or ameliorates the severity of a symptom associated with a disease; (x) reduces the duration of a symptom associated with a disease associated; (xi) prevents the recurrence of a symptom associated with a disease; (xii) inhibits the development or onset of a symptom of a disease; and/or (xiii) inhibits of the progression of a symptom associated with a disease. In some embodiments, an effective amount of a SMSM or a pharmaceutically acceptable salt thereof is an amount effective to restore the amount of an RNA transcript of a gene to the amount of the RNA transcript detectable in healthy patients or cells from healthy patients. In other embodiments, an effective amount of a SMSM or a pharmaceutically acceptable salt thereof is an amount effective to restore the amount an RNA isoform and/or protein isoform of a gene to the amount of the RNA isoform and/or protein isoform detectable in healthy patients or cells from healthy patients.

In some embodiments, an effective amount of a SMSM or a pharmaceutically acceptable salt thereof is an amount effective to decrease the aberrant amount of an RNA transcript of a gene which associated with a disease. In some embodiments, an effective amount of a SMSM or a pharmaceutically acceptable salt thereof is an amount effective to decrease the amount of the aberrant expression of an isoform of a gene. In some embodiments, an effective amount of a SMSM or a pharmaceutically acceptable salt thereof is an amount effective to result in a substantial change in the amount of an RNA transcript (*e.g*., an mRNA transcript), alternative splice variant, or isoform.

In some embodiments, an effective amount of a SMSM or a pharmaceutically acceptable salt thereof is an amount effective to increase the amount of an RNA transcript (*e.g*., an mRNA transcript) of a gene that is beneficial for the prevention and/or treatment of a disease. In some embodiments, an effective amount of a SMSM or a pharmaceutically acceptable salt thereof is an amount effective to increase the amount of an alternative splice variant of an RNA transcript of a gene that is beneficial for the prevention and/or treatment of a disease. In some embodiments, an effective amount of a SMSM or a pharmaceutically acceptable salt thereof is an amount effective to increase the amount of an isoform of a gene that is beneficial for the prevention and/or treatment of a disease.

In some embodiments, an effective amount of a SMSM or a pharmaceutically acceptable salt thereof is an amount effective to decrease the amount of an RNA transcript (*e.g*., an mRNA transcript) which causes or is related to the symptoms of the condition or disease. In particular embodiments, the SMSM decreases the amount of an RNA transcript that causes or relates to the symptoms of the condition or disease by modulating one or more splicing elements of the RNA transcript. In some embodiments, the SMSM promotes skipping of one or more exons. In some embodiments, the SMSM promotes inclusion of one or more exons. In some embodiments, the SMSM promotes inclusion of one or more exons and/or introns that relate to nonsense-mediated mRNA decay (NMD). In some embodiments, the one or more exons harbor a premature termination codon. In particular embodiments, the premature stop codon is an in-frame codon that does not cause frameshift of the downstream exon(s). In some embodiments, inclusion of the one or more exons causes a reading frameshift in a downstream exon, for example, in the immediately downstream exon, introducing a premature termination codon.

A method of treating a disease or a condition in a subject in need thereof can comprise administering to the subject a therapeutically effective amount of a compound described herein or a pharmaceutically acceptable salt thereof. In some embodiments, the present disclosure relates to a method for the treatment, prevention and/or delay of progression of a disease or a condition associated with a gene listed in Table 2.

Non-limiting examples of effective amounts of a SMSM or a pharmaceutically acceptable salt thereof are described herein. For example, the effective amount may be the amount required to prevent and/or treat a disease associated with the aberrant amount of an mRNA transcript of gene in a human subject. In general, the effective amount will be in a range of from about 0.001 mg/kg/day to about 500 mg/kg/day for a patient having a weight in a range of between about 1 kg to about 200 kg. The typical adult subject is expected to have a median weight in a range of between about 70 and about 100 kg.

In one embodiment, a SMSM described herein can be used in the preparation of medicaments for the treatment of diseases or conditions described herein. In addition, a method for treating any of the diseases or conditions described herein in a subject in need of such treatment, can involve administration of pharmaceutical compositions that include at least one SMSM described herein or a pharmaceutically acceptable salt, thereof, in a therapeutically effective amount to a subject.

In certain embodiments, a SMSM described herein can be administered for prophylactic and/or therapeutic treatments. In certain therapeutic applications, the compositions are administered to a patient already suffering from a disease or a condition, in an amount sufficient to cure or at least partially arrest at least one of the symptoms of the disease or the condition. Amounts effective for this use depend on the severity and course of the disease or the condition, previous therapy, the patient's health status, weight, and response to the drugs, and the judgment of the treating physician. Therapeutically effective amounts are optionally determined by methods including, but not limited to, a dose escalation clinical trial. In prophylactic applications, compositions containing a SMSM described herein can be administered to a patient susceptible to or otherwise at risk of a particular disease, disorder, or condition.

### Methods of Administering

The compositions described herein can be administered to the subject in a variety of ways, including parenterally, intravenously, intradermally, intramuscularly, colonically, rectally or intraperitoneally. In some embodiments, the small molecule splicing modulator (SMSM) or a pharmaceutically acceptable salt thereof is administered by intraperitoneal injection, intramuscular injection, subcutaneous injection, or intravenous injection of the subject. In some embodiments, the pharmaceutical compositions can be administered parenterally, intravenously, intramuscularly or orally. The oral agents comprising a small molecule splicing modulator can be in any suitable form for oral administration, such as liquid, tablets, capsules, or the like. The compositions of the present disclosure can be administered to a subject using any suitable methods known in the art. Suitable formulations for use in the present disclosure and methods of delivery are generally well known in the art. For example, the small molecule splicing modulators described herein can be formulated as pharmaceutical compositions with a pharmaceutically acceptable diluent, carrier, or excipient.

### Dosing and Schedules

The SMSMs utilized in the methods of the disclosure can be, *e.g*., administered at dosages that may be varied depending upon the requirements of the subject, the severity of the condition being treated and/or imaged, and/or the SMSM being employed. For example, dosages can be empirically determined considering the type and stage of disease diagnosed in a particular subject and/or the type of imaging modality being used in conjunction with the SMSMs. The dose administered to a subject, in the context of the present disclosure should be sufficient to affect a beneficial diagnostic or therapeutic response in the subject. The size of the dose also can be determined by the existence, nature, and extent of any adverse side-effects that accompany the administration of a SMSM in a particular subject.

Within the scope of the present description, the effective amount of a SMSM or a pharmaceutically acceptable salt thereof for use in the manufacture of a medicament, the preparation of a pharmaceutical kit or in a method for preventing and/or treating a disease in a human subject in need thereof, is intended to include an amount in a range of from about 1 µg to about 50 grams.

The compositions of the present disclosure can be administered as frequently as necessary.

### Subjects

The subjects that can be treated with the SMSMs and methods described herein can be any subject that produces mRNA that is subject to alternative splicing, *e.g.,* the subject may be a eukaryotic subject, such as a plant or an animal. In some embodiments, the subject is a mammal, *e.g.*, human. In some embodiments, the subject is a human. In some embodiments, the subject is a non-human animal. In some embodiments, the subject is a fetus, an embryo, or a child. In some embodiments, the subject is a non-human primate such as chimpanzee, and other apes and monkey species; farm animals such as cattle, horses, sheep, goats, swine; domestic animals such as rabbits, dogs, and cats; laboratory animals including rodents, such as rats, mice and guinea pigs, and the like.

In some embodiments, the subject is prenatal (*e.g.,* a fetus), a child (*e.g.,* a neonate, an infant, a toddler, a preadolescent), an adolescent, a pubescent, or an adult (*e.g.,* an early adult, a middle-aged adult, a senior citizen).

### Methods of Making Compounds

Compounds described herein can be synthesized using standard synthetic techniques or using methods known in the art in combination with methods described herein. Unless otherwise indicated, conventional methods of mass spectroscopy, NMR, HPLC, protein chemistry, biochemistry, recombinant DNA techniques and pharmacology can be employed. Compounds can be prepared using standard organic chemistry techniques such as those described in, for example, March's Advanced Organic Chemistry, 6th Edition, John Wiley and Sons, Inc. Alternative reaction conditions for the synthetic transformations described herein may be employed such as variation of solvent, reaction temperature, reaction time, as well as different chemical reagents and other reaction conditions. The starting materials can be available from commercial sources or can be readily prepared. By way of example only, provided are schemes for preparing the SMSMs described herein.

Suitable reference books and treatise that detail the synthesis of reactants useful in the preparation of compounds described herein, or provide references to articles that describe the preparation, include for example, "Synthetic Organic Chemistry", John Wiley & Sons, Inc., New York; S. R. Sandler et al., "Organic Functional Group Preparations," 2nd Ed., Academic Press, New York, 1983; H. O. House, "Modern Synthetic Reactions", 2nd Ed., W. A. Benjamin, Inc. Menlo Park, Calif. 1972; T. L. Gilchrist, "Heterocyclic Chemistry", 2nd Ed., John Wiley & Sons, New York, 1992; J. March, "Advanced Organic Chemistry: Reactions, Mechanisms and Structure", 4th Ed., Wiley Interscience, New York, 1992. Additional suitable reference books and treatise that detail the synthesis of reactants useful in the preparation of compounds described herein, or provide references to articles that describe the preparation, include for example, Fuhrhop, J. and Penzlin G. "Organic Synthesis: Concepts, Methods, Starting Materials", Second, Revised and Enlarged Edition (1994) John Wiley & Sons ISBN: 3 527-29074-5; Hoffman, R.V. "Organic Chemistry, An Intermediate Text" (1996) Oxford University Press, ISBN 0-19-509618-5; Larock, R. C. "Comprehensive Organic Transformations: A Guide to Functional Group Preparations" 2nd Edition (1999) Wiley-VCH, ISBN: 0-471-19031-4; March, J. "Advanced Organic Chemistry: Reactions, Mechanisms, and Structure" 4th Edition (1992) John Wiley & Sons, ISBN: 0-471-60180-2; Otera, J. (editor) "Modern Carbonyl Chemistry" (2000) Wiley-VCH, ISBN: 3-527-29871-1; Patai, S. "Patai's 1992 Guide to the Chemistry of Functional Groups" (1992) Interscience ISBN: 0-471-93022-9; Solomons, T. W. G. "Organic Chemistry" 7th Edition (2000) John Wiley & Sons, ISBN: 0-471-19095-0; Stowell, J.C., "Intermediate Organic Chemistry" 2nd Edition (1993) Wiley-Interscience, ISBN: 0-471-57456-2; "Industrial Organic Chemicals: Starting Materials and Intermediates: An Ullmann's Encyclopedia" (1999) John Wiley & Sons, ISBN: 3-527-29645-X, in 8 volumes; "Organic Reactions" (1942-2000) John Wiley & Sons, in over 55 volumes; and "Chemistry of Functional Groups" John Wiley & Sons, in 73 volumes.

In the reactions described, it may be necessary to protect reactive functional groups, for example hydroxy, amino, imino, thio or carboxy groups, where these are desired in the final product, in order to avoid their unwanted participation in reactions. A detailed description of techniques applicable to the creation of protecting groups and their removal are described in Greene and Wuts, Protective Groups in Organic Synthesis, 3rd Ed., John Wiley & Sons, New York, NY, 1999, and Kocienski, Protective Groups, Thieme Verlag, New York, NY, 1994.

SMSMs can be made using known techniques and further chemically modified, in some embodiments, to facilitate intranuclear transfer to, *e.g.,* a splicing complex component, a spliceosome or a pre-mRNA molecule. One of ordinary skill in the art will appreciate the standard medicinal chemistry approaches for chemical modifications for intranuclear transfer (*e.g*., reducing charge, optimizing size, and/or modifying lipophilicity).

### General Synthesis Scheme 1 (R²⁴ being Hydrogen or alkyls).

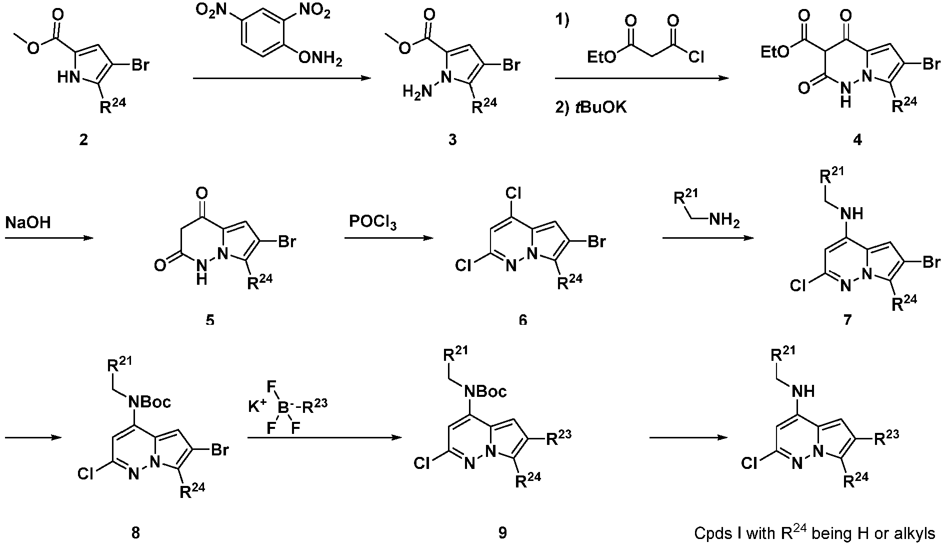

### General Synthesis Scheme 2:

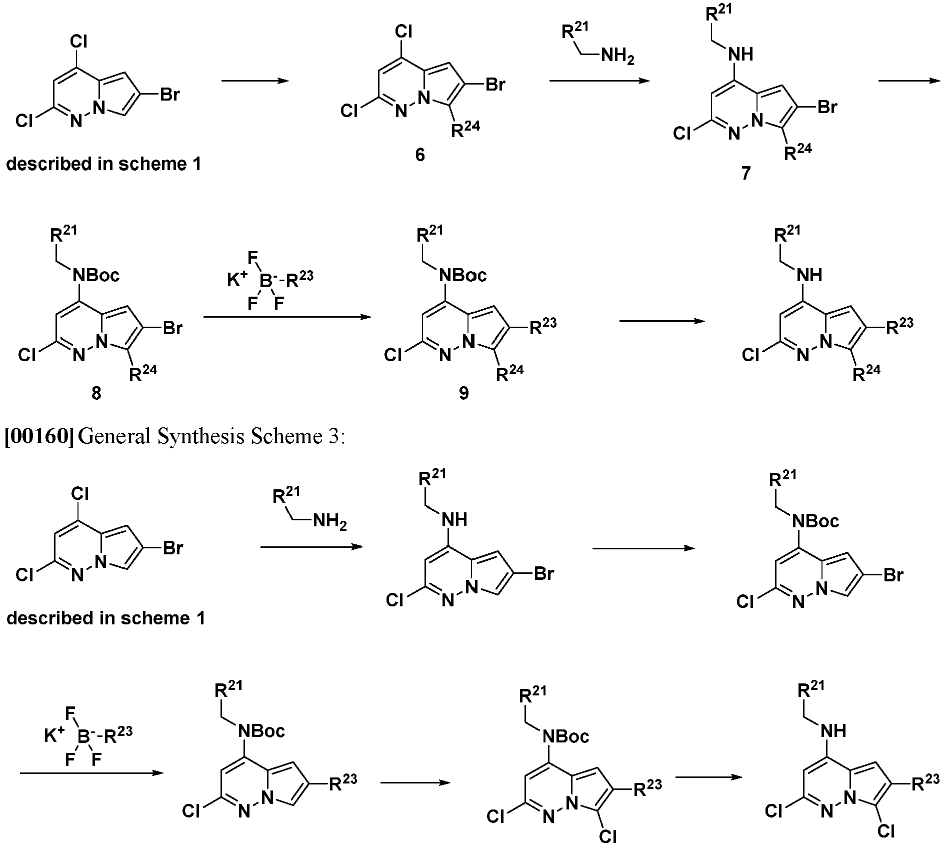

### EXAMPLES

These examples are provided for illustrative purposes only and not to limit the scope of the claims provided herein. The starting materials and reagents used for the synthesis of the compounds described herein may be synthesized or can be obtained from commercial sources, such as, but not limited to, Sigma-Aldrich, Acros Organics, Fluka, and Fisher Scientific.

### Example A1. Synthesis of Intermediates of type 8

### Intermediate 8-1: tert-butyl (6-bromo-2-chloro-7-methylpyrrolo[1,2-b]pyridazin-4-yl)(furan-2-ylmethyl)carbamate

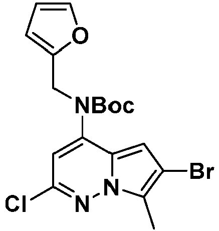

**Step 1:** Into a 500-mL round bottom flask purged and maintained with an inert atmosphere of nitrogen were added ethyl 5-methyl-1H-pyrrole-2-carboxylate (20 g, 130.5 mmol, 1 eq.), NBS (23.2 g, 130.5 mmol, 1 eq.) and DCM (200 mL) at 0°C. The resulting solution was stirred for 2h at RT. The resulting mixture was extracted with DCM (3 x 300 mL). The combined organic layers were washed with water (3x300 mL), dried over anhydrous Na₂SO₄. After filtration, the filtrate was concentrated under reduced pressure. The residue was purified by trituration with MeOH (100 mL) to afford ethyl 4-bromo-5-methyl-1H-pyrrole-2-carboxylate (20 g, 66%) as a white solid.

**Step 2:** A solution of ethyl 4-bromo-5-methyl-1H-pyrrole-2-carboxylate (20 g, 86.2 mmol, 1 eq.) in THF (200 ml) was treated with sodium hydride (4.14 g, 172.4 mmol, 2 eq.) for 2 h at 0°C under nitrogen atmosphere followed by the addition of O-(2,4-dinitrophenyl)hydroxylamine (25.74 g, 129.3 mmol, 1.5 eq.) in portions at RT. The resulting mixture was stirred for 2h at RT. The aqueous layer was extracted with EtOAc (2x100 mL). The resulting mixture was concentrated under reduced pressure. The residue was purified by silica gel column chromatography, eluted with PE / EA (5: 1) to afford ethyl 1-amino-4-bromo-5-methylpyrrole-2-carboxylate (13.5 g, 63%) as a yellow semi-solid.

**Step 3:** A solution of ethyl 1-amino-4-bromo-5-methylpyrrole-2-carboxylate (13.5 g, 54.65 mmol, 1 eq.) and ethyl 3-chloro-3-oxopropanoate (9.87 g, 65.5 mmol, 1.2 eq.), pyridine (8.64 g, 109.3 mmol, 2 eq.) in DCM (200 ml) was stirred for 2h at RT. The aqueous layer was extracted with DCM (2x100 mL). The resulting mixture was concentrated under reduced pressure. The residue was purified by silica gel column chromatography, eluted with PE / EA (5: 1) to afford ethyl 4-bromo-1-(3-ethoxy-3-oxopropanamido)-5-methylpyrrole-2-carboxylate (7.5 g, 38%) as a light yellow semi-solid.

**Step 4:** A solution of ethyl 4-bromo-1-(3-ethoxy-3-oxopropanamido)-5-methylpyrrole-2-carboxylate (7.5 g, 20.7 mmol, 1 equiv) and t-BuOK (4.66 g, 41.5 mmol, 2 equiv) in THF (100mL) was stirred for 4h at room temperature. The resulting mixture was concentrated under vacuum. The resulting mixture containing ethyl 6-bromo-7-methyl-2,4-dioxo-1H,3H-pyrrolo[1,2-b]pyridazine-3-carboxylate was used in the next step directly without further purification.

**Step 5:** A solution of ethyl 6-bromo-7-methyl-2,4-dioxo-1H,3H-pyrrolo[1,2-b]pyridazine-3-carboxylate (5 g, 15.8 mmol, 1 eq.) and NaOH (3.173 g, 79 mmol, 5 eq.) in 1,4-dioxane : water (1:1) (100mL) was stirred for 16h at 80°C. The mixture was acidified to pH 7 with conc. HCl. The precipitated solids were collected by filtration. The residue was purified by reversed-phase flash chromatography (column, C18; mobile phase, MeCN in water (0.1% FA), 10% to 50% gradient in 10 min; detector, UV 254 nm) to yield 6-bromo-7-methyl-1H,3H-pyrrolo[1,2-b]pyridazine-2,4-dione (2.8 g, 73%) as a brown solid.

**Step 6:** A solution of 6-bromo-7-methyl-1H,3H-pyrrolo[1,2-b]pyridazine-2,4-dione (2.8 g, 11.5 mmol, 1 eq.) and DIEA (2.9g, 23.0 mmol, 2 eq.) in POCl₃ (30mL) was stirred for 3 days at 125°C. The mixture was acidified to pH 7 with conc. HCl. The precipitated solids were collected by filtration. The residue was purified by reversed-phase flash chromatography (column, C18; mobile phase, MeCN in Water (0.1% FA), 10% to 50% gradient in 10 min; detector, UV 254 nm) to yield 6-bromo-2,4-dichloro-7-methylpyrrolo[1,2-b]pyridazine (200 mg, 6.2%) as a light yellow solid.

**Step 7:** A solution of 6-bromo-2,4-dichloro-7-methylpyrrolo[1,2-b]pyridazine (200 mg, 0.714 mmol, 1 eq.) and furan-2-ylmethanamine (90 mg, 0.928 mmol, 1.3 eq.), DIEA (185 mg, 1.428 mmol, 2 eq.) in DMSO (3mL) was stirred for 4h at 100°C. The residue was purified by reversed-phase flash chromatography (column, C18; mobile phase, MeCN in water (0.1% FA), 30% to 100% gradient in 18 min; detector, UV 254 nm) to yield 6-bromo-2-chloro-N-(furan-2-ylmethyl)-7-methylpyrrolo[1,2-b]pyridazin-4-amine (140 mg, 57%) as a yellow solid.

**Step 8:** A solution of 6-bromo-2-chloro-N-(furan-2-ylmethyl)-7-methylpyrrolo[1,2-b]pyridazin-4-amine (97 mg, 0.285 mmol, 1 eq.) and Boc₂O (93 mg, 0.427 mmol, 1.5 eq.), DMAP (3.5 mg, 0.0.028 mmol, 0.1 eq.), TEA (57 mg, 0.570 mmol, 2 eq.) in DCM (2ml) was stirred for 4h at RT. The resulting mixture was diluted with water (20mL). The resulting mixture was extracted with CH₂Cl₂ (2 x 20mL). The combined organic layers were washed with NaCl (1x10 mL), dried over anhydrous Na₂SO₄. After filtration, the filtrate was concentrated under reduced pressure. The residue was purified by silica gel column chromatography, eluted with PE / EA (10: 1) to yield *tert-butyl (6-*bromo-2-chloro-7-methylpyrrolo[1,2-b]pyridazin-4-yl)(furan-2-ylmethyl)carbamate (95 mg, 76%) as a yellow solid.

### Intermediate 8-2: tert-butyl (6-bromo-2,7-dichloropyrrolo[1,2-b]pyridazin-4-yl)(furan-2-ylmethyl)carbamate

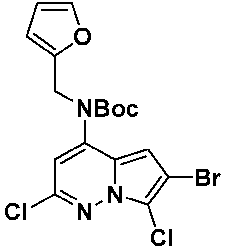

**Step 1:** To a stirred mixture of 6-bromo-2,4-dichloropyrrolo[1,2-b]pyridazine (compound prepared in analogy to the descriptions in intermediate **8-1)** (200 mg, 0.752 mmol, 1 eq.) in TFA (5 mL) was added NCS (120 mg, 0.902 mmol, 1.2 eq.) in portions at 0°C under nitrogen atmosphere. The resulting mixture was stirred for 10min at 0°C under nitrogen atmosphere. The resulting mixture was diluted with CH₂Cl₂ (10 mL). The reaction was quenched by the addition of water (20mL) at 0°C. The resulting mixture was extracted with CH₂Cl₂ (3 x 20mL). The combined organic layers were washed with brine (1x10 mL), dried over anhydrous Na₂SO₄. After filtration, the filtrate was concentrated under reduced pressure. The residue was purified by reversed-phase flash chromatography (column, C18; mobile phase, MeCN in water (10mmol/L NH₄HCO₃), 20% to 100% gradient in 25 min; detector, UV 254 nm) to yield 6-bromo-2,4,7-trichloropyrrolo[1,2-b]pyridazine (170 mg, 75%) as a yellow solid.

**Step 2:** To a stirred solution of 6-bromo-2,4,7-trichloropyrrolo[1,2-b]pyridazine (500 mg, 1.665 mmol, 1 eq.) and furan-2-ylmethanamine (243 mg, 2.498 mmol, 1.5 eq.) in DMSO (5 mL) was added DIEA (430 mg, 3.33 mmol, 2 eq.) dropwise at RT under air atmosphere. The resulting mixture was stirred for 2h at 100°C under air atmosphere. The mixture was allowed to cool down to RT. The residue was purified by reversed-phase flash chromatography (column, C18; mobile phase, MeCN in water (10mmol/L NH₄HCO₃), 50% to 100% gradient in 20 min; detector, UV 254 nm) to yield 6-bromo-2,7-dichloro-N-(furan-2-ylmethyl)pyrrolo[1,2-b]pyridazin-4-amine (500 mg, 83%) as a light yellow solid.

**Step 3:** To a stirred solution of 6-bromo-2,7-dichloro-N-(furan-2-ylmethyl)pyrrolo[1,2-b]pyridazin-4-amine (500 mg, 1.385 mmol, 1 eq.) in DCM (10 mL) was added DMAP (169 mg, 1.385 mmol, 1 eq.) and di-tert-butyl dicarbonate (453 mg, 2.078 mmol, 1.5 eq.) in portions at RT under air atmosphere. The resulting mixture was stirred for 2h at RT under air atmosphere. The resulting mixture was concentrated under reduced pressure. The residue was purified by silica gel column chromatography, eluted with PE / EA (10: 1) to afford *tert-butyl* (6-bromo-2,7-dichloropyrrolo[1,2-b]pyridazin-4-yl)(furan-2-ylmethyl)carbamate (600 mg, 94%) as a yellow solid.

### Example A2. Synthesis of potassium trifluoroborate salts

### Borate salt 1: Potassium ((2R,3S)-2-((tert-butoxycarbonyl)amino)-3-fluorobutyl)trifluoroborate

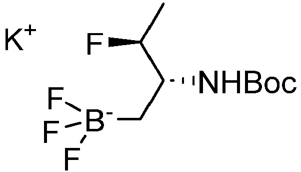

Step 1: To a stirred solution of imidazole (144.76 g, 2126.348 mmol, 4 equiv) in DCM (3720 mL) were added SOCl₂ (57.84 mL, 797.38 mmol, 1.5 equiv) and DIEA (185.19 mL, 1063.17 mmol, 2 eq.) dropwise at 0°C. The resulting mixture was stirred for 0.5h at 0oC. To the above mixture was added methyl (2S,3R)-2-[(tert-butoxycarbonyl)amino]-3-hydroxybutanoate (124 g, 531.587 mmol, 1 eq.) in DCM (620ml) dropwise at 0°C. The resulting mixture was stirred for additional overnight at RT. The resulting mixture was washed with 3 x 800 mL of HCl (0.5M). The resulting mixture was concentrated under reduced pressure. The crude product was used in the next step directly without further purification.

**Step 2:** A solution of 3-(tert-butyl) 4-methyl (4S,5R)-5-methyl-1,2,3-oxathiazolidine-3,4-dicarboxylate 2-oxide (144 g, 515.55 mmol, 1 eq.) in H₂O (1008 mL) and acetonitrile (1872 mL) was treated with NaIO₄ (132.33 g, 618.66 mmol, 1.2 eq.) and ruthenium(iv) oxide hydrate (1.56 g, 10.31 mmol, 0.02 eq.) at 0°C under nitrogen atmosphere. The resulting mixture was stirred for 1h at 0°C under nitrogen atmosphere. The resulting mixture was filtered, the filter cake was washed with EtOAc (5 x 1000 mL). The filtrate was concentrated under reduced pressure. The residue was purified by silica gel column chromatography, eluted with PE / EA (1: 1) to provide 3-(tert-butyl) 4-methyl (4S,5R)-5-methyl-1,2,3-oxathiazolidine-3,4-dicarboxylate 2,2-dioxide (145 g, 95%) as a light yellow oil.

**Step 3:** A solution of 3-(tert-butyl) 4-methyl (4S,5R)-5-methyl-1,2,3-oxathiazolidine-3,4-dicarboxylate 2,2-dioxide (144 g, 487.62 mmol, 1 eq.) in THF (975 mL) was treated with Et₃N.₃HF (430.11 mL, 3169.55 mmol, 6.5 eq.) at 60°C under nitrogen atmosphere. The resulting mixture was stirred for 3 days at 60°C under nitrogen atmosphere. The mixture was neutralized to pH 7 with NaOH (20%). The aqueous layer was extracted with EtOAc (3 x 800 mL). The resulting mixture was concentrated under reduced pressure. The residue was purified by silica gel column chromatography, eluted with PE / EA (1: 1) to afford methyl (2R,3S)-2-((tert-butoxycarbonyl)amino)-3-fluorobutanoate (55 g, 48%) as a light yellow oil.

**Step 4:** A solution of methyl (2R,3S)-2-((tert-butoxycarbonyl)amino)-3-fluorobutanoate (55 g, 233.79 mmol, 1 eq.) in EtOH (500 mL) was treated with NaBH₄ (22.11 g, 584.47 mmol, 2.5 eq.) at 0°C under nitrogen atmosphere. The resulting mixture was stirred overnight at 0°C under nitrogen atmosphere. The reaction was quenched by the addition of Water/Ice (500mL) at 0°C. The aqueous layer was extracted with EtOAc (3x400mL). The resulting mixture was concentrated under reduced pressure. The residue was purified by silica gel column chromatography, eluted with PE / EA (1: 1) to afford tert-butyl N-[(2R,3S)-3-fluoro- 1-hydroxybutan-2-yl]carbamate (46 g, 95%) as a yellow solid.

**Step 5:** To a stirred mixture of tert-butyl N-[(2R,3S)-3-fluoro-1-hydroxybutan-2-yl]carbamate (44 g, 212.31 mmol, 1 eq.) and triphenylphosphine (103.02 g, 392.77 mmol, 1.85 eq.) in THF (170 mL) and DCM (880 mL) was added NBS (69.91 g, 392.77 mmol, 1.85 eq.) in portions at - 20°C under air atmosphere. The resulting mixture was stirred overnight at RT under air atmosphere. The resulting mixture was washed with 1x500 mL of water, washed with brine (1x200 mL) and dried over anhydrous Na₂SO₄. After filtration, the filtrate was concentrated under reduced pressure. The residue was purified by silica gel column chromatography, eluted with PE / EA (10: 1) to afford tert-butyl N-[(2S,3S)-1-bromo-3-fluorobutan-2-yl]carbamate (30 g, 52%) as a light yellow solid.

**Step 6:** A mixture of tert-butyl N-[(2S,3S)-1-bromo-3-fluorobutan-2-yl]carbamate (15.2 g, 56.27 mmol, 1 eq.), copper(I) iodide (1.07 g, 5.63 mmol, 0.1 eq.), triphenylphosphine (1.92 g, 7.31 mmol, 0.13 eq.), 4,4,5,5-tetramethyl-2-(4,4,5,5-tetramethyl-1,3,2-dioxaborolan-2-yl)-1,3,2-dioxaborolane (18.57 g, 73.15 mmol, 1.3 eq.) and methoxylithium (4.27 g, 112.53 mmol, 2 eq.) in DMF (150 mL) was stirred for 16h at RT under air atmosphere. The resulting mixture was diluted with water (500mL). The resulting mixture was filtered, the filter cake was washed with MTBE (1x100 mL). The resulting mixture was extracted with MTBE (3 x300 mL). The combined organic layers were washed with brine (2x100 mL), dried over anhydrous Na₂SO₄. After filtration, the filtrate was concentrated under reduced pressure. The resulting mixture was used in the next step directly without further purification.

**Step 7:** A mixture of tert-butyl N-[(2R,3S)-3-fluoro-1-(4,4,5,5-tetramethyl-1,3,2-dioxaborolan-2-yl)butan-2-yl] carbamate (15.2 g, 47.92 mmol, 1 eq.) and KHF₂ (8.8 g, 112.67 mmol, 2.35 eq.) in MTBE (600 mL) and H₂O (4 mL) was stirred for 16h at RT under air atmosphere. The resulting mixture was concentrated under vacuum. The residue was purified by trituration with MTBE. The mixture was stirred for 30 min and filtered. The filter cake was diluted with acetone (800 mL) and stirred for another 30 min. The resulting mixture was filtered, and the filter cake was washed with acetone (1x50 mL). The filtrate was concentrated under reduced pressure. This resulted in potassium ((2R,3S)-2-((*tert*-butoxycarbonyl)amino)-3-fluorobutyl)trifluoroborate (8 g, 56%) as a white solid.

### Borate salt 2: Potassium (R)-(2-((tert-butoxycarbonyl)amino)-3-fluoropropyl)trifluoroborate

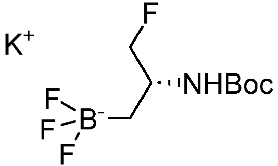

**Step 1:** To a stirred solution of Imidazole (96.79 g, 1421.71 mmol, 4 eq.) in DCM (3000 mL) were added SOCl₂ (38.67 mL, 533.14 mmol, 1.5 eq.) and DIEA (123.82 mL, 710.85 mmol, 2 eq.) dropwise at 0°C. The resulting mixture was stirred for 0.5 h at 0°C. To the mixture was added tert-butyl N-[(2S)-1-(benzyloxy)-3-hydroxypropan-2-yl]carbamate (100 g, 355.43 mmol, 1 eq.) in DCM (500 mL) dropwise at 0°C. The resulting mixture was stirred overnight at RT. The resulting mixture was washed with 3x1000 mL of HCl (0.5 mol/L) and concentrated under reduced pressure. The crude product was used in the next step directly without further purification.

**Step 2:** A solution oftert-butyl (4R)-4-[(benzyloxy)methyl]-2-oxo-1,2lambda4,3-oxathiazolidine-3-carboxylate (122 g, 372.63 mmol, 1 eq.) in H₂O (854 mL) and acetonitrile (1586 mL) was treated with NaIO₄ (95.64 g, 447.16 mmol, 1.2 eq.) and ruthenium(iv) oxide hydrate (1.13 g, 7.45 mmol, 0.02 eq.) at 0°C under nitrogen atmosphere. The resulting mixture was stirred for 1h at 0 °C. The resulting mixture was filtered, the filter cake was washed with EtOAc (4 x 1000 mL). The filtrate was concentrated under reduced pressure. The residue was purified by silica gel column chromatography, eluted with PE / EA (2: 1) to afford tert-butyl (4R)-4-[(benzyloxy)methyl]-2,2-dioxo-1,21ambda6,3-oxathiazolidine-3-carboxylate (117 g, 91%) as a light yellow solid.

**Step 3:** A solution oftert-butyl (4R)-4-[(benzyloxy)methyl]-2,2-dioxo-1,2lambda6,3-oxathiazolidine-3-carboxylate (117 g, 340.72 mmol, 1 eq.) in THF (1725 mL) was treated with TBAF (340.72 mL, 340.720 mmol, 1 equiv) at 0°C under nitrogen atmosphere. The resulting mixture was stirred overnight at room temperature. The reaction was quenched by the addition of citric acid (10%) (1500 mL) at RT. The aqueous layer was extracted with EtOAc (3 x 1000 mL). The combined organic layers were concentrated under reduced pressure. The residue was purified by silica gel column chromatography, eluted with PE / EA (3: 1) to afford tert-butyl N-[(2R)-1-(benzyloxy)-3-fluoropropan-2-yl]carbamate (76 g, 79%) as a colorless oil.

**Step 4:** A solution oftert-butyl N-[(2R)-1-(benzyloxy)-3-fluoropropan-2-yl]carbamate (76 g, 268.226 mmol, 1 equiv) in EtOAc (760 mL) was treated with Pd/C (15.2 g, 20%) at room temperature. The resulting mixture was stirred overnight at 50°C under hydrogen atmosphere. The resulting mixture was filtered, the filter cake was washed with EtOAc (300 mL). The filtrate was concentrated under reduced pressure. The residue was purified by silica gel column chromatography, eluted with PE / EA (1:2) to afford tert-butyl N-[(2R)-1-fluoro-3-hydroxypropan-2-yl]carbamate (49.8 g, 96%) as a colorless oil.

**Step 5:** To a stirred mixture of tert-butyl N-[(2R)-1-fluoro-3-hydroxypropan-2-yl]carbamate (10.5 g, 54.34 mmol, 1 eq.) and triphenylphosphine (17.15 g, 65.21 mmol, 1.2 eq.) in DCM (100 mL) and THF (100 mL) was added NBS (11.62 g, 65.28 mmol, 1.2 eq.) in portions at 0°C under nitrogen atmosphere. The resulting mixture was stirred for 16 h at room temperature under nitrogen atmosphere. The residue was washed with water (2x50 mL) and brine (50 mL). The organic layers were concentrated under reduced pressure. The residue was purified by silica gel column chromatography, eluted with PE / EA (4: 1) to afford tert-butyl N-[(2S)-1-bromo-3-fluoropropan-2-yl]carbamate (3.5 g, 25%) as a yellow solid.

**Step 6:** A mixture of tert-butyl N-[(2S)-1-bromo-3-fluoropropan-2-yl]carbamate (3.5 g, 13.66 mmol, 1 eq.), copper(I) iodide (0.26 g, 1.37 mmol, 0.1 eq.), triphenylphosphine (0.36 g, 1.37 mmol, 0.1 eq.), Bis(pinacolato) diboron (6.94 g, 27.33 mmol, 2 eq.) and methoxylithium (1.04 g, 27.33 mmol, 2 eq.) in DMF (60 mL) was stirred for 16 h at RT under air atmosphere. The resulting mixture was diluted with water (60 mL) and extracted with MTBE (3 x 100 mL). The combined organic layers were washed with brine (1x50 mL), dried over anhydrous Na₂SO₄. After filtration, the filtrate was concentrated under reduced pressure. The crude product was used in the next step directly without further purification.

**Step 7:** A mixture of tert-butyl N-[(2R)-1-fluoro-3-(4,4,5,5-tetramethyl-1,3,2-dioxaborolan-2-yl)propan-2-yl]carbamate (5 g, 16.49 mmol, 1 eq.) and fluorine potassium hydride (2.57 g, 32.98 mmol, 2 eq.) in MTBE (200 mL) and H₂O (2 mL) was stirred for 16 h at room temperature under air atmosphere. The resulting mixture was concentrated under vacuum. The residue was diluted with MTBE (200 mL). The mixture was stirred for 30 min and filtered. The filter cake was diluted with acetone (300 mL) and stirred for another 30 min. The resulting mixture was filtered, and the filter cake was washed with acetone (1x50 mL). The filtrate was concentrated under reduced pressure to afford potassium (*R*)-(2-((*tert*-butoxycarbonyl)amino)-3-fluoropropyl)trifluoroborate (3 g, 64%) as an off-white solid. The crude product was used in the next step directly without further purification.

### General procedures

Cross coupling reaction between an intermediate of type 4 with a trifluoroborate salt

A mixture of an intermediate **8** (1 eq.), a potassium trifluoroborate salt (1.5 eq.), Pd₂(dba)₃ or Pd(Amphos)₂Cl₂ (0.1 eq.) and Cs₂CO₃ (2 eq.) in Toluene / H₂O (25/1) was stirred at 100 °C under a nitrogen atmosphere until completion of the reaction. The resulting mixture was concentrated under reduced pressure. The residue was purified by silica gel column chromatography, to afford the desired compound.

### Example 1. Synthesis of 6-((2R,3S)-2-amino-3-fluorobutyl)-2-chloro-N-(furan-2-ylmethyl)-7-methylpyrrolo[1,2-b]pyridazin-4-amine (Compound 1)

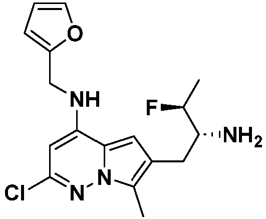

**Step 1:** Using the general cross coupling reaction between the *tert-butyl* (6-bromo-2-chloro-7-methylpyrrolo[1,2-b]pyridazin-4-yl)(furan-2-ylmethyl)carbamate (intermediate **8-1)** and potassium ((2*R*,3*S*)-2-((*tert*-butoxycarbonyl)amino)-3-fluorobutyl)trifluoroborate (borate salt **1)** was obtained 20 mg of *tert-butyl* (6-((2*R*,3*S*)-2-((*tert*-butoxycarbonyl)amino)-3-fluorobutyl)-2-chloro-7-methylpyrrolo[1,2-b]pyridazin-4-yl)(furan-2-ylmethyl)carbamate as a yellow solid.

**Step 2:** A solution of *tert-butyl* (6-((2*R*,3*S*)-2-((*tert*-butoxycarbonyl)amino)-3-fluorobutyl)-2-chloro-7-methylpyrrolo[1,2-b]pyridazin-4-yl)(furan-2-ylmethyl)carbamate (50 mg, 0.091 mmol, 1 eq.) and TFA (0.5 mL) in DCM (2mL) was stirred for 1h at RT. The residue was purified by reversed-phase flash chromatography (column, C18; mobile phase, MeCN in water (0.1% FA), 10% to 35% gradient in 10 min; detector, UV 254 nm) to yield 6-((2*R*,3*S*)-2-amino-3-fluorobutyl)-2-chloro-N-(furan-2-ylmethyl)-7-methylpyrrolo[1,2-b]pyridazin-4-amine (5 mg, 16%). LC-MS-(ES, *m*/*z):* [M+H]⁺= 351.10.

### Example 2. Synthesis of 6-((2R,3S)-2-amino-3-fluorobutyl)-2,7-dichloro-N-(furan-2-ylmethyl)pyrrolo[1,2-b]pyridazin-4-amine (Compound 2)

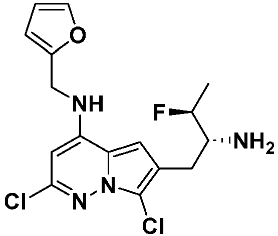

In analogy to example 1, but using in the first step *tert-butyl* (6-bromo-2,7-dichloropyrrolo[1,2-b]pyridazin-4-yl)(furan-2-ylmethyl)carbamate (intermediate 8-2), was obtained 12 mg of 6-((2*R*,3*S*)-2-amino-3-fluorobutyl)-2,7-dichloro-N-(furan-2-ylmethyl)pyrrolo[1,2-b]pyridazin-4-amine. LC-MS-(ES, *m*/*z):* [M+H]⁺= 371.05.

### Example 3. Synthesis of (R)-6-(2-amino-3-fluoropropyl)-2,7-dichloro-N-(furan-2-ylmethyl)pyrrolo[1,2-b]pyridazin-4-amine (Compound 3)

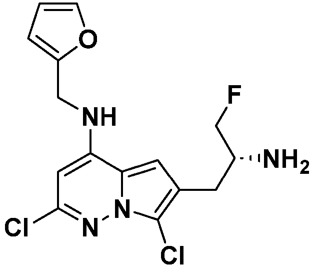

In analogy to example 1, but using in the first step *tert-butyl* (6-bromo-2,7-dichloropyrrolo[1,2-b]pyridazin-4-yl)(furan-2-ylmethyl)carbamate (intermediate **8-2)** and potassium (*R*)-(2-((*tert*-butoxycarbonyl)amino)-3-fluoropropyl)trifluoroborate (borate salt **2)** was obtained 35 mg of (R)-6-(2-amino-3-fluoropropyl)-2,7-dichloro-N-(furan-2-ylmethyl)pyrrolo[1,2-b]pyridazin-4-amine. LC-MS-(ES, *m*/*z):* [M+H]⁺= 357.10.

### Example 4. Synthesis of 6-((2R,3S)-2-amino-3-fluorobutyl)-2,7-dichloro-N-(thiazol-2-ylmethyl)pyrrolo[1,2-b]pyridazin-4-amine (Compound 4)

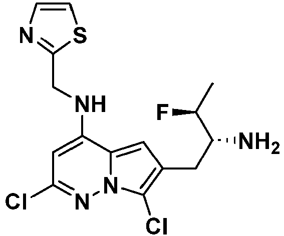

**Step 1:** To a stirred solution of methyl 4-bromo-1H-pyrrole-2-carboxylate (25 g, 122.53 mmol, 1 eq.) in DMF (500 mL) was added NaH (4.70 g, 196.05 mmol, 1.6 eq.) in portions at 0°C under nitrogen atmosphere. The resulting mixture was stirred for 1h at 0°C. To the above mixture was added O-(2,4-dinitrophenyl)hydroxylamine (36.60 g, 183.80 mmol, 1.5 eq.) in DMF (100 mL) dropwise over 1h at 0°C. The resulting mixture was stirred for 1h at RT. The reaction was quenched by the addition of water/Ice (500mL) at RT. The resulting mixture was extracted with EtOAc (4 x 500mL). The combined organic layers were washed with brine (1x100 mL), dried over anhydrous Na₂SO₄. After filtration, the filtrate was concentrated under reduced pressure. The residue was purified by silica gel column chromatography, eluted with PE / EA (4:1) to afford methyl 1-amino-4-bromopyrrole-2-carboxylate (25 g, 93%) as a yellow solid.

Step 2: To a stirred solution of methyl 1-amino-4-bromopyrrole-2-carboxylate (25 g, 114.13 mmol, 1 eq.) in DCM (300 mL) was added DMAP (1.39 g, 11.41 mmol, 0.1 eq.) and Et₃N (12.70 g, 125.55 mmol, 1.1 eq.) dropwise at 0°C under nitrogen atmosphere. To the above mixture was added ethyl 3-chloro-3-oxopropanoate (17.18 g, 114.13 mmol, 1 eq.) in DCM (50 mL) dropwise over 30min at 0°C. The resulting mixture was stirred for an additional 3h at RT. The reaction was quenched by the addition of water/Ice (100 mL) at RT. The resulting mixture was extracted with EtOAc (3 x 500mL). The combined organic layers were washed with brine (1x100 mL), dried over anhydrous Na₂SO₄. After filtration, the filtrate was concentrated under reduced pressure. The residue was purified by silica gel column chromatography, eluted with PE / EA (2:1) to afford methyl 4-bromo-1-(3-ethoxy-3-oxopropanamido)pyrrole-2-carboxylate (27 g, 71%) as a yellow solid.

**Step 3:** To a stirred solution of methyl 4-bromo-1-(3-ethoxy-3-oxopropanamido)pyrrole-2-carboxylate (20 g, 60.03 mmol, 1 eq.) in THF (200 mL) was added t-BuOK in THF (120 mL, 1 mol /L, 2 eq.) dropwise at 0°C under nitrogen atmosphere. The resulting mixture was stirred for 1h at RT under nitrogen atmosphere. The mixture was acidified to pH 7 with HCl (aq.). The resulting mixture was filtered, the filter cake was washed with water (1x100 mL) and dried under vacuo. The crude product mixture was used in the next step directly without further purification. This resulted in ethyl 6-bromo-2,4-dioxo-1H,3H-pyrrolo[1,2-b]pyridazine-3-carboxylate (17 g, 94%) as a white solid.

**Step 4:** To a stirred solution of ethyl 6-bromo-2,4-dioxo-1H,3H-pyrrolo[1,2-b]pyridazine-3-carboxylate (17 g, 56.46 mmol, 1 equiv) in H₂O (600 mL) was added NaOH (4.52 g, 112.92 mmol, 2 eq.) in H₂O (20 mL) dropwise at 0°C under nitrogen atmosphere. The resulting mixture was stirred for 1 day at 110 °C under nitrogen atmosphere. The mixture was allowed to cool down to RT. The mixture was acidified to pH 6 with HCl (aq.). The precipitated solids were collected by filtration and washed with water (1x100 mL). The crude product mixture was used in the next step directly without further purification. This resulted in 6-bromo-1H,3H-pyrrolo[1,2-b]pyridazine-2,4-dione (11 g, 85%) as a brown solid.

**Step 5:** Into a 40mL vial were added 6-bromo-1H,3H-pyrrolo[1,2-b]pyridazine-2,4-dione (1 g, 4.366 mmol, 1 eq.) and DIEA (1.13 g, 8.73 mmol, 2 eq.) at RT. To the above mixture was added POCl₃ (10 mL, 107.29 mmol, 24.5 eq.) dropwise over 1min at RT. The resulting mixture was stirred for an additional 2 days at 100°C. The mixture was allowed to cool down to RT. The reaction was quenched by the addition of water (30mL) at RT. The resulting mixture was extracted with EtOAc (3 x 20mL). The combined organic layers were washed with brine (1x10 mL), dried over anhydrous Na₂SO₄. After filtration, the filtrate was concentrated under reduced pressure. The residue was purified by silica gel column chromatography, eluted with PE / EA (12:1) to afford 6-bromo-2,4-dichloropyrrolo[1,2-b]pyridazine (200 mg, 17%) as a yellow solid.

**Step 6:** A mixture of 6-bromo-2,4-dichloropyrrolo[1,2-b]pyridazine (500 mg, 1.880 mmol, 1 equiv) 1-(1,3-thiazol-2-yl)methanamine (258 mg, 2.256 mmol, 1.2 eq.) and DIEA (486 mg, 3.760 mmol, 2 eq.) in DMSO (5 mL) was stirred for 3h at 100°C under air atmosphere. The residue was purified by reversed-phase flash chromatography (column, C18; mobile phase, MeCN in water (10mmol/L NH₄HCO₃), 50% to 80% gradient in 10 min; detector, UV 254 nm) to yield in 6-bromo-2-chloro-N-(1,3-thiazol-2-ylmethyl)pyrrolo[1,2-b]pyridazin-4-amine (500 mg, 77%) as a yellow oil.

**Step 7:** A mixture of 6-bromo-2-chloro-N-(1,3-thiazol-2-ylmethyl)pyrrolo[1,2-b]pyridazin-4-amine (500 mg, 1.455 mmol, 1 equiv), (Boc)₂O (476 mg, 2.18 mmol, 1.5 eq.), TEA (294 mg, 2.910 mmol, 2 eq.) and DMAP (178 mg, 1.455 mmol, 1 eq.) in DCM (5 mL) was stirred for 3h at RT under air atmosphere. And then concentrated under vacuo. The residue was purified by silica gel column chromatography, eluted with PE / EA (10:1) to afford *tert-butyl* (6-bromo-2-chloropyrrolo[1,2-b]pyridazin-4-yl)(thiazol-2-ylmethyl)carbamate (500 mg, 78%) as a yellow oil.

**Step 8:** A mixture of *tert-butyl* (6-bromo-2-chloropyrrolo[1,2-b]pyridazin-4-yl)(thiazol-2-ylmethyl)carbamate (600 mg, 1.35 mmol, 1 eq.), potassium ((2*R*,3*S*)-2-((*tert*-butoxycarbonyl)amino)-3-fluorobutyl)trifluoroborate (borate salt 1) (418 mg, 1.622 mmol, 1.2 eq.), PdCl₂(AMPHOS)₂ (95.7 mg, 0.135 mmol, 0.1 eq.) and Cs₂CO₃ (881 mg, 2.70 mmol, 2 eq.) in toluene (15mL) and H₂O (0.5 mL) was stirred for overnight at 100°C under nitrogen atmosphere. The reaction mixture was cooled down to RT, filtered on decalite aand concentrated under vacuo. The residue was purified by silica gel column chromatography, eluted with PE / EA (10:1) to afford *tert-butyl* (6-((2*R*,3*S*)-2-((*tert-*butoxycarbonyl)amino)-3-fluorobutyl)-2-chloropyrrolo[1,2-b]pyridazin-4-yl)(thiazol-2-ylmethyl)carbamate (250 mg, 50%) as a yellow solid.

**Step 9:** To a stirred solution of *tert-butyl* (6-((2*R*,3*S*)-2-((*tert*-butoxycarbonyl)amino)-3-fluorobutyl)-2-chloropyrrolo[1,2-b]pyridazin-4-yl)(thiazol-2-ylmethyl)carbamate (200 mg, 0.361 mmol, 1 eq.) in THF (4 mL) were added a solution of 1,3-dichloro-5,5-dimethylimidazolidine-2,4-dione (43 mg, 0.217 mmol, 0.6 eq.) in THF (1 mL) dropwise at -40°C. The resulting mixture was stirred for an additional 1h at RT. The residue was purified by reversed-phase flash chromatography (column, C18 silica gel; mobile phase, MeCN in water (10mmol/L NH₄HCO₃), 50% to 90% gradient in 10 min; detector, UV 254 nm) to yield *tert-butyl* (6-((2*R*,3*S*)-2-((*tert*-butoxycarbonyl)amino)-3-fluorobutyl)-2,7-dichloropyrrolo[1,2-b]pyridazin-4-yl)(thiazol-2-ylmethyl)carbamate (140 mg, 66%) as a yellow oil.

**Step 10:** To a stirred solution of *tert-butyl* (6-((2R,3S)-2-((tert-butoxycarbonyl)amino)-3-fluorobutyl)-2,7-dichloropyrrolo[1,2-b]pyridazin-4-yl)(thiazol-2-ylmethyl)carbamate (70 mg, 0.119 mmol, 1 eq.) in DCM (1 mL) was added TFA (0.44 mL) dropwise at 0°C under air atmosphere. The resulting mixture was stirred for an additional 2h at RT. The resulting mixture was concentrated under vacuum. The crude product was purified by Prep-HPLC (Column: YMC-Actus Triart C18 ExRS 30* 150 mm, 5m; Mobile Phase A: water(10mmol/L NH₄HCO₃+0.05%NH₃.H₂O), Mobile Phase B: ACN; Flow rate: 50 mL/min mL/min; Gradient: 23% B to 57% B in 10 min; Wave Length: 254nm/220nm nm; RT1(min): 6.378) to afford 6-((2*R*,3*S*)-2-amino-3-fluorobutyl)-2,7-dichloro-N-(thiazol-2-ylmethyl)pyrrolo[1,2-b]pyridazin-4-amine. LC-MS-(ES, *m*/*z):* [M+H]⁺= 388.10.

### Example 5: ATXN3 Quantitative Splicing Assay.

Human neuroblastoma SK-N-MC cells were plated in 384-well plates at 20,000 cells/well. Twenty-four hours after plating, cells were treated with compounds for 24 h at appropriate concentrations ranging from 30 µM to 0.6 nM (0.3% DMSO). Treated cells were lysed in 15 µL of lysis buffer, and cDNA was synthesized using the Fast Advanced Cells-to-Ct kit. Two µL of each cDNA was used in qPCR reactions to confirm the exon 4 skipped transcripts of ATXN3. A second set of primers/probe E4E5 was used to detect the transcripts containing exon 4. The third set of primers/probe E8E9 was used to detect total gene level of ATXN3. The qPCR reactions were prepared in 384-well plates in 10 µL volume, using TaqMan^{™} Fast Advanced Master Mix with primers and probes shown in the table below. Reactions were run in a Quant Studio 6 qPCR instrument with default settings.

The primers and probes are listed below in **Table 3.**

**Table 3.**

| **Target Sequence** | **Forward Primer** | **Probe** | **Reverse Primer** |
|---|---|---|---|
| ATXN3 E4skpping-FAM | SEQ ID NO: 2 | SEQ ID NO: 3 | SEQ ID NO: 4 |
| | 5' GCAGCCTTCTGGAAATA TGG 3' | 5’ TTCTCTATTCAGAAATG AAAGATCATT 3’ | 5' CTGGACCCGTCAAGAG AGAA 3' |
| ATXN3 E4E5-Cy5 | SEQ ID NO: 5 | SEQ ID NO: 6 | SEQ ID NO: 7 |
| | 5’ TGTTCAACAGTCCAGAG TATCAG 3’ | 5’ AGGCTCAGGATCGATCC TATAAATGAAAGA 3’ | 5' ACCCGTCAAGAGAGAA TTCAAG 3' |
| ATXN3 E8E9-total-FAM | SEQ ID NO: 8 | SEQ ID NO: 9 | SEQ ID NO: 10 |
| | 5’ GATGAGGAGGATTTGCA [?] GAGG 3’ | 5’ ATGTTTCTGGAACTACCT TGCATACTTAGCTG 3’ | 5' CCTGATGTCTGTGTCAT ATCTTGA 3' |
| TBP-YAK (endogenou s control) | SEQ ID NO: 11 | SEQ ID 12 | SEQ ID |
| | 5’ TCGGAGAGTTCTGGGAT T 3’ | 5' CCGCAGCTGCAAAATAT TGTATCCACA 3' | 5' AAGTGCAATGGTCTTTA GGT 3' |

### Example 6: ATXN3 total protein assay.

Human neuroblastoma SK-N-MC cells were seeded at 10,000 cells/well in 384 well plates one day prior to compound treatment. The concentrations of compounds were tested at appropriate doses ranging from 30 µM to 0.6 nM. After incubation for 48 hours, the cells were lysed with 25 µL of lysis buffer containing protease inhibitors, and total ATXN3 protein levels were assessed by Mesoscale Discovery (MSD) assay developed with one pair of anti-ATXN3 antibodies. The capture and detect antibodies were raised in mouse and rabbit respectively. Anti-rabbit MSD-ST antibody was used for secondary antibody.

ATXN3 recombinant protein was used for standards. The readouts were captured with 35 µL of MSD read buffer and multi-array 384-well high binding plates.

One plate replica was carried out for parallel viability testing by CellTiter Glo^{®} 2.0 with a seeding density of 4,000 cells/well. Compounds were incubated for 48 hours. The viability readouts were carried out by Envision according to the manufacturer's instructions.

Compounds were tested as outlined in Examples 5 and 6 above and the results are shown below in **Table 4.**

**Table 4**

| * IC₅₀/EC₅₀ range (nM): 0.01 ≤ A ≤ 100; 101 ≤ B ≤ 500; 501 ≤ C ≤ 5000; 5001 ≤ D ≤ 10000; 10001 ≤ E ≤ 40,000. | |
|---|---|
| Compound | ATXN3 Potency - Splicing E4E5 IC50 Mean (nM) |
| 1 | B |
| 2 | A |
| 3 | A |
| 4 | B |

## Claims

1. A compound of Formula (I), or a pharmaceutically acceptable salt thereof: 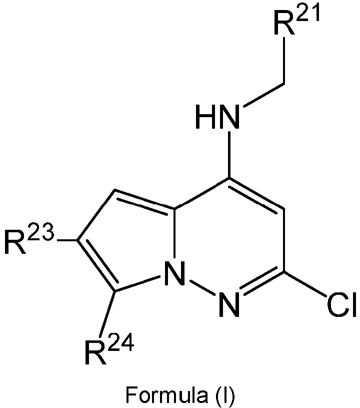 wherein,
R²¹ is furanyl or thiazolyl, each of which is unsubstituted or substituted with 1, 2, or 3, independently selected R^{1A} groups; each R^{1A} is independently selected from halo, CN, NO₂, C₁₋₆ alkyl, C₂₋₆ alkenyl, C₂₋₆ alkynyl, C₁₋₆ haloalkyl, C₁₋₆ alkoxy, - C(=O)OH, -C(=O)C₁₋₆ alkyl, -C(=O)C₁₋₆ haloalkyl, and -C(=O)C₁₋₆ alkoxy
R²³ is selected from the group consisting of H, azido, halo, CN, NO₂, C₁₋₆ alkyl, C₂₋₆ alkenyl, C₂₋₆ alkynyl, C₁₋₆ heteroalkyl, -(C₁₋₆ alkylene)-C₃₋₁₀ cycloalkyl, -(C₁₋₆ alkylene)-4-10 membered heterocycloalkyl, -(C₁₋₆ heteroalkylene)-C₃₋₁₀ cycloalkyl, - (C₁₋₆ heteroalkylene)-4-10 membered heterocycloalkyl, C₃₋₁₀ cycloalkyl, C₆₋₁₀ aryl, 5-10 membered heteroaryl, 4- 10 membered heterocycloalkyl, OR^{a3}, SR^{a3}, C(=O)R^{b3}, C(=O)OR^{b3}, NR^{c3}R^{d3}, C(=O)NR^{c3}R^{d3}, -OC(=O)NR^{c3}R^{d3}, NR^{c3}C(=O)R^{b3}, NR^{c3}C(=O)OR^{b3}, NR^{c3}C(=O)NR^{c3}R^{d3}, NR^{c3}S(=O)₂R^{b3}, NR^{c3}S(=O)₂NR^{c3}R^{d3}, S(O)NR^{c3}R^{d3}, and S(O)₂NR^{c3}R^{d3}, wherein the C₁₋₆ alkyl, C₂₋₆ alkenyl, C₂₋₆ alkynyl, C₁₋₆ heteroalkyl, C₁₋₆ alkylene, C₁₋₆ heteroalkylene, C₃₋₁₀ cycloalkyl, C₆₋₁₀ aryl, 5-10 membered heteroaryl, and 4-10 membered heterocycloalkyl are each optionally substituted by 1, 2, 3, or 4 independently selected R²⁰ groups;
R²⁴ is selected from the group consisting of H, azido, halo, CN, NO₂, C₁₋₆ alkyl, C₂₋₆ alkenyl, C₂₋₆ alkynyl, C₃₋₁₀ cycloalkyl, C₆₋₁₀ aryl, 5-10 membered heteroaryl, 4- 10 membered heterocycloalkyl, OR^{a4}, C(=O)R^{b4}, C(=O)OR^{b4}, NR^{c4}R^{d4}, C(=O)NR^{c4}R^{d4}, - OC(=O)NR^{c4}R^{d4}, NR^{c4}C(=O)R^{b4}, NR^{c4}C(=O)OR^{b4}, NR^{c4}C(=O)NR^{c4}R^{d4}, NR^{c4}S(=O)₂R^{b4}, NR^{c4}S(=O)₂NR^{c4}R^{d4}, S(O)NR^{c4}R^{d4}, and S(O)₂NR^{c4}R^{da}, wherein the C₁₋₆ alkyl, C₃₋₁₀ cycloalkyl, C₂₋₆ alkenyl, C₂₋₆ alkynyl, C₆₋₁₀ aryl, 5-10 membered heteroaryl, and 4-10 membered heterocycloalkyl are each optionally substituted by 1, 2, 3, or 4 independently selected R²⁰ groups;
each R^{a3}, R^{b3}, R^{c3}, R^{d3}, R^{a4}, R^{b4}, R^{c4}, and R^{d4}, is independently selected from the group consisting of H, C₁₋₆ alkyl, C₂₋₆ alkenyl, C₂₋₆ alkynyl, C₁₋₆ hydroxyalkyl, C₁₋₆ haloalkyl, C₁₋₆ alkoxy, - (C₁₋₆ alkylene)-C₁₋₆ alkoxy, C₃₋₁₀ cycloalkyl, -(C₁₋₆ alkylene)-C₃₋₁₀ cycloalkyl, C₆₋₁₀ aryl, 5-10 membered heteroaryl, and 4-10 membered heterocycloalkyl, wherein the C₁₋₆ alkyl, C₂₋₆ alkenyl, C₂₋₆ alkynyl, C₃₋₁₀ cycloalkyl, - (C₁₋₆ alkylene)-C₃₋₁₀ cycloalkyl, C₆₋₁₀ aryl, 5-10 membered heteroaryl, and 4-10 membered heterocycloalkyl are each optionally substituted by 1, 2, 3, or 4 independently selected R²⁰ groups;
or R^{c3} and R^{d3} together with the N atom to which they are connected, come together to form a 5-10 membered heteroaryl or 4-10 membered heterocycloalkyl ring, each optionally substituted by 1, 2, 3, or 4 independently selected R²⁰ groups;
or R^{c4} and R^{d4} together with the N atom to which they are connected, come together to form a 5-10 membered heteroaryl or 4-10 membered heterocycloalkyl ring, each optionally substituted by 1, 2, 3, or 4 independently selected R²⁰ groups; and
each R²⁰ is independently selected from the group consisting of OH, SH, CN, NO₂, halo, oxo, C₁₋₄ alkyl, C₂₋₄ alkenyl, C₂₋₄ alkynyl, C₁₋₄ haloalkyl, C₁₋₄ cyanoalkyl, C₁₋₄ hydroxyalkyl, C₁₋₄ alkoxy, -(C₁₋₄ alkyl)-(C₁₋₄ alkoxy), -(C₁₋₄ alkoxy)-(C₁₋₄ alkoxy), C₁₋₄ haloalkoxy, C₃₋₆ cycloalkyl, phenyl, 5-6 membered heteroaryl, 4-6 membered heterocycloalkyl, amino, C₁₋₄ alkylamino, di(C₁₋₄ alkyl)amino, carbamyl, C₁₋₄ alkylcarbamyl, di(C₁₋₄ alkyl)carbamyl, carbamoyl, C₁₋₄ alkylcarbamoyl, di(C₁₋₄ alkyl)carbamoyl, C₁₋₄ alkylcarbonyl, C₁₋₄ alkoxycarbonyl, C₁₋₄ alkylcarbonylamino, C₁₋₄ alkylsulfonylamino, aminosulfonyl, C₁₋₄ alkylaminosulfonyl, di(C₁₋₄ alkyl)aminosulfonyl, aminosulfonylamino, C₁₋₄ alkylaminosulfonylamino, di(C₁₋₄ alkyl)aminosulfonylamino, aminocarbonylamino, C₁₋₄ alkylaminocarbonylamino, di(C₁₋₄ alkyl)aminocarbonylamino, and amidinyl.

2. The compound of claim 1, or a pharmaceutically acceptable salt thereof, wherein:
each R^{1A} is independently selected from C₁₋₆ alkyl, C₁₋₆ haloalkyl, and halo.

3. The compound of claim 1 or 2, or a pharmaceutically acceptable salt thereof, wherein:
R^{1A} is fluoro.

4. The compound of any one of claims 1 to 3, or a pharmaceutically acceptable salt thereof, wherein:
R²¹ is selected from the group consisting of

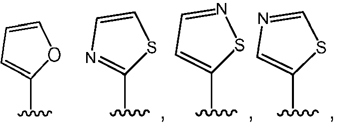

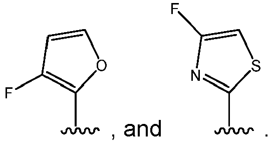

5. The compound of claim 1 or 4, or a pharmaceutically acceptable salt thereof, wherein:
R²¹ is

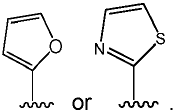

6. The compound of any one of the preceding claims, wherein:
R²³ is substituted or unsubstituted C₁₋₆ alkyl or substituted or unsubstituted C₁₋₆ heteroalkyl.

7. The compound of claim 6, or a pharmaceutically acceptable salt, wherein:
R²³ is CH₂CHNH₂CH₃;
R²³ is CH₂CHNH₂CH₂OH;
R²³ is CH₂CHNH₂CHFCH₃;
R²³ is CH₂CHNH₂CH₂CH₂OH;
R²³ is CH₂CHNH₂CH₂F; or
R²³ is CH₂CHNH₂CH₂OCH₃.

8. The compound of claim 7, or a pharmaceutically acceptable salt thereof, wherein:
R²³ is CH₂CHNH₂CH₂OCD₃.

9. The compound of any one of claims 1-8, or a pharmaceutically acceptable salt thereof, wherein:
R²⁴ is selected from the group consisting of hydrogen, OH, halo, CN, substituted or unsubstituted C₁₋₆ alkyl, substituted or unsubstituted C₁₋₆ alkoxyl, substituted or unsubstituted C₃₋₆ cycloalkyl, substituted or unsubstituted C₂₋₄ alkenyl, and substituted or unsubstituted C₂₋₄ alkynyl.

10. The compound of any one of claims 1-8, or a pharmaceutically acceptable salt thereof, wherein:
R²⁴ is substituted or unsubstituted C₁₋₆ alkyl.

11. The compound of any one of claims 1-8, or a pharmaceutically acceptable salt thereof, wherein:
R²⁴ is selected from the group consisting of F, Cl, and Br.

12. The compound of claim 1, or a pharmaceutically acceptable salt thereof, which is a compound selected from the following compounds, or a pharmaceutically acceptable salt thereof:
| | |
|---|---|
| 1 | 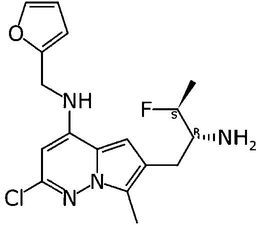 |
| 2 | 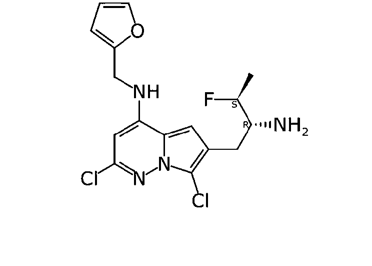 |
| 3 | 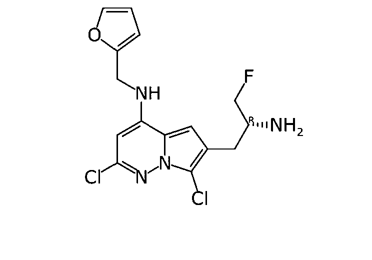 |
| 4 | 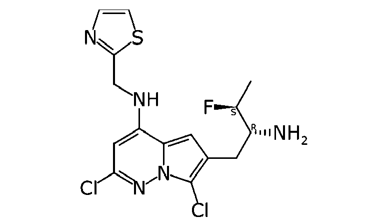 |

13. A pharmaceutical composition comprising:
the compound of any one of the preceding claims, or the pharmaceutically acceptable salt thereof, and
a pharmaceutically acceptable excipient or carrier.

## Patentansprüche

1. Verbindung der Formel (I) oder ein pharmazeutisch annehmbares Salz davon: 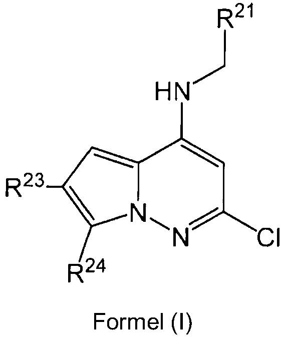 worin
R²¹ Furanyl oder Thiazolyl ist, die jeweils unsubstituiert oder mit 1, 2 oder 3 jeweils unabhängig voneinander ausgewählten Gruppen R^{1A} substituiert sind; die R^{1A} jeweils unabhängig voneinander aus Halogen, CN, NO₂, C₁₋₆-Alkyl, C₂₋₆-Alkenyl, C₂₋₆-Alkinyl, C₁₋₆-Halogenalkyl, C₁₋₆-Alkoxy, -C(=O)OH, -C(=O)C₁₋₆-Alkyl, -C(=O)C₁₋₆-Halogenalkyl und -C(=O)C₁₋₆-Alkoxy ausgewählt ist,
R²³ aus der aus H, Azido, Halogen, CN, NO₂, C₁₋₆-Alkyl, C₂₋₆-Alkenyl, C₂₋₆-Alkinyl, C₁₋₆-Heteroalkyl, -(C₁₋₆-Alkylen)-C₃₋₁₀-cycloalkyl, -(C₁₋₆-Alkylen)-(4- bis 10-gliedriges heterocycloalkyl), -(C₁₋₆-Heteroalkylen)-C₃₋₁₀-cycloalkyl, -(C₁₋₆-Heteroalkylen)-(4- bis 10-gliedriges heterocycloalkyl), C₃₋₁₀-Cycloalkyl, C₆₋₁₀-Aryl, 5- bis 10-gliedrigem Heteroaryl, 4- bis 10-gliedrigem Heterocycloalkyl, OR^{a3}, SR^{a3}, C(=O)R^{b3}, C(=O)OR^{b3}, NR^{c3}R^{d3}, C(=O)NR^{c3}R^{d3}, -OC(=O)NR^{c3}R^{d3}, NR^{c3}C(=O)R^{b3}, NR^{c3}C(=O)OR^{b3}, NR^{c3}C(=O)NR^{c3}R^{d3}, NR^{c3}S(=O)₂R^{b3}, NR^{c3}S(=O)₂NR^{c3}R^{d3}, S(O)NR^{c3}R^{d3} und S(O)₂NR^{c3}R^{d3} bestehenden Gruppe ausgewählt ist, wobei das C₁₋₆-Alkyl, C₂₋₆-Alkenyl, C₂₋₆-Alkinyl, C₁₋₆-Heteroalkyl, C₁₋₆-Alkylen, C₁₋₆-Hetero-alkylen, C₃₋₁₀-Cycloalkyl, C₆₋₁₀-Aryl, 5- bis 10-gliedrige Heteroaryl und 4- bis 10-gliedrige Heterocycloalkyl jeweils gegebenenfalls mit 1, 2, 3 oder 4 jeweils unabhängig voneinander ausgewählten Gruppen R²⁰ substituiert sind;
R²⁴ aus der aus H, Azido, Halogen, CN, NO₂, C₁₋₆-Alkyl, C₂₋₆-Alkenyl, C₂₋₆-Alkinyl, C₃₋₁₀-Cycloalkyl, C₆₋₁₀-Aryl, 5- bis 10-gliedrigem Heteroaryl, 4- bis 10-gliedrigem Heterocycloalkyl, OR^{a4}, C(=O)R^{b4}, C(=O)OR^{b4}, NR^{c4}R^{d4}, C(=O)NR^{c4}R^{d4}, -OC(=O)NR^{c4}R^{d4}, NR^{c4}C(=O)R^{b4}, NR^{c4}C(=O)OR^{b4}, NR^{c4}C(=O)NR^{c4}R^{d4}, NR^{c4}S(=O)₂R^{b4}, NR^{c4}S(=O)₂NR^{c4}R^{d4}, S(O)NR^{c4}Rd⁴ und S(O)₂NR^{c4}R^{d4} bestehenden Gruppe ausgewählt ist, wobei das C₁₋₆-Alkyl, C₃₋₁₀-Cycloalkyl, C₂₋₆-Alkenyl, C₂₋₆-Alkinyl, C₆₋₁₀-Aryl, 5- bis 10-gliedrige Heteroaryl und 4- bis 10-gliedrige Heterocycloalkyl jeweils gegebenenfalls mit 1, 2, 3 oder 4 jeweils unabhängig voneinander ausgewählten Gruppen R²⁰ substituiert sind;
die R^{a3}, R^{b3}, R^{c3}, R^{d3}, R^{a4}, R^{b4}, R^{c4} und R^{d4} jeweils unabhängig voneinander aus der aus H, C₁₋₆-Alkyl, C₂₋₆-Alkenyl, C₂₋₆-Alkinyl, C₁₋₆-Hydroxyalkyl, C₁₋₆-Halogenalkyl, C₁₋₆-Alkoxy, -(C₁₋₆-Alkylen)-C₁₋₆-alkoxy, C₃₋₁₀-Cycloalkyl, -(C₁₋₆-Alkylen)-C₃₋₁₀-cycloalkyl, C₆₋₁₀-Aryl, 5- bis 10-gliedrigem Heteroaryl und 4- bis 10-gliedrigem Heterocycloalkyl bestehenden Gruppe ausgewählt sind, wobei das C₁₋₆-Alkyl, C₂₋₆-Alkenyl, C₂₋₆-Alkinyl, C₃₋₁₀-Cycloalkyl, -(C₁₋₆-Alkylen)-C₃₋₁₀-cycloalkyl, C₆₋₁₀-Aryl, 5- bis 10-gliedrige Heteroaryl und 4- bis 10-gliedrige Heterocycloalkyl jeweils gegebenenfalls mit 1, 2, 3 oder 4 jeweils unabhängig voneinander ausgewählten Gruppen R²⁰ substituiert sind;
oder R^{c3} und R^{d3} zusammen mit dem N-Atom, an das sie gebunden sind, einen 5- bis 10-gliedrigen Heteroaryl- oder 4- bis 10-gliedrigen Heterocycloalkyl-Ring bilden, der jeweils gegebenenfalls mit 1, 2, 3 oder 4 jeweils unabhängig voneinander ausgewählten Gruppen R²⁰ substituiert ist;
oder R^{c4} und R^{d4} zusammen mit dem N-Atom, an das sie gebunden sind, einen 5- bis 10-gliedrigen Heteroaryl- oder 4- bis 10-gliedrigen Heterocycloalkyl-Ring bilden, der jeweils gegebenenfalls mit 1, 2, 3 oder 4 jeweils unabhängig voneinander ausgewählten Gruppen R²⁰ substituiert ist; und
die R²⁰ jeweils unabhängig voneinander aus der aus OH, SH, CN, NO₂, Halogen, Oxo, C₁₋₄-Alkyl, C₂₋₄-Alkenyl, C₂₋₄-Alkinyl, C₁₋₄-Halogenalkyl, C₁₋₄-Cyanoalkyl, C₁₋₄-Hydroxyalkyl, C₁₋₄-Alkoxy, -(C₁₋₄-Alkyl)-(C₁₋₄-alkoxy), -(C₁₋₄-Alkoxy)-(C₁₋₄-alkoxy), C₁₋₄-Halogenalkoxy, C₃₋₆-Cycloalkyl, Phenyl, 5- bis 6-gliedrigem Heteroaryl, 4- bis 6-gliedrigem Heterocycloalkyl, Amino, C₁₋₄-Alkylamino, Di(C₁₋₄-alkyl)amino, Carbamyl, C₁₋₄-Alkylcarbamyl, Di(C₁₋₄-alkyl)-carbamyl, Carbmamoyl, C₁₋₄-Alkylcarbamoyl, Di(C₁₋₄-alkyl)carbamoyl, C₁₋₄-Alkylcarbonyl, C₁₋₄-Alkoxycarbonyl, C₁₋₄-Alkylcarbonylamino, C₁₋₄-Alkylsulfonylamino, Aminosulfonyl, C₁₋₄-Alkylaminosulfonyl, Di(C₁₋₄-alkyl)aminosulfonyl, Aminosulfonylamino, C₁₋₄-Alkylaminosulfonylamino, Di(C₁₋₄-alkyl)aminosulfonylamino, Aminocarbonylamino, C₁₋₄-Alkylaminocarbonylamino, Di(C₁₋₄-alkyl)aminocarbonylamino und Amidinyl bestehenden Gruppe ausgewählt sind.

2. Verbindung nach Anspruch 1 oder ein pharmazeutisch annehmbares Salz davon, worin:
die R^{1A} jeweils unabhängig voneinander aus C₁₋₆-Alkyl, C₁₋₆-Halogenalkyl und Halogen bestehenden Gruppe ausgewählt sind.

3. Verbindung nach Anspruch 1 oder 2 oder ein pharmazeutisch annehmbares Salz davon, worin:
R^{1A} Fluor ist.

4. Verbindung nach einem der Ansprüche 1 bis 3 oder ein pharmazeutisch annehmbares Salz davon, worin:
R²¹ aus der aus 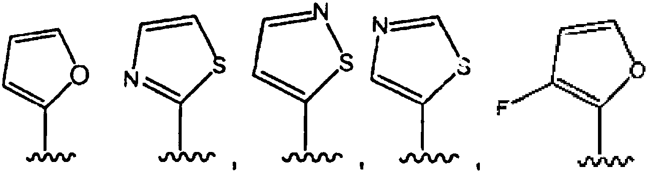 und 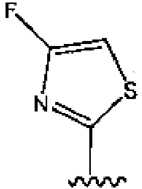 bestehenden Gruppe ausgewählt ist.

5. Verbindung nach Anspruch 1 oder 4 oder ein pharmazeutisch annehmbares Salz davon, worin:
R²¹ 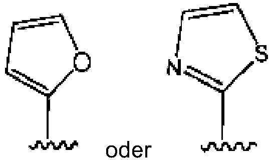 ist.

6. Verbindung nach einem der vorangegangenen Ansprüche, worin:
R²³ substituiertes oder unsubstituiertes C₁₋₆-Alkyl oder substituiertes oder unsubstituiertes C₁₋₆-Heteroalkyl ist.

7. Verbindung nach Anspruch 6 oder pharmazeutisch annehmbares Salz, worin:
R²³ CH₂CHNH₂CH₃ ist;
R²³ CH₂CHNH₂CH₂OH ist;
R²³ CH₂CHNH₂CHFCH₃ ist;
R²³ CH₂CHNH₂CH₂CH₂OH ist;
R²³ CH₂CHNH₂CH₂F ist; oder
R²³ CH₂CHNH₂CH₂OCH₃ ist.

8. Verbindung nach Anspruch 7 oder pharmazeutisch annehmbares Salz davon, worin:
R²³ CH₂CHNH₂CH₂OCD₃ ist.

9. Verbindung nach einem der Ansprüche 1 bis 8 oder pharmazeutisch annehmbares Salz davon, worin:
R²⁴ aus der aus Wasserstoff, OH, Halogen, CN, substituiertem oder unsubstituiertem C₁₋₆-Alkyl, substituiertem oder unsubstituiertem C₁₋₆-Alkoxy, substituiertem oder unsubstituiertem C₃₋₆-Cycloalkyl, substituiertem oder unsubstituiertem C₂₋₄-Alkenyl und substituiertem oder unsubstituiertem C₂₋₄-Alkinyl bestehenden Gruppe ausgewählt ist.

10. Verbindung nach einem der Ansprüche 1 bis 8 oder ein pharmazeutisch annehmbares Salz davon, worin:
R²⁴ substituiertes oder unsubstituiertes C₁₋₆-Alkyl ist.

11. Verbindung nach einem der Ansprüche 1 bis 8 oder ein pharmazeutisch annehmbares Salz davon, worin:
R²⁴ aus der aus F, Cl und Br bestehenden Gruppe ausgewählt ist.

12. Verbindung nach Anspruch 1 oder ein pharmazeutisch annehmbares Salz davon, die eine aus den folgenden Verbindungen ausgewählte Verbindung oder ein pharmazeutisch annehmbares Salz davon ist:
| | |
|---|---|
| 1 | 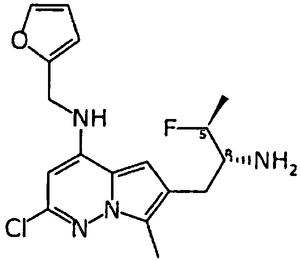 |
| 2 | 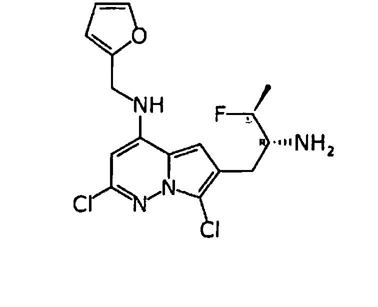 |
| 3 | 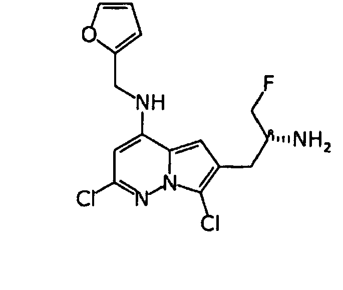 |
| 4 | 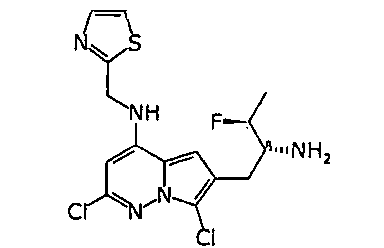 |

13. Pharmazeutische Zusammensetzung, die Folgendes umfasst:
eine Verbindung nach einem der vorangegangenen Ansprüche oder ein pharmazeutisch annehmbares Salz davon und
einen pharmazeutisch annehmbaren Hilfsstoff oder Träger.

## Revendications

1. Composé de formule (I), ou un sel pharmaceutiquement acceptable de celui-ci : 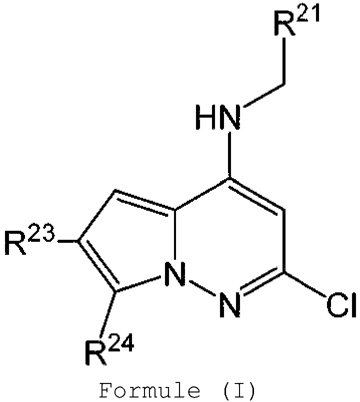 dans lequel,
R²¹ est du furanyle ou du thiazolyle, dont chacun est non substitué ou substitué par 1, 2 ou 3 groupes R^{1A} choisis indépendamment ; chaque R^{1A} est choisi indépendamment parmi halo, CN, NO₂, alkyle en C₁₋₆, alcényle en C₂₋₆, alcynyle en C₂₋₆, haloalkyle en C₁₋₆, alcoxy en C₁₋₆, -C(=O)OH, -C(=O) alkyle en C₁₋₆, -C(=O) haloalkyle en C₁₋₆ et -C(=O) alcoxy en C₁₋₆
R²³ est choisi dans le groupe constitué par H, azido, halo, CN, NO₂, alkyle en C₁₋₆, alcényle en C₂₋₆, alcynyle en C₂₋₆, hétéroalkyle en C₁₋₆, - (alkylène en C₁₋₆) cycloalkyle en C₃₋₁₀, - (alkylène en C₁₋₆) hétérocycloalkyle à 4 à 10 chaînons, - (hétéroalkylène en C₁₋₆) cycloalkyle en C₃₋₁₀, - (hétéroalkylène en C₁₋₆) hétérocycloalkyle à 4 à 10 chaînons, cycloalkyle en C₃₋₁₀, aryle en C₆₋₁₀, hétéroaryle à 5 à 10 chaînons, hétérocycloalkyle à 4 à 10 chaînons, OR^{a3}, SR^{a3}, C(=O)R^{b3}, C(=O)OR^{b3}, NR^{c3}R^{d3}, C(=O)NR^{c3}R^{d3}, -OC(=O)NR^{c3}R^{d3}, NR^{c3}C(=O)R^{b3}, NR^{c3}C(=O)OR^{b3}, NR^{c3}C(=O)NR^{c3}R^{d3}, NR^{c3}S(=O)₂R^{b3}, NR^{c3}S(=O)₂NR^{c3}R^{d3}, S(O)NR^{c3}R^{d3} et S (O)₂NR^{c3}R^{d3}, dans lequel l'alkyle en C₁₋₆, l'alcényle en C₂₋₆, l'alcynyle en C₂₋₆, l'hétéroalkyle en C₁₋₆, l'alkylène en C₁₋₆, l'hétéroalkylène en C₁₋₆, le cycloalkyle en C₃₋₁₀, l'aryle en C₆₋₁₀, l'hétéroaryle à 5 à 10 chaînons et l'hétérocycloalkyle à 4 à 10 chaînons sont chacun facultativement substitués par 1, 2, 3 ou 4 groupes R²⁰ choisis indépendamment ;
R²⁴ est choisi dans le groupe constitué par H, azido, halo, CN, NO₂, alkyle en C₁₋₆, alcényle en C₂₋₆, alcynyle en C₂₋₆, cycloalkyle en C₃₋₁₀, aryle en C₆₋₁₀, hétéroaryle à 5 à 10 chaînons, hétérocycloalkyle à 4 à 10 chaînons, OR^{a4}, C(=O)R^{b4}, C(=O)Or^{b4}, NR^{c4}R^{d4}, C(=O)NR^{c4}R^{d4}, -OC(=O)NR^{c4}R^{d4}, NR^{c4}C(=O)R^{b4}, NR^{c4}C(=O)OR^{b4}, NR^{c4}C(=O)NR^{c4}R^{d4}, NR^{c4}S(=O)₂R^{b4}, NR^{c4}S(=O)₂NR^{c4}R^{d4}, S(O)NR^{c4}R^{d4}, et S(O)₂NR^{c4}R^{d4}, dans lequel l'alkyle en C₁₋₆, le cycloalkyle en C₃₋₁₀, l'alcényle en C₂₋₆, l'alcynyle en C₆₋₁₀, l'aryle en C_{5 à 10}, l'hétéroaryle à 5 à 10 chaînons, et l'hétérocycloalkyle à 4 à 10 chaînons sont chacun facultativement substitués par 1, 2, 3 ou 4, groupes R²⁰ choisis indépendamment ;
chaque R^{a3}, R^{b3}, R^{c3}, R^{d3}, R^{a4}, R ^{b4}, R^{c4} et R^{d4} est choisi indépendamment dans le groupe constitué de H, alkyle en C₁₋₆, alcényle en C₂₋₆, alcynyle en C₂₋₆, hydroxyalkyle en C₁₋₆, haloalkyle en C₁₋₆, alcoxy en C₁₋₆, -(alkylène en C₁₋₆)-alcoxy en C₁₋₆, cycloalkyle en C₃₋₁₀, -(alkylène en C₁₋₆)-cycloalkyle en C₃₋₁₀, aryle en C₆₋₁₀, hétéroaryle à 5 à 10 chaînons et hétérocycloalkyle à 4 à 10 chaînons, dans lequel l'alkyle en C₁₋₆, l'alcényle en C₂₋₆, l'alcynyle en C₂₋₆, le cycloalkyle en C₃₋₁₀, le -(alkylène en C₁₋₆)-cycloalkyle en C₃₋₁₀, l'aryle en C₆₋₁₀, l'hétéroaryle à 5 à 10 chaînons et l'hétérocycloalkyle à 4 à 10 chaînons sont chacun facultativement substitués par 1, 2, 3 ou 4 groupes R²⁰ choisis indépendamment ;
ou R^{c3} et R^{d3}, ensemble avec l'atome de N auquel ils sont connectés, se réunissent pour former un cycle hétéroaryle de 5 à 10 chaînons ou hétérocycloalkyle de 4 à 10 chaînons, substitués chacun facultativement par 1, 2, 3 ou 4 groupes R²⁰ choisis indépendamment ;
ou R^{c4} et R^{d4} ensemble avec l'atome N auquel ils sont connectés, se réunissent pour former un cycle hétéroaryle de 5 à 10 membres ou hétérocycloalkyle de 4 à 10 membres, chacun facultativement substitué par 1, 2, 3 ou 4 groupes R²⁰ sélectionnés indépendamment ; et
chaque R²⁰ est choisi indépendamment dans le groupe constitué de OH, SH, CN, NO₂, halo, oxo, alkyle en C₁₋₄, alcényle en C₂₋₄, alcynyle en C₂₋₄, haloalkyle en C₁₋₄, cyanoalkyle en C₁₋₄, hydroxyalkyle en C₁₋₄, alcoxy en C₁₋₄, - (alkyle en C₁₋₄) - (alcoxy en C₁₋₄), - (alcoxy en C₁₋₄) - (alcoxy en C₁₋₄), haloalcoxy en C₁₋₄, cycloalkyle en C₃₋₆, phényle, hétéroaryle à 5-6 chaînons, hétérocycloalkyle à 4-6 chaînons, amino, alkylamino en C₁₋₄, di (alkyl en C₁₋₄) amino, carbamyle, alkylcarbamyle en C₁₋₄, di(alkyl en C₁₋₄)carbamyle, carbamoyle, alkylcarbamoyle en C₁₋₄, di(alkyl en C₁₋₄) carbamoyle, alkylcarbonyle en C₁₋₄ , alcoxycarbonyle en C₁₋₄, alkylcarbonylamino en C₁₋₄, alkylsulfonylamino en C₁₋₄, aminosulfonyle, alkylaminosulfonyle en C₁₋₄, et amidonyle.

2. Composé selon la revendication 1, ou un sel pharmaceutiquement acceptable de celui-ci, dans lequel :
chaque R^{1A} est choisi indépendamment parmi un alkyle en C₁₋₆, un haloalkyle en C₁₋₆ et un halo.

3. Composé selon la revendication 1 ou 2, ou un sel pharmaceutiquement acceptable de celui-ci, dans lequel :
R^{1A} est fluoro.

4. Composé selon l'une quelconque des revendications 1 à 3, ou un sel pharmaceutiquement acceptable de celui-ci, dans lequel :
R²¹ est choisi dans le groupe constitué de

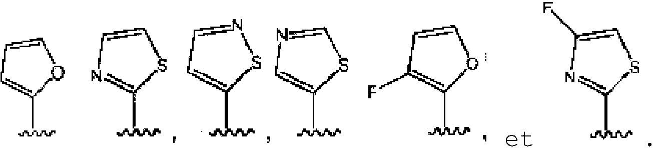

5. Composé selon la revendication 1 ou 4, ou un sel pharmaceutiquement acceptable de celui-ci, dans lequel :
R²¹ est

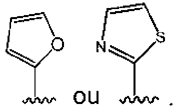

6. Composé selon l'une quelconque des revendications précédentes, dans lequel :
R²³ est un alkyle en C₁₋₆ substitué ou non substitué ou un hétéroalkyle en C₁₋₆ substitué ou non substitué.

7. Composé selon la revendication 6, ou un sel pharmaceutiquement acceptable, dans lequel :
R²³ est CH₂CHNH₂CH₃ ;
R²³ est CH₂CHNH₂CH₂OH ;
R²³ est CH₂CHNH₂CHFCH₃ ;
R²³ est CH₂CHNH₂CH₂CH₂OH ;
R²³ est CH₂CHNH₂CH₂F ; ou
R²³ est CH₂CHNH₂CH₂OCH₃.

8. Composé selon la revendication 7, ou un sel pharmaceutiquement acceptable de celui-ci, dans lequel :
R²³ est CH₂CHNH₂CH₂OCD₃.

9. Composé selon l'une quelconque des revendications 1 à 8, ou un sel pharmaceutiquement acceptable de celui-ci, dans lequel :
R²⁴ est choisi dans le groupe constitué de hydrogène, OH, halo, CN, alkyle en C₁₋₆ substitué ou non substitué, alcoxyle en C₁₋₆ substitué ou non substitué, cycloalkyle en C₃₋₆ substitué ou non substitué, alcényle en C₂₋₄ substitué ou non substitué et alcynyle en C₂₋₄ substitué ou non substitué.

10. Composé selon l'une quelconque des revendications 1 à 8, ou un sel pharmaceutiquement acceptable de celui-ci, dans lequel :
R²⁴ est un alkyle en C₁₋₆ substitué ou non substitué.

11. Composé selon l'une quelconque des revendications 1 à 8, ou un sel pharmaceutiquement acceptable de celui-ci, dans lequel :
R²⁴ est choisi dans le groupe constitué de F, Cl et Br.

12. Composé selon la revendication 1, ou un sel pharmaceutiquement acceptable de celui-ci, qui est un composé choisi parmi les composés suivants, ou un sel pharmaceutiquement acceptable de celui-ci :
| | |
|---|---|
| 1 | 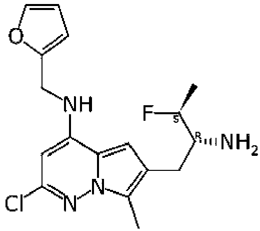 |
| 2 | 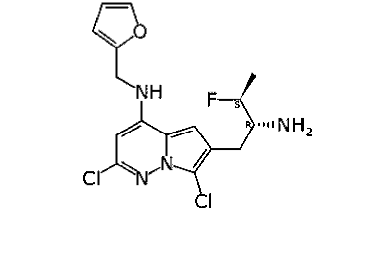 |
| 3 | 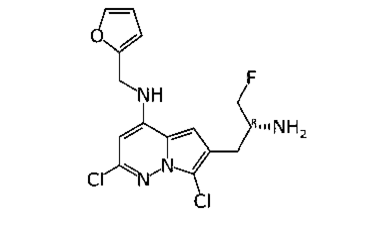 |
| 4 | 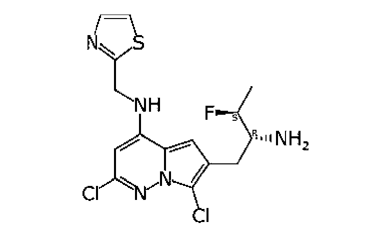 |

13. Composition pharmaceutique, comprenant :
le composé selon l'une quelconque des revendications précédentes, ou le sel pharmaceutiquement acceptable de celui-ci, et
un excipient ou un support pharmaceutiquement acceptable.
